# EUROPEAN PATENT SPECIFICATION

(11) **EP 2 753 611 B1**
(45) Date of publication and mention of the grant of the patent: **11.04.2018**
(21) Application number: 12829739.7
(22) Date of filing: 16.08.2012
(51) Int. Cl.: C07D 405/04, C07D 405/14, C07D 471/04, C07D 473/00, C07D 498/04, A61K 31/343, A61K 45/06, A61K 31/4045, A61K 31/416, A61K 31/4184, A61K 31/4375, A61K 31/4439, A61K 31/52, A61P 31/12, A61P 31/14

(54) **SUBSTITUTED BENZOFURAN COMPOUNDS AND METHODS OF USE THEREOF FOR THE TREATMENT OF VIRAL DISEASES**
SUBSTITUIERTE BENZOFURANVERBINDUNGEN UND VERFAHREN ZU IHRER VERWENDUNG FÜR DIE BEHANDLUNG VON VIRENERKRANKUNGEN
COMPOSÉS BENZOFURANES SUBSTITUÉS ET LEURS PROCÉDÉS D'UTILISATION POUR LE TRAITEMENT DE MALADIES VIRALES

(30) Priority: 08.09.2011 WO PCT/CN2011/079463
(43) Date of publication of application: 16.07.2014
(73) Proprietor: Merck Sharp & Dohme Corp., Rahway, NJ 07065-0907 (US); MSD Italia S.r.l., 00189 Rome (IT)
(72) Inventor: MCCOMAS, Casey Cameron, West Point, Pennsylvania 19486 (US); LIVERTON, Nigel J., West Point, Pennsylvania 19486 (US); HABERMANN, Joerg, 00040 Rome (IT); KOCH, Uwe, 00040 Rome (IT); NARJES, Frank, 00040 Rome (IT); LI, Peng, Shanghai 200131 (CN); PENG, Xuanjia, Shanghai 200131 (CN); SOLL, Richard, Shanghai 200131 (CN); WU, Hao, Shanghai 200131 (CN); PALANI, Anandan, Rahway, New Jersey 07670 (US); DAI, Xing, Rahway, New Jersey 07670 (US); LIU, Hong, Rahway, New Jersey 07670 (US); HE, Shuwen, Rahway, New Jersey 07670 (US); DANG, Qung, Rahway, New Jersey 07670 (US)
(74) Representative: Jaap, David Robert
(86) International application number: PCT/CN2012/080216
(87) International publication number: WO 2013/034048

(56) References cited:
- WO-A1-2010/030592
- WO-A1-2011/106929
- WO-A1-2011/106992
- WO-A1-2013/033971
- WO-A2-2004/041201
- CN-A- 1 731 993

## Description

### FIELD OF THE INVENTION

The present invention relates to novel Substituted Benzofuran Compounds, compositions comprising at least one Substituted Benzofuran Compound, and the Substituted Benzofuran Compounds for use in treating or preventing HCV infection in a patient.

### BACKGROUND OF THE INVENTION

Hepatitis C virus (HCV) infection is a major health problem that leads to chronic liver disease, such as cirrhosis and hepatocellular carcinoma, in a substantial number of infected individuals. Current treatments for HCV infection include immunotherapy with recombinant interferon-a alone or in combination with the nucleoside analog ribavirin.

Several virally-encoded enzymes are putative targets for therapeutic intervention, including a metalloprotease (NS2-3), a serine protease (NS3, amino acid residues 1-180), a helicase (NS3, full length), an NS3 protease cofactor (NS4A), a membrane protein (NS4B), a zinc metalloprotein (NS5A) and an RNA-dependent RNA polymerase (NS5B).

HCV NS5B polymerase is described, for example, in Behrens et al., EMBO J. 15(1) 12-22 (1996). Antagonists of NS5B activity are known to be inhibitors of HCV replication. See Carroll et al., J. Biol. Chem.. 278(14) 11979-84 (2003).

There is a clear and long-felt need to develop effective therapeutics for treatment of HCV infection. Specifically, there is a need to develop compounds that selectively inhibit HCV viral replication and that would be useful for treating HCV-infected patients.

WO 2004/041201 and WO 2010/030592 relate to benzofuran derivatives useful in the treatment of Hepatitis C viral infection. WO 2011/106929 and WO 2011/106992 relate to substituted benzofurans found to be inhibitors of hepatitis C virus NS5B polymerase. WO 2013/033971 relates to tetracyclic heterocyclic compounds useful in the treatment of viral diseases.

### SUMMARY OF THE INVENTION

In one aspect, the present invention provides Compounds of Formula (I) 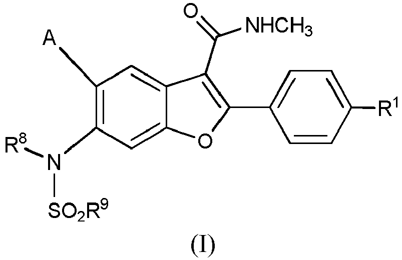 and pharmaceutically acceptable salts thereof, wherein:
A has the structure: 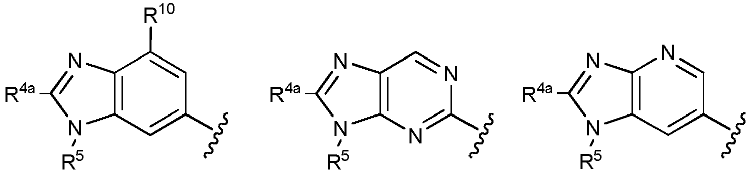 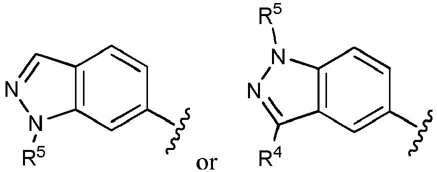
R¹ is halo;
R⁴ is selected from H, halo, pyridyl and phenyl, wherein said phenyl group can be optionally substituted with C₁-C₆ alkyl, C₁-C₆ haloalkyl or halo;
R^{4a} is selected from H, methyl and phenyl, wherein said phenyl group can be optionally substituted with up to 2 groups, which can be the same or different, and are selected from C₁-C₆ alkyl, C₁-C₆ haloalkyl, halo, -CN, -O-(C₁-C₆ alkyl) and -O-(C₁-C₆ haloalkyl);
R⁵ is H; C₁-C₆ hydroxyalkyl; -C(O)O-t-butyl; pyridyl; tetrahydropyranyl; phenyl, which can be optionally substituted with -O-(C₁-C₆ alkyl), halo, -CN, -CF₃, -OCF₃ or -SO₂CH₃; benzyl, which can be optionally substituted with -CF₃; and pyrrolidinyl, which can be optionally substituted on its ring nitrogen atom with -C(O)O-t-butyl;
R⁸ and R⁹ are each C₁-C₆ alkyl and
each occurrence of R¹⁰ is independently H or halo.

The Compounds of Formula (I) (also referred to herein as the "Substituted Benzofuran Compounds") and pharmaceutically acceptable salts thereof can be useful, for example, for inhibiting HCV viral replication or replicon activity, and for treating or preventing HCV infection in a patient. Without being bound by any specific theory, it is believed that the Substituted Benzofuran Compounds inhibit HCV viral replication by inhibiting HCV NS5B.

Accordingly, the present invention provides at least one Substituted Benzofuran Compound for use in treating or preventing HCV infection in a patient.

The details of the invention are set forth in the accompanying detailed description below.

### DETAILED DESCRIPTION OF THE INVENTION

The present invention relates to novel Substituted Benzofuran Compounds, compositions comprising at least one Substituted Benzofuran Compound, and methods of using the Substituted Benzofuran Compounds for treating or preventing HCV infection in a patient.

### Definitions and Abbreviations

The terms used herein have their ordinary meaning and the meaning of such terms is independent at each occurrence thereof. That notwithstanding and except where stated otherwise, the following definitions apply throughout the specification and claims. Chemical names, common names, and chemical structures may be used interchangeably to describe the same structure. If a chemical compound is referred to using both a chemical structure and a chemical name and an ambiguity exists between the structure and the name, the structure is understood to predominate. These definitions apply regardless of whether a term is used by itself or in combination with other terms, unless otherwise indicated. Hence, the definition of "alkyl" applies to "alkyl" as well as the "alkyl" portions of "hydroxyalkyl," "haloalkyl," "-O-alkyl," etc...

As used herein, and throughout this disclosure, the following terms, unless otherwise indicated, shall be understood to have the following meanings:
A "patient" is a human or non-human mammal. In one embodiment, a patient is a human. In another embodiment, a patient is a chimpanzee.

The term "effective amount" as used herein means that amount of active compound or pharmaceutical agent that elicits the biological or medicinal response in a tissue, system, animal or human that is being sought by a researcher, veterinarian, medical doctor or other clinician. In one embodiment, the effective amount is a "therapeutically effective amount" for the alleviation of one or more symptoms of the disease or condition being treated. In another embodiment, the effective amount is a "prophylactically effective amount" for reduction of the severity or likelihood of one or more symptoms of the disease or condition. In another embodiment, the effective amount is a "therapeutically effective amount" for inhibition of HCV viral replication and/or HCV viral production. The term also includes herein the amount of active compound sufficient to inhibit HCV NS5B activity and thereby elicit the response being sought (*i.e*., an "inhibition effective amount"). When the active compound (*i.e*., active ingredient) is administered as the salt, references to the amount of active ingredient are to the free acid or free base form of the compound.

The term "preventing," as used herein with respect to an HCV viral infection or HCV-virus related disorder, refers to reducing the likelihood of HCV infection.

The term "alkyl," as used herein, refers to an aliphatic hydrocarbon group having one of its hydrogen atoms replaced with a bond. An alkyl group may be straight or branched and contain from about 1 to about 20 carbon atoms. In one embodiment, an alkyl group contains from about 1 to about 12 carbon atoms. In different embodiments, an alkyl group contains from 1 to 6 carbon atoms (C₁-C₆ alkyl) or from about 1 to about 3 carbon atoms (C₁-C₃ alkyl). Examples of alkyl groups include methyl, ethyl, n-propyl, isopropyl, n-butyl, sec-butyl, isobutyl, tert-butyl, n-pentyl, neopentyl, isopentyl, n-hexyl, isohexyl and neohexyl. An alkyl group may be unsubstituted or substituted by one or more substituents which may be the same or different, each substituent being independently selected from the group consisting of halo, alkenyl, alkynyl, aryl, cycloalkyl, cyano, hydroxy, -O-alkyl, -O-aryl, -alkylene-O-alkyl, alkylthio, -NH₂, - NH(alkyl), -N(alkyl)₂, -NH(cycloalkyl), -O-C(O)-alkyl, -O-C(O)-aryl, -O-C(O)-cycloalkyl, - C(O)OH and -C(O)O-alkyl. In one embodiment, an alkyl group is linear. In another embodiment, an alkyl group is branched. Unless otherwise indicated, an alkyl group is unsubstituted.

The term "alkenyl," as used herein, refers to an aliphatic hydrocarbon group containing at least one carbon-carbon double bond and having one of its hydrogen atoms replaced with a bond. An alkenyl group may be straight or branched and contain from about 2 to about 15 carbon atoms. In one embodiment, an alkenyl group contains from about 2 to about 12 carbon atoms. In another embodiment, an alkenyl group contains from about 2 to about 6 carbon atoms. Examples of alkenyl groups include ethenyl, propenyl, n-butenyl, 3-methylbut-2-enyl, n-pentenyl, octenyl and decenyl. An alkenyl group may be unsubstituted or substituted by one or more substituents which may be the same or different, each substituent being independently selected from the group consisting of halo, alkenyl, alkynyl, aryl, cycloalkyl, cyano, hydroxy, - O-alkyl, -O-aryl, -alkylene-O-alkyl, alkylthio, -NH₂, -NH(alkyl), -N(alkyl)₂, -NH(cycloalkyl), - O-C(O)-alkyl, -O-C(O)-aryl, -O-C(O)-cycloalkyl, -C(O)OH and -C(O)O-alkyl. The term "C₂-C₆ alkenyl" refers to an alkenyl group having from 2 to 6 carbon atoms. Unless otherwise indicated, an alkenyl group is unsubstituted.

The term "alkynyl," as used herein, refers to an aliphatic hydrocarbon group containing at least one carbon-carbon triple bond and having one of its hydrogen atoms replaced with a bond. An alkynyl group may be straight or branched and contain from about 2 to about 15 carbon atoms. In one embodiment, an alkynyl group contains from about 2 to about 12 carbon atoms. In another embodiment, an alkynyl group contains from about 2 to about 6 carbon atoms. Examples of alkynyl groups include ethynyl, propynyl, 2-butynyl and 3-methylbutynyl. An alkynyl group may be unsubstituted or substituted by one or more substituents which may be the same or different, each substituent being independently selected from the group consisting of halo, alkenyl, alkynyl, aryl, cycloalkyl, cyano, hydroxy, -O-alkyl, -O-aryl, -alkylene-O-alkyl, alkylthio, -NH₂, -NH(alkyl), -N(alkyl)₂, -NH(cycloalkyl), -O-C(O)-alkyl, -O-C(O)-aryl, -O-C(O)-cycloalkyl, -C(O)OH and -C(O)O-alkyl. The term "C₂-C₆ alkynyl" refers to an alkynyl group having from 2 to 6 carbon atoms. Unless otherwise indicated, an alkynyl group is unsubstituted.

The term "alkylene," as used herein, refers to an alkyl group, as defined above, wherein one of the alkyl group's hydrogen atoms has been replaced with a bond. Examples of alkylene groups include -CH₂-, -CH₂CH₂-, -CH₂CH₂CH₂-, -CH₂CH₂CH₂CH₂-, - CH(CH₃)CH₂CH₂-, -CH(CH₃)- and -CH₂CH(CH₃)CH₂-. In one embodiment, an alkylene group has from 1 to about 6 carbon atoms. In another embodiment, an alkylene group is branched. In another embodiment, an alkylene group is linear. In one embodiment, an alkylene group is - CH₂-. The term "C₁-C₆ alkylene" refers to an alkylene group having from 1 to 6 carbon atoms.

The term "aryl," as used herein, refers to an aromatic monocyclic or multicyclic ring system comprising from about 6 to about 14 carbon atoms. In one embodiment, an aryl group contains from about 6 to about 10 carbon atoms. An aryl group can be optionally substituted with one or more "ring system substituents" which may be the same or different, and are as defined herein below. In one embodiment, an aryl group can be optionally fused to a cycloalkyl or cycloalkanoyl group. Examples of aryl groups include phenyl and naphthyl. In one embodiment, an aryl group is phenyl. Unless otherwise indicated, an aryl group is unsubstituted.

The term "cycloalkyl," as used herein, refers to a non-aromatic mono- or multicyclic ring system comprising from about 3 to about 10 ring carbon atoms. In one embodiment, a cycloalkyl contains from about 5 to about 10 ring carbon atoms. In another embodiment, a cycloalkyl contains from about 3 to about 7 ring atoms. In another embodiment, a cycloalkyl contains from about 5 to about 7 ring atoms. In another embodiment, a cycloalkyl contains from about 5 to about 6 ring atoms. The term "cycloalkyl" also encompasses a cycloalkyl group, as defined above, which is fused to an aryl (*e.g*., benzene) or heteroaryl ring. Examples of monocyclic cycloalkyls include cyclopropyl, cyclobutyl, cyclopentyl, cyclohexyl, cycloheptyl and cyclooctyl. Examples of multicyclic cycloalkyls include 1-decalinyl, norbornyl and adamantyl. A cycloalkyl group can be optionally substituted with one or more "ring system substituents" which may be the same or different, and are as defined herein below. In one embodiment, a cycloalkyl group is unsubstituted. The term "3 to 7-membered cycloalkyl" refers to a cycloalkyl group having from 3 to 7 ring carbon atoms. Unless otherwise indicated, a cycloalkyl group is unsubstituted. A ring carbon atom of a cycloalkyl group may be functionalized as a carbonyl group. An illustrative example of such a cycloalkyl group (also referred to herein as a "cycloalkanoyl" group) includes, cyclobutanoyl:

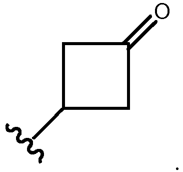

The term "cycloalkenyl," as used herein, refers to a non-aromatic mono- or multicyclic ring system comprising from about 4 to about 10 ring carbon atoms and containing at least one endocyclic double bond. In one embodiment, a cycloalkenyl contains from about 4 to about 7 ring carbon atoms. In another embodiment, a cycloalkenyl contains 5 or 6 ring atoms. Examples of monocyclic cycloalkenyls include cyclopentenyl, cyclohexenyl, cyclohepta-1,3-dienyl, and the like. A cycloalkenyl group can be optionally substituted with one or more "ring system substituents" which may be the same or different, and are as defined herein below. A ring carbon atom of a cycloalkyl group may be functionalized as a carbonyl group. In one embodiment, a cycloalkenyl group is cyclopentenyl. In another embodiment, a cycloalkenyl group is cyclohexenyl. The term "4 to 7-membered cycloalkenyl" refers to a cycloalkenyl group having from 4 to 7 ring carbon atoms. Unless otherwise indicated, a cycloalkenyl group is unsubstituted.

The term "halo," as used herein, means -F, -Cl, -Br or -I.

The term "haloalkyl," as used herein, refers to an alkyl group as defined above, wherein one or more of the alkyl group's hydrogen atoms has been replaced with a halogen. In one embodiment, a haloalkyl group has from 1 to 6 carbon atoms. In another embodiment, a haloalkyl group is substituted with from 1 to 3 F atoms. Examples of haloalkyl groups include - CH₂F, -CHF₂, -CF₃, -CH₂Cl and -CCl₃. The term "C₁-C₆ haloalkyl" refers to a haloalkyl group having from 1 to 6 carbon atoms.

The term "hydroxyalkyl," as used herein, refers to an alkyl group as defined above, wherein one or more of the alkyl group's hydrogen atoms has been replaced with an -OH group. In one embodiment, a hydroxyalkyl group has from 1 to 6 carbon atoms. Examples of hydroxyalkyl groups include -CH₂OH, -CH₂CH₂OH, -CH₂CH₂CH₂OH and -CH₂CH(OH)CH₃. The term "C₁-C₆ hydroxyalkyl" refers to a hydroxyalkyl group having from 1 to 6 carbon atoms.

The term "heteroaryl," as used herein, refers to an aromatic monocyclic or multicyclic ring system comprising about 5 to about 14 ring atoms, wherein from 1 to 4 of the ring atoms is independently O, N or S and the remaining ring atoms are carbon atoms. In one embodiment, a heteroaryl group has 5 to 10 ring atoms. In another embodiment, a heteroaryl group is monocyclic and has 5 or 6 ring atoms. In another embodiment, a heteroaryl group is bicyclic and has 9 or 10 ring atoms. A heteroaryl group can be optionally substituted by one or more "ring system substituents" which may be the same or different, and are as defined herein below. A heteroaryl group is joined via a ring carbon atom, and any nitrogen atom of a heteroaryl can be optionally oxidized to the corresponding N-oxide. The term "heteroaryl" also encompasses a heteroaryl group, as defined above, which is fused to a benzene ring. The term "heteroaryl" also encompasses any fused polycyclic ring system containing at least one ring heteroatom selected from N, O and S, wherein at least one ring of the fused polycyclic ring system is aromatic. For example, the term "9 to 10-membered bicyclic heteroaryl" encompasses a non-aromatic 5 membered heterocyclic ring that is fused to a benzene or pyridyl ring. Examples of heteroaryls include pyridyl, pyrazinyl, furanyl, thienyl, pyrimidinyl, pyridone (including N-substituted pyridones), isoxazolyl, isothiazolyl, oxazolyl, oxadiazolyl, thiazolyl, pyrazolyl, furazanyl, pyrrolyl, triazolyl, 1,2,4-thiadiazolyl, pyrazinyl, pyridazinyl, quinoxalinyl, phthalazinyl, oxindolyl, imidazo[1,2-a]pyridinyl, imidazo[2,1-b]thiazolyl, benzofurazanyl, indolyl, azaindolyl, benzimidazolyl, benzothienyl, quinolinyl, imidazolyl, benzimidazolyl, thienopyridyl, quinazolinyl, thienopyrimidyl, pyrrolopyridyl, imidazopyridyl, isoquinolinyl, benzoazaindolyl, 1,2,4-triazinyl, benzothiazolyl and the like, and all isomeric forms thereof. The term "heteroaryl" also refers to partially saturated heteroaryl moieties such as, for example, tetrahydroisoquinolyl, tetrahydroquinolyl and the like. In one embodiment, a heteroaryl group is a 5-membered heteroaryl. In another embodiment, a heteroaryl group is a 6-membered heteroaryl. In another embodiment, a heteroaryl group comprises a 5- to 6-membered heteroaryl group fused to a benzene ring. Unless otherwise indicated, a heteroaryl group is unsubstituted.

The term "heterocycloalkyl," as used herein, refers to a non-aromatic saturated monocyclic or multicyclic ring system comprising 3 to about 11 ring atoms, wherein from 1 to 4 of the ring atoms are independently O, S, N or Si, and the remainder of the ring atoms are carbon atoms. A heterocycloalkyl group can be joined via a ring carbon, ring silicon atom or ring nitrogen atom. In one embodiment, a heterocycloalkyl group is monocyclic and has from about 3 to about 7 ring atoms. In another embodiment, a heterocycloalkyl group is monocyclic has from about 4 to about 7 ring atoms. In another embodiment, a heterocycloalkyl group is monocyclic has from about 5 to about 7 ring atoms. In another embodiment, a heterocycloalkyl group is bicyclic and has from about 7 to about 11 ring atoms. In still another embodiment, a heterocycloalkyl group is monocyclic and has 5 or 6 ring atoms. In one embodiment, a heterocycloalkyl group is monocyclic. In another embodiment, a heterocycloalkyl group is bicyclic. There are no adjacent oxygen and/or sulfur atoms present in the ring system. Any -NH group in a heterocycloalkyl ring may exist protected such as, for example, as an -N(BOC), - N(Cbz), -N(Tos) group and the like; such protected heterocycloalkyl groups are considered part of this invention. The term "heterocycloalkyl" also encompasses a heterocycloalkyl group, as defined above, which is fused to an aryl (e.g., benzene) or heteroaryl ring. A heterocycloalkyl group can be optionally substituted by one or more "ring system substituents" which may be the same or different, and are as defined herein below. The nitrogen or sulfur atom of the heterocycloalkyl can be optionally oxidized to the corresponding N-oxide, S-oxide or S,S-dioxide. Examples of monocyclic heterocycloalkyl rings include oxetanyl, piperidyl, pyrrolidinyl, piperazinyl, morpholinyl, thiomorpholinyl, thiazolidinyl, 1,4-dioxanyl, tetrahydrofuranyl, tetrahydrothiophenyl, delta-lactam, delta-lactone, silacyclopentane, silapyrrolidine and the like, and all isomers thereof. Illustrative examples of a silyl-containing heterocycloalkyl group include:

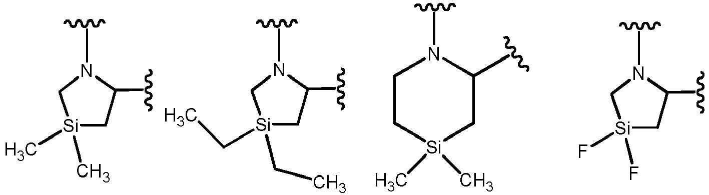

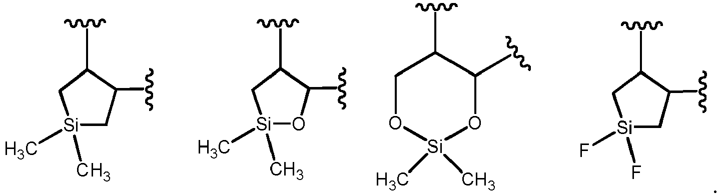

A ring carbon atom of a heterocycloalkyl group may be functionalized as a carbonyl group. An illustrative example of such a heterocycloalkyl group is:

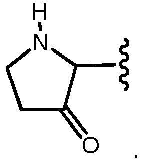

In one embodiment, a heterocycloalkyl group is a 5-membered monocyclic heterocycloalkyl. In another embodiment, a heterocycloalkyl group is a 6-membered monocyclic heterocycloalkyl. The term "3 to 7-membered monocyclic cycloalkyl" refers to a monocyclic heterocycloalkyl group having from 3 to 7 ring atoms. The term "4 to 7-membered monocyclic cycloalkyl" refers to a monocyclic heterocycloalkyl group having from 4 to 7 ring atoms. The term "5 to 7-membered monocyclic cycloalkyl" refers to a monocyclic heterocycloalkyl group having from 5 to 7 ring atoms. The term "7 to 11-membered bicyclic heterocycloalkyl" refers to a bicyclic heterocycloalkyl group having from 7 to 11 ring atoms. Unless otherwise indicated, an heterocycloalkyl group is unsubstituted.

The term "heterocycloalkenyl," as used herein, refers to a heterocycloalkyl group, as defined above, wherein the heterocycloalkyl group contains from 4 to 10 ring atoms, and at least one endocyclic carbon-carbon or carbon-nitrogen double bond. A heterocycloalkenyl group can be joined via a ring carbon or ring nitrogen atom. In one embodiment, a heterocycloalkenyl group has from 4 to 7 ring atoms. In another embodiment, a heterocycloalkenyl group is monocyclic and has 5 or 6 ring atoms. In another embodiment, a heterocycloalkenyl group is bicyclic. A heterocycloalkenyl group can optionally substituted by one or more ring system substituents, wherein "ring system substituent" is as defined above. The nitrogen or sulfur atom of the heterocycloalkenyl can be optionally oxidized to the corresponding N-oxide, S-oxide or S,S-dioxide. Examples of heterocycloalkenyl groups include 1,2,3,4-tetrahydropyridinyl, 1,2-dihydropyridinyl, 1,4-dihydropyridinyl, 1,2,3,6-tetrahydropyridinyl, 1,4,5,6-tetrahydropyrimidinyl, 2-pyrrolinyl, 3-pyrrolinyl, 2-imidazolinyl, 2-pyrazolinyl, dihydroimidazolyl, dihydrooxazolyl, dihydrooxadiazolyl, dihydrothiazolyl, 3,4-dihydro-2H-pyranyl, dihydrofuranyl, fluoro-substituted dihydrofuranyl, 7-oxabicyclo[2.2.1]heptenyl, dihydrothiophenyl, dihydrothiopyranyl, and the like and the like. A ring carbon atom of a heterocycloalkenyl group may be functionalized as a carbonyl group. In one embodiment, a heterocycloalkenyl group is a 5-membered heterocycloalkenyl. In another embodiment, a heterocycloalkenyl group is a 6-membered heterocycloalkenyl. The term "4 to 7-membered heterocycloalkenyl" refers to a heterocycloalkenyl group having from 4 to 7 ring atoms. Unless otherwise indicated, a heterocycloalkenyl group is unsubstituted.

The term "ring system substituent," as used herein, refers to a substituent group attached to an aromatic or non-aromatic ring system which, for example, replaces an available hydrogen on the ring system. Ring system substituents may be the same or different, each being independently selected from the group consisting of alkyl, alkenyl, alkynyl, aryl, heteroaryl, - alkylene-aryl, -arylene-alkyl, -alkylene-heteroaryl, -alkenylene-heteroaryl, -alkynylene-heteroaryl, -OH, hydroxyalkyl, haloalkyl, -O-alkyl, -O-haloalkyl, -alkylene-O-alkyl, -O-aryl, -O-alkylene-aryl, acyl, -C(O)-aryl, halo, -NO₂, -CN, -SF₅, -C(O)OH, -C(O)O-alkyl, -C(O)O-aryl, - C(O)O-alkylene-aryl, -S(O)-alkyl, -S(O)₂-alkyl, -S(O)-aryl, -S(O)₂-aryl, -S(O)-heteroaryl, - S(O)₂-heteroaryl, -S-alkyl, -S-aryl, -S-heteroaryl, -S-alkylene-aryl, -S-alkylene-heteroaryl, - S(O)₂-alkylene-aryl, -S(O)₂-alkylene-heteroaryl, -Si(alkyl)₂, -Si(aryl)₂, -Si(heteroaryl)₂, - Si(alkyl)(aryl), -Si(alkyl)(cycloalkyl), - Si(alkyl)(heteroaryl), cycloalkyl, heterocycloalkyl, -O-C(O)-alkyl, -O-C(O)-aryl, -O-C(O)-cycloalkyl, -C(=N-CN)-NH₂, -C(=NH)-NH₂, -C(=NH)-NH(alkyl), -N(Y₁)(Y₂), -alkylene-N(Y₁)(Y₂), -C(O)N(Y₁)(Y₂) and -S(O)₂N(Y₁)(Y₂), wherein Y₁ and Y₂ can be the same or different and are independently selected from the group consisting of hydrogen, alkyl, aryl, cycloalkyl, and -alkylene-aryl. "Ring system substituent" may also mean a single moiety which simultaneously replaces two available hydrogens on two adjacent carbon atoms (one H on each carbon) on a ring system. Examples of such moiety are methylenedioxy, ethylenedioxy, -C(CH₃)₂- and the like which form moieties such as, for example:

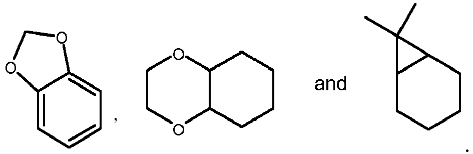

The term "silylalkyl," as used herein, refers to an alkyl group as defined above, wherein one or more of the alkyl group's hydrogen atoms has been replaced with a -Si(R^{x})₃ group, wherein each occurrence of R^{x} is independently C₁-C₆ alkyl, phenyl or a 3- to 6-membered cycloalkyl group. In one embodiment, a silylalkyl group has from 1 to 6 carbon atoms. In another embodiment, a silyl alkyl group contains a -Si(CH₃)₃ moiety. Examples of silylalkyl groups include -CH₂-Si(CH₃)₃ and -CH₂CH₂-Si(CH₃)₃.

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound' or "stable structure" is meant a compound that is sufficiently robust to survive isolation to a useful degree of purity from a reaction mixture.

The term "in substantially purified form," as used herein, refers to the physical state of a compound after the compound is isolated from a synthetic process (*e.g*., from a reaction mixture), a natural source, or a combination thereof. The term "in substantially purified form," also refers to the physical state of a compound after the compound is obtained from a purification process or processes described herein or well-known to the skilled artisan (*e.g*., chromatography, recrystallization and the like), in sufficient purity to be characterizable by standard analytical techniques described herein or well-known to the skilled artisan.

It should also be noted that any carbon as well as heteroatom with unsatisfied valences in the text, schemes, examples and tables herein is assumed to have the sufficient number of hydrogen atom(s) to satisfy the valences.

When a functional group in a compound is termed "protected", this means that the group is in modified form to preclude undesired side reactions at the protected site when the compound is subjected to a reaction. Suitable protecting groups will be recognized by those with ordinary skill in the art as well as by reference to standard textbooks such as, for example, T. W. Greene et al, Protective Groups in Organic Synthesis (1991), Wiley, New York.

When any substituent or variable (*e.g*., alkyl, R⁵, etc.) occurs more than one time in any constituent or in Formula (I), its definition on each occurrence is independent of its definition at every other occurrence, unless otherwise indicated.

As used herein, the term "composition" is intended to encompass a product comprising the specified ingredients in the specified amounts, as well as any product which results, directly or indirectly, from combination of the specified ingredients in the specified amounts.

Prodrugs and solvates of the compounds of the invention are also described. A discussion of prodrugs is provided in T. Higuchi and V. Stella, Pro-drugs as Novel Delivery Systems (1987) 14 of the A.C.S. Symposium Series, and in Bioreversible Carriers in Drug Design, (1987) Edward B. Roche, ed., American Pharmaceutical Association and Pergamon Press. The term "prodrug" means a compound (*e.g*., a drug precursor) that is transformed *in vivo* to provide a Substituted Benzofuran Compound or a pharmaceutically acceptable salt or solvate of the compound. The transformation may occur by various mechanisms (*e.g*., by metabolic or chemical processes), such as, for example, through hydrolysis in blood.

For example, if a Substituted Benzofuran Compound or a pharmaceutically acceptable salt, hydrate or solvate of the compound contains a carboxylic acid functional group, a prodrug can comprise an ester formed by the replacement of the hydrogen atom of the acid group with a group such as, for example, (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as β-dimethylaminoethyl), carbamoyl-(C₁-C₂)alkyl, N,N-di (C₁-C₂)alkylcarbamoyl-(C₁-C₂)alkyl and piperidino-, pyrrolidino- or morpholino(C₂-C₃)alkyl, and the like.

Similarly, if a Substituted Benzofuran Compound contains an alcohol functional group, a prodrug can be formed by the replacement of the hydrogen atom of the alcohol group with a group such as, for example, (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, α-amino(C₁-C₄)alkyl, α-amino(C₁-C₄)alkylene-aryl, arylacyl and α-aminoacyl, or α-aminoacyl-α-aminoacyl, where each α-aminoacyl group is independently selected from the naturally occurring L-amino acids, - P(O)(OH)₂, -P(O)(O(C₁-C₆)alkyl)₂ or glycosyl (the radical resulting from the removal of a hydroxyl group of the hemiacetal form of a carbohydrate), and the like.

If a Substituted Benzofuran Compound incorporates an amine functional group, a prodrug can be formed by the replacement of a hydrogen atom in the amine group with a group such as, for example, R-carbonyl-, RO-carbonyl-, NRR'-carbonyl- wherein R and R' are each independently (C₁-C₁₀)alkyl, (C₃-C₇) cycloalkyl, benzyl, a natural α-aminoacyl, - C(OH)C(O)OY¹ wherein Y¹ is H, (C₁-C₆)alkyl or benzyl, -C(OY²)Y³ wherein Y² is (C₁-C₄) alkyl and Y³ is (C₁-C₆)alkyl; carboxy (C₁-C₆)alkyl; amino(C₁-C₄)alkyl or mono-N- or di-N,N-(C₁-C₆)alkylaminoalkyl; -C(Y⁴)Y⁵ wherein Y⁴ is H or methyl and Y⁵ is mono-N- or di-N,N-(C₁-C₆)alkylamino morpholino; piperidin-1-yl or pyrrolidin-1-yl, and the like.

Pharmaceutically acceptable esters of the present compounds are also described and include the following groups: (1) carboxylic acid esters obtained by esterification of the hydroxy group of a hydroxyl compound, in which the non-carbonyl moiety of the carboxylic acid portion of the ester grouping is selected from straight or branched chain alkyl (*e.g.,* methyl, ethyl, n-propyl, isopropyl, t-butyl, sec-butyl or n-butyl), alkoxyalkyl (*e.g*., methoxymethyl), aralkyl (*e.g.,* benzyl), aryloxyalkyl (for example, phenoxymethyl), aryl (*e.g.,* phenyl optionally substituted with, for example, halogen, C₁₋₄alkyl, -O-(C₁₋₄alkyl) or amino); (2) sulfonate esters, such as alkyl- or aralkylsulfonyl (for example, methanesulfonyl); (3) amino acid esters (*e.g*., L-valyl or L-isoleucyl); (4) phosphonate esters and (5) mono-, di- or triphosphate esters. The phosphate esters may be further esterified by, for example, a C₁₋₂₀ alcohol or reactive derivative thereof, or by a 2,3-di (C₆₋₂₄)acyl glycerol.

One or more compounds of the invention may exist in unsolvated as well as solvated forms with pharmaceutically acceptable solvents such as water, ethanol, and the like. "Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Examples of solvates include ethanolates, methanolates, and the like. A "hydrate" is a solvate wherein the solvent molecule is water.

One or more compounds of the invention may optionally be converted to a solvate. Preparation of solvates is generally known. Thus, for example, M. Caira et al, J. Pharmaceutical Sci., 93(3), 601-611 (2004) describe the preparation of the solvates of the antifungal fluconazole in ethyl acetate as well as from water. Similar preparations of solvates, hemisolvate, hydrates and the like are described by E. C. van Tonder et al, AAPS PharmSciTechours. , 5(1), article 12 (2004); and A. L. Bingham et al, Chem. Commun., 603-604 (2001). A typical, non-limiting, process involves dissolving the inventive compound in desired amounts of the desired solvent (organic or water or mixtures thereof) at a higher than room temperature, and cooling the solution at a rate sufficient to form crystals which are then isolated by standard methods. Analytical techniques such as, for example IR spectroscopy, show the presence of the solvent (or water) in the crystals as a solvate (or hydrate).

The Substituted Benzofuran Compounds can form salts which are also within the scope of this invention. Reference to a Substituted Benzofuran Compound herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a Substituted Benzofuran Compound contains both a basic moiety, such as, but not limited to a pyridine or imidazole, and an acidic moiety, such as, but not limited to a carboxylic acid, zwitterions ("inner salts") may be formed and are included within the term "salt(s)" as used herein. In one embodiment, the salt is a pharmaceutically acceptable (*i.e*., non-toxic, physiologically acceptable) salt. In another embodiment, the salt is other than a pharmaceutically acceptable salt. Salts of the Compounds of Formula (I) may be formed, for example, by reacting a Substituted Benzofuran Compound with an amount of acid or base, such as an equivalent amount, in a medium such as one in which the salt precipitates or in an aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates ("mesylates"), naphthalenesulfonates, nitrates, oxalates, phosphates, propionates, salicylates, succinates, sulfates, tartarates, thiocyanates, toluenesulfonates (also known as tosylates) and the like. In one embodiment, a compound of formula (I) is present as its dihydrochloride salt. In another embodiment, a compound of formula (I) is present as its dimesylate salt. Additionally, acids which are generally considered suitable for the formation of pharmaceutically useful salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; and in The Orange Book (Food & Drug Administration, Washington, D.C. on their website). Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamine, t-butyl amine, choline, and salts with amino acids such as arginine, lysine and the like. Basic nitrogen-containing groups may be quarternized with agents such as lower alkyl halides (*e.g*., methyl, ethyl, and butyl chlorides, bromides and iodides), dialkyl sulfates (*e.g*., dimethyl, diethyl, and dibutyl sulfates), long chain halides (*e.g*., decyl, lauryl, and stearyl chlorides, bromides and iodides), aralkyl halides (*e.g*., benzyl and phenethyl bromides), and others.

All such acid salts and base salts are intended to be pharmaceutically acceptable salts within the scope of the invention and all acid and base salts are considered equivalent to the free forms of the corresponding compounds for purposes of the invention.

Diastereomeric mixtures can be separated into their individual diastereomers on the basis of their physical chemical differences by methods well-known to those skilled in the art, such as, for example, by chromatography and/or fractional crystallization. Enantiomers can be separated by converting the enantiomeric mixture into a diastereomeric mixture by reaction with an appropriate optically active compound (*e.g*., chiral auxiliary such as a chiral alcohol or Mosher's acid chloride), separating the diastereomers and converting (*e.g*., hydrolyzing) the individual diastereomers to the corresponding pure enantiomers. Sterochemically pure compounds may also be prepared by using chiral starting materials or by employing salt resolution techniques. Also, some of the Substituted Benzofuran Compounds may be atropisomers (*e.g*., substituted biaryls) and are considered as part of this invention. Enantiomers can also be directly separated using chiral chromatographic techniques.

It is also possible that the Substituted Benzofuran Compounds may exist in different tautomeric forms, and all such forms are embraced within the scope of the invention. For example, all keto-enol and imine-enamine forms of the compounds are included in the invention.

All stereoisomers (for example, geometric isomers, optical isomers and the like) of the present compounds (including those of the salts), such as those which may exist due to asymmetric carbons on various substituents, including enantiomeric forms (which may exist even in the absence of asymmetric carbons), rotameric forms, atropisomers, and diastereomeric forms, are contemplated within the scope of this invention. If a Substituted Benzofuran Compound incorporates a double bond or a fused ring, both the cis- and trans-forms, as well as mixtures, are embraced within the scope of the invention.

Individual stereoisomers of the compounds of the invention may, for example, be substantially free of other isomers, or may be admixed, for example, as racemates or with all other, or other selected, stereoisomers. The chiral centers of the present invention can have the S or R configuration as defined by the *IUPAC* 1974 Recommendations. The use of the terms "salt", is intended to apply equally to the salt of enantiomers, stereoisomers, rotamers, tautomers, positional isomers or racemates of the inventive compounds.

In the Compounds of Formula (I), the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds of generic Formula I. For example, different isotopic forms of hydrogen (H) include protium (¹H) and deuterium (²H). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched Compounds of Formula (I) can be prepared without undue experimentation by conventional techniques well known to those skilled in the art or by processes analogous to those described in the Schemes and Examples herein using appropriate isotopically-enriched reagents and/or intermediates. In one embodiment, a Compound of Formula (I) has one or more of its hydrogen atoms replaced with deuterium.

Polymorphic forms of the Substituted Benzofuran Compounds, and of the salts of the Substituted Benzofuran Compounds, are intended to be included in the present invention.

The following abbreviations are used below and have the following meanings: Ac is acyl; AcOH is acetic acid; BOC or Boc is *tert*-butyloxycarbonyl; Boc₂O is Boc anhydride; dba is dibenzylideneacetone; DCM is dichloromethane; DMA is *N,N-*dimethylacetamide; DMF is *N,N*-dimethylformamide; dppf is diphenylphosphinoferrocene; dppm is bis(diphenylphosphino)methane; EDCI is 1-ethyl-3-(3-dimethylaminopropyl)carbodiimide; EtOAc is ethyl acetate; Et₂O is diethyl ether; Et₃N is triethylamine; HOBT is 1-hydroxy 1H-benzotriazole; HPLC is high performance liquid chromatography; HRMS is high resolution mass spectrometry; KOAc is potassium acetate; LCMS is liquid chromatography/mass spectrometry; MeOH is methanol; PdCl₂(dppf)₂ is [1,1'-Bis(diphenylphosphino)ferrocene]dichloro palladium(II); PE is petroleum ether; pinacol₂B₂ is bis(pinacolato)diboron; p-TsOH is p-toluenesulfonic acid; TFA is trifluoroacetic acid; THF is tetrahydrofuran; and TLC is thin-layer chromatography.

### The Compounds of Formula (I)

The present invention provides Substituted Benzofuran Compounds of Formula (I): 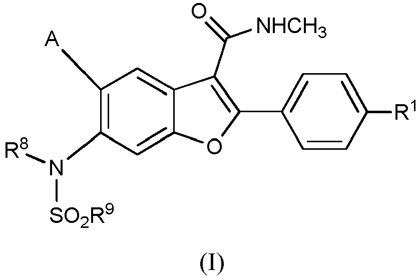 wherein A, R¹, R⁴, R^{4a}, R⁵, R^{8,} R⁹ and R¹⁰ are as defined above for the Compounds of Formula (I).

In one embodiment, R¹ represents a single halo substituent.

In another embodiment, R¹ represents a single F substituent.

In one embodiment, for the compounds of formula (I), R¹ is a single F group; R⁸ and R⁹ are each methyl; and each occurrence of R¹⁰ is independently H or F.

In one embodiment, R⁸ and R⁹ are each methyl.

In another embodiment of the invention, the compound of the invention is one of compounds: 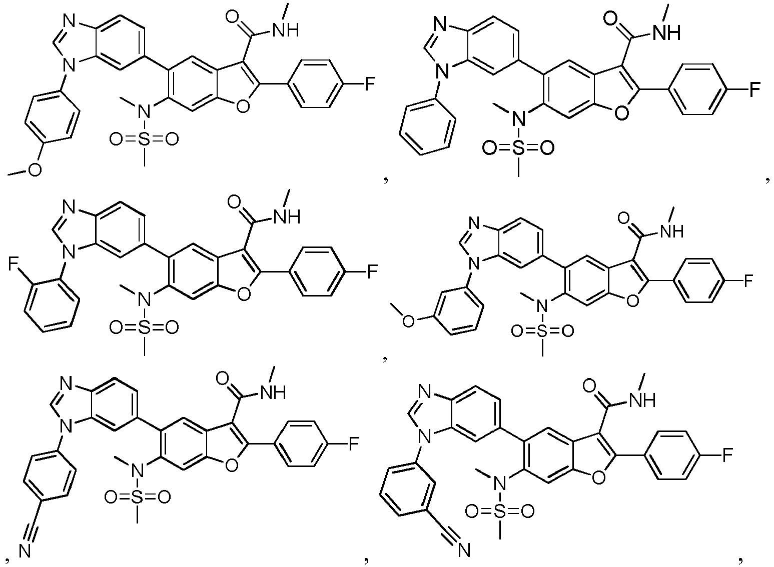 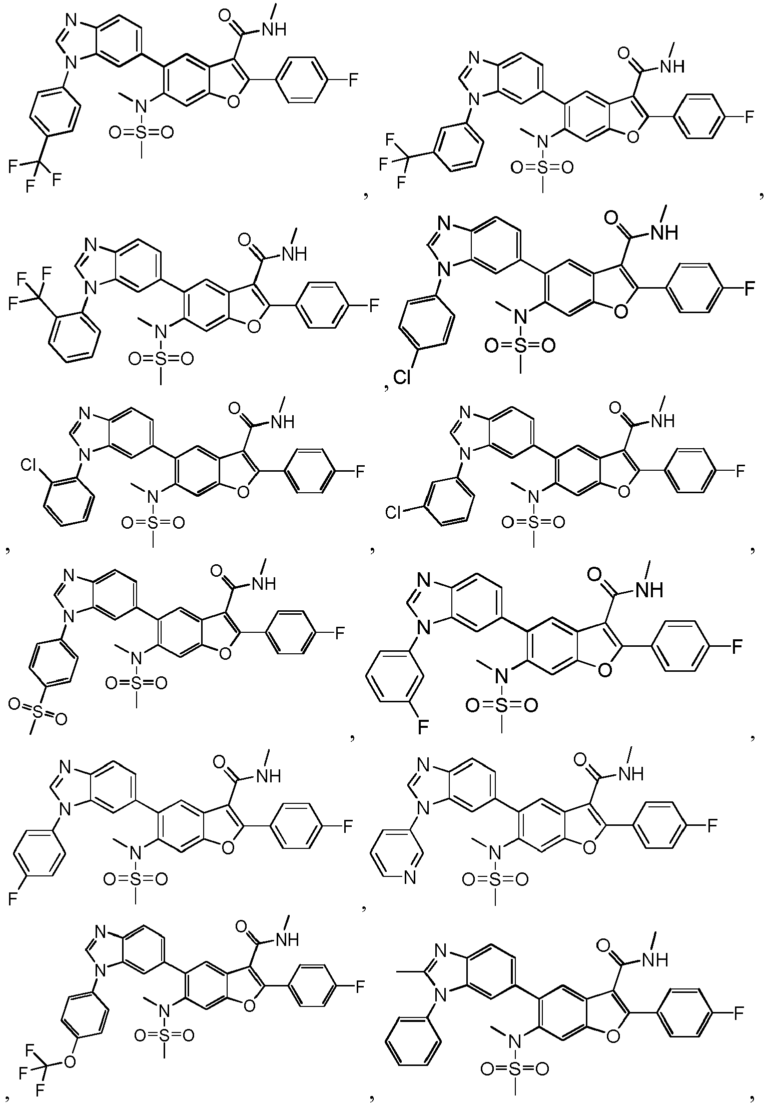 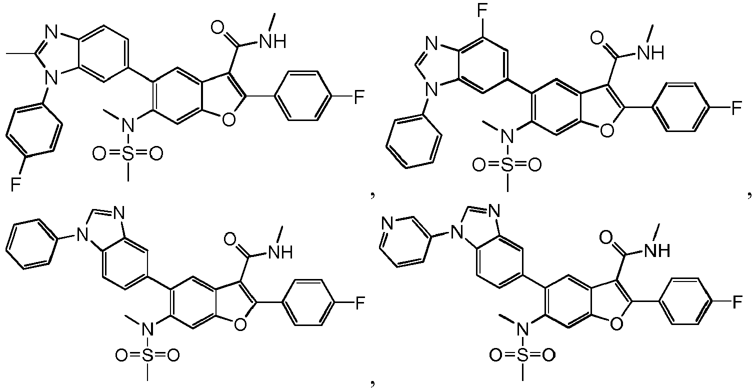 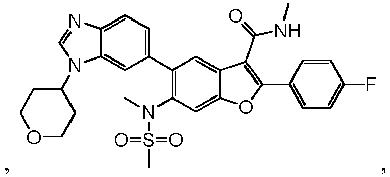 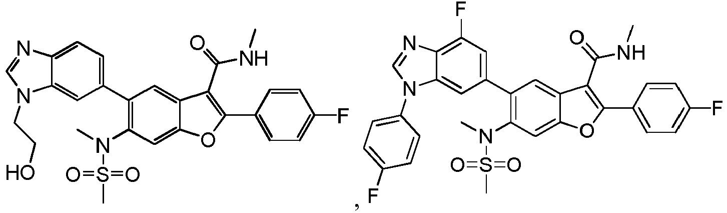 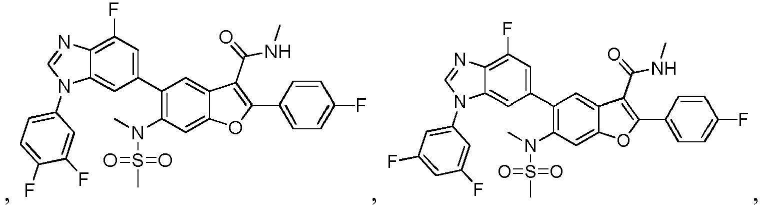 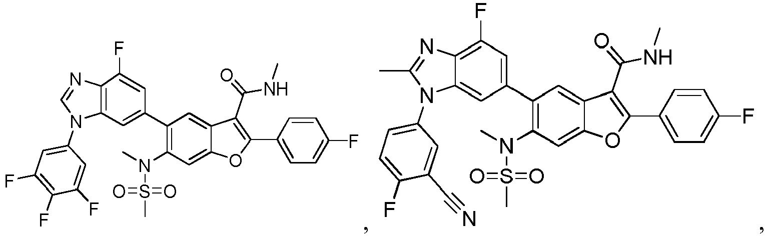 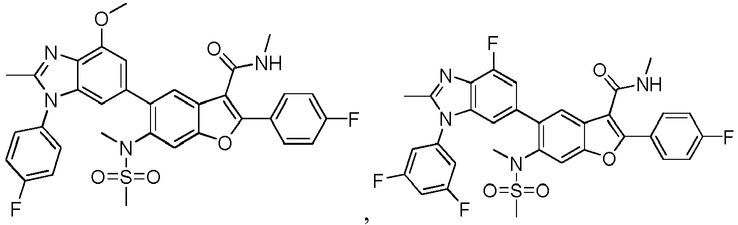 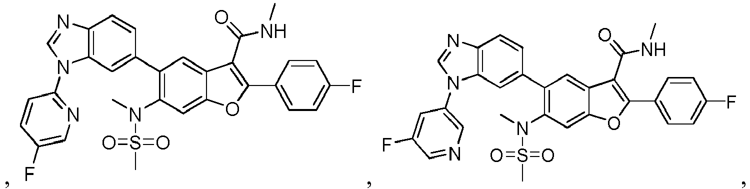 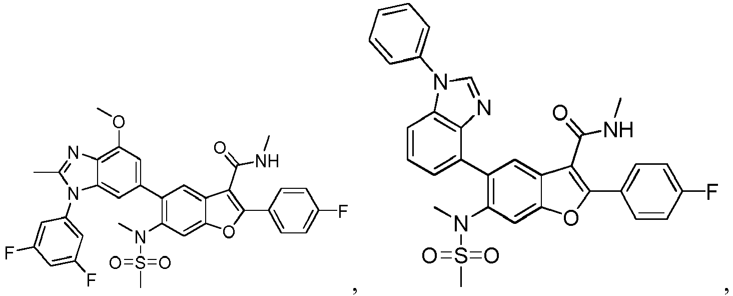 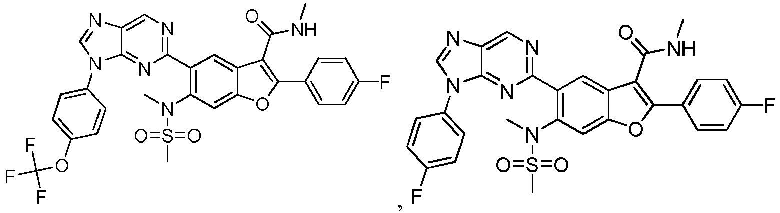 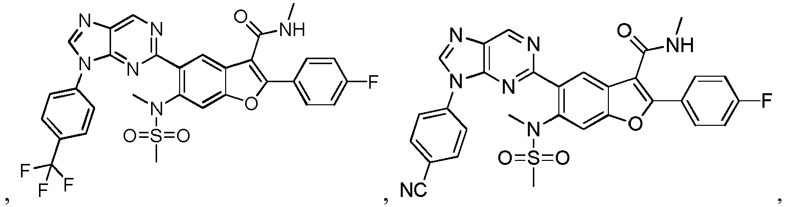 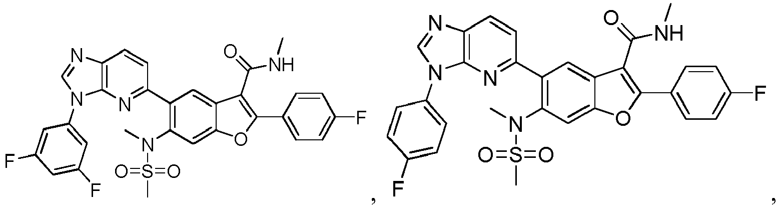 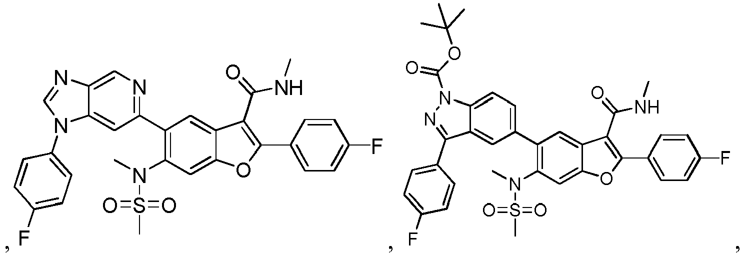 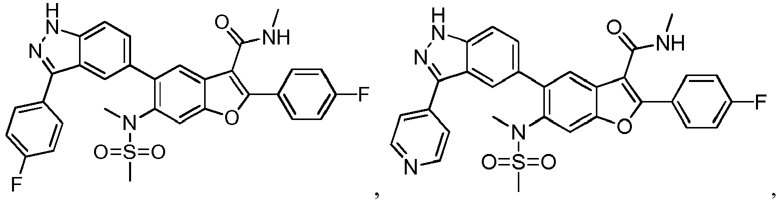 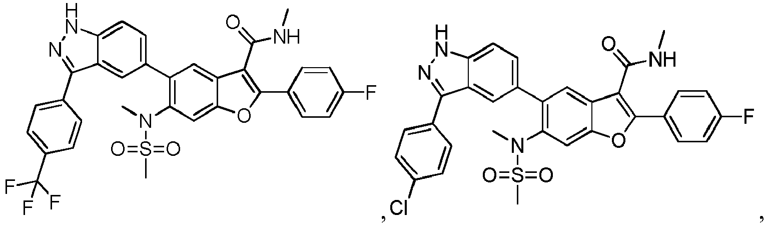 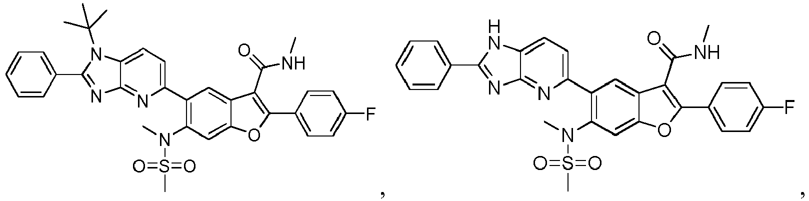 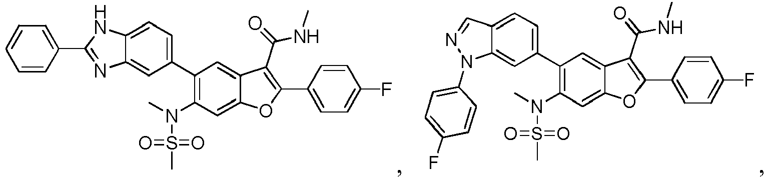 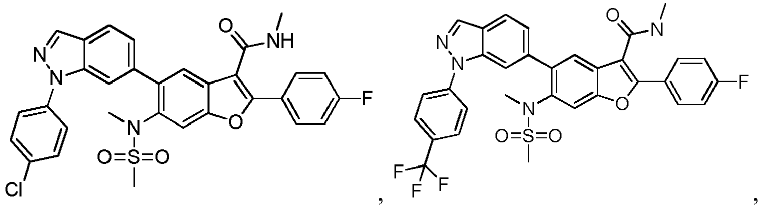 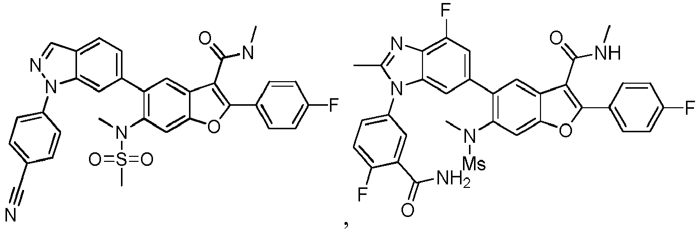 and 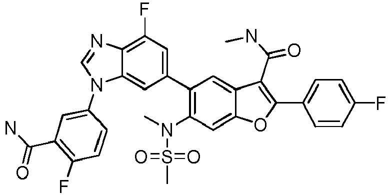 or a pharmaceutically acceptable salt thereof.

Other embodiments of the present invention include the following:
(a) A pharmaceutical composition comprising an effective amount of a compound of formula (I) and a pharmaceutically acceptable carrier.
(b) The pharmaceutical composition of (a), further comprising a second therapeutic agent selected from the group consisting of HCV antiviral agents, immunomodulators, and anti-infective agents.
(c) The pharmaceutical composition of (b), wherein the HCV antiviral agent is an antiviral selected from the group consisting of direct inhibitors of HCV, including but not limited to NS3 and NS3/4A protease inhibitors, NS5A inhibitors and HCV NS5B polymerase inhibitors.
(d) A pharmaceutical combination that is (i) a compound of formula (I) and (ii) a second therapeutic agent selected from the group consisting of HCV antiviral agents, immunomodulators, and anti-infective agents; wherein the compound of formula (I) and the second therapeutic agent are each employed in an amount that renders the combination effective for inhibiting HCV NS5B activity, or for inhibiting HCV viral replication, or for treating HCV infection and/or reducing the likelihood or severity of symptoms of HCV infection.
(e) The combination of (d), wherein the HCV antiviral agents are one or more antiviral agents selected from the group consisting of direct inhibitors of HCV, including NS3 and NS3/4A protease inhibitors, NS5A inhibitors and HCV NS5B polymerase inhibitors.
(f) A compound of formula (I) for use in inhibiting HCV NS5B activity in a subject.
(g) A compound of formula (I) for use in preventing and/or treating infection by HCV in a subject in need thereof.
(h) The compound for use of (g), wherein the compound of formula (I) is administered in combination with an effective amount of at least one second therapeutic agent selected from the group consisting of HCV antiviral agents, immunomodulators, and anti-infective agents.
(i) The compound for use of (h), wherein the HCV antiviral agent is an antiviral selected from the group consisting of direct inhibitors of HCV, including NS3 and NS3/4A protease inhibitors, NS5A inhibitors and HCV NS5B polymerase inhibitors.
(j) A compound of formula (I) for use in inhibiting HCV viral replication and/or HCV viral production in a cell-based system, which comprises administering to the subject an effective amount of a compound of formula (I) in combination with an effective amount of at least one second therapeutic agent selected from the group consisting of HCV antiviral agents, immunomodulators, and anti-infective agents.
(k) The compound for use of (j), wherein the HCV antiviral agent is an antiviral selected from the group consisting of direct inhibitors of HCV, including NS3 and NS3/4A protease inhibitors, NS5A inhibitors and HCV NS5B polymerase inhibitors.
(l) A compound of formula (I) for use in inhibiting HCV NS5B activity in a subject, which comprises administering to the subject the pharmaceutical composition of (a), (b), or (c) or the combination of (d) or (e).
(m) A compound of formula (I) for use in treating HCV infection and/or reducing the likelihood or severity of symptoms of HCV infection in a subject, which comprises administering to the subject the pharmaceutical composition of (a), (b), or (c) or the combination of (d) or (e).

In the embodiments of the compounds and salts provided above, it is to be understood that each embodiment may be combined with one or more other embodiments, to the extent that such a combination provides a stable compound or salt and is consistent with the description of the embodiments. It is further to be understood that the embodiments of compositions and treatments provided as (a) through (m) above are understood to include all embodiments of the compounds and/or salts, including such embodiments as result from combinations of embodiments.

Additional embodiments of the invention include the pharmaceutical compositions, combinations, uses and treatments set forth in (a) through (m) above, wherein the compound of the present invention employed therein is a compound of one of the embodiments, aspects, classes, sub-classes, or features of the compounds described above. In all of these embodiments, the compound may optionally be used in the form of a pharmaceutically acceptable salt or hydrate as appropriate.

### Methods For Making the Compounds of Formula (I)

The Compounds of Formula (I) may be prepared from known or readily prepared starting materials, following methods known to one skilled in the art of organic synthesis. Methods useful for making the Compounds of Formula (I) are set forth in the Examples below and generalized in Schemes 1-5 below. Alternative synthetic pathways and analogous structures will be apparent to those skilled in the art of organic synthesis. All stereoisomers and tautomeric forms of the compounds are contemplated.

Some commercially available starting materials and intermediates used for the synthesis of the Compounds of Formula (I) are available which contain intact fused polycyclic bicyclic ring systems. These starting materials and intermediates are available from commercial suppliers such as Sigma-Aldrich (St. Louis, MO) and Acros Organics Co. (Fair Lawn, NJ). Such starting materials and intermediates compounds are used as received.

Scheme 1 shows a method useful for making compounds of formula **F**, which correspond to the Compounds of Formula (I), wherein R¹ is F; R⁸ and R⁹ are each methyl; and R¹⁰ is H. 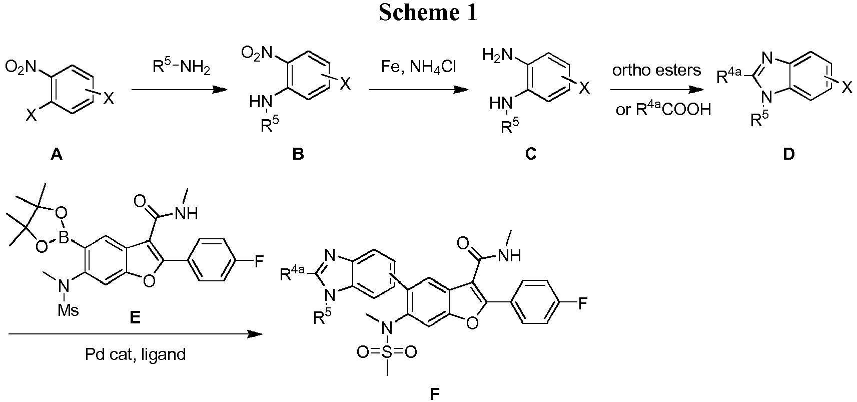 Wherein R^{4a} and R⁵ are defined above for the Compounds of Formula (I).

Starting from compound **A** (X = Cl, Br or I,) which coupling with amines (R₁ = Ar or Alk) afford compounds of formula **B**. Compoundsfof formula **C** can be generated by reduction of the nitro group in compounds **B**, and the amino groups in compounds **C** is then cyclized with ortho esters or carboxylic acids to furnish compounds **D** (R = H, Alk or Ar). Transition metal mediated coupling of compounds **D** with compound **E** provides the target compounds of general structure **F**.

Scheme 2 shows an alternate method useful for making compounds of formula **F**, which correspond to the Compounds of Formula (I), wherein R¹ is F; R⁸ and R⁹ are each methyl; and R¹⁰ is H

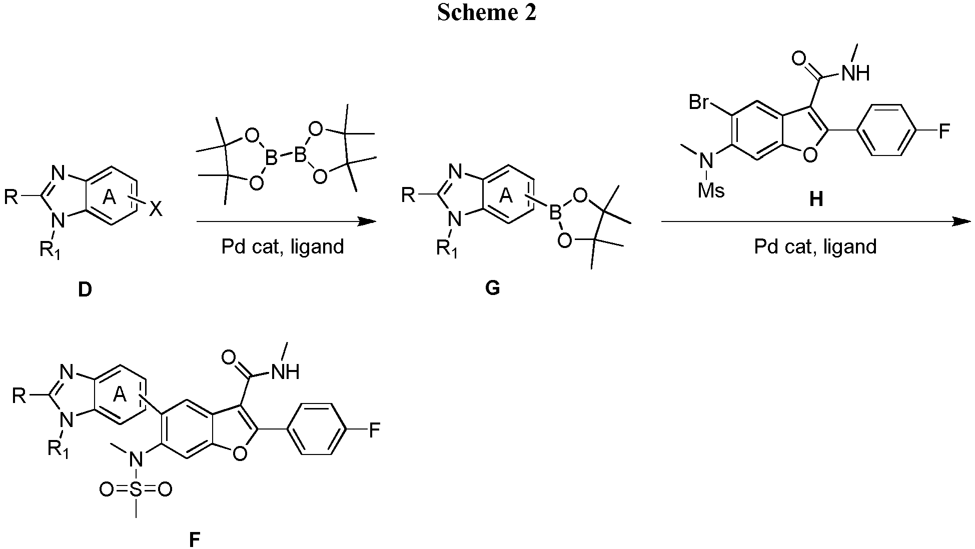

Compounds of formula **D** can be converted to corresponding boronic esters **G** by reacting with bis(pinacolato)diboron in the presence of a palladium catalyst. Compounds of formula G can then be coupled with a brominated benzofuran compound of formula H to provide compounds of formula **F**.

Scheme 3 (illustrative) shows a method useful for making compounds of formula **K**, which correspond to the Compounds of Formula (I), wherein R¹ is F and R⁸ and R⁹ are each methyl.

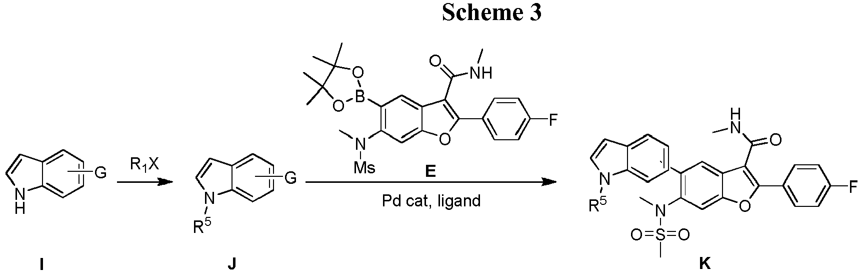

Compounds of formula **I** (A = Cl, Br or I) can be converted to compounds of formula **J** (R⁵ = alkyl, aryl or heteroaryl) in the presence of transition metal catalyst or alkylation condition. A compound of formula **J** can then be reacted with a compound of formula **E** to provide the compounds of formula **K**.

Scheme 4 shows a method useful for making compounds of formula **P**, which correspond to the Compounds of Formula (I), wherein R¹ is F and R⁸ and R⁹ are each methyl; and R¹⁰ is H.

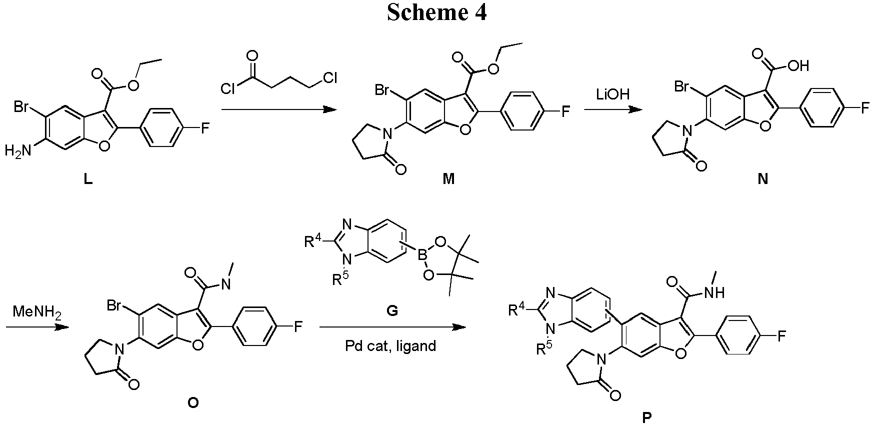

Compound **L** can be reacted with treated with 4-chlorobutanoyl chloride to provide compound **M**. Base-catalyzed hydrolysis of **M** provides carboxylic acid compound **N**, which can be condensed with methanamine using common amide forming reagents to provide compound **O**. Transition metal mediated coupling of compound **O** with compound **G** provides the compounds of formula **P**.

Scheme 5 shows a method useful for making compound **E**, which is a intermediate useful for making the Compounds of Formula (I).

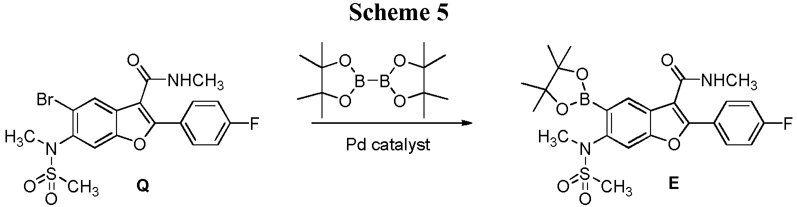

Intermediate boronic acid compound **E** can be made by reacting bromo-substitued benzofuran compound Q with bis(pinacolato)diboron in the presence of an appropriate palladium catalyst.

One skilled in the art of organic synthesis will recognize that the synthesis of compounds with multiple reactive functional groups, such as -OH and NH₂, may require protection of certain functional groups (*i.e*., derivatization for the purpose of chemical compatibility with a particular reaction condition). Suitable protecting groups for the various functional groups of these compounds and methods for their installation and removal are well-known in the art of organic chemistry. A summary of many of these methods can be found in Greene & Wuts, Protecting Groups in Organic Synthesis, John Wiley & Sons, 3rd edition (1999).

One skilled in the art of organic synthesis will also recognize that one route for the synthesis of the Compounds of Formula (I) may be more desirable depending on the choice of appendage substituents. Additionally, one skilled in the relevant art will recognize that in some cases the order of reactions may differ from that presented herein to avoid functional group incompatibilities and thus adjust the synthetic route accordingly.

Compounds of formula **E**, **F**, **K** and **P** may be further elaborated using methods that would be well-known to those skilled in the art of organic synthesis or, for example, the methods described in the Examples below, to make the full scope of the Compounds of Formula (I).

The starting materials used and the intermediates prepared using the methods set forth above in Schemes 1-5 may be isolated and purified if desired using conventional techniques, including but not limited to filtration, distillation, crystallization, chromatography and alike. Such materials can be characterized using conventional means, including physical constants and spectral data.

### EXAMPLES

### General Methods

The compounds described herein can be prepared according to the procedures of the following schemes and examples, using appropriate materials and are further exemplified by the following specific examples. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. All temperatures are degrees Celsius unless otherwise noted. Mass spectra (MS) were measured by electrospray ion-mass spectroscopy (ESI). ¹H NMR spectra were recorded at 400-500 MHz. Compounds described herein were synthesized as a racemic mixture unless otherwise stated in the experimental procedures.

### Example 1

### Preparation of Compound 1

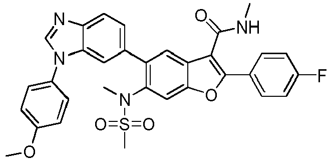

### Step 1 - Synthesis of 5-bromo-N-(4-methoxyphenyl)-2-nitroaniline

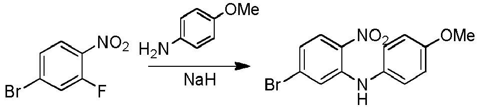

To a solution of 4-methoxyaniline (560 mg, 4.6 mmol) in anhydrous THF (10 mL) was added NaH (273 mg, 6.8 mmol) little by little at ice-bath, and then the mixture was allowed to stir at room temperature for 30 minutes. After 4-bromo-2-fluoro-1-nitrobenzene (1.0 g, 4.6 mmol) in THF was added dropwise, the mixture was allowed to stir at 30∼40 °C overnight. After the reaction mixture was quenched with water, the mixture was extracted with EtOAc. The organic phase was dried with Na₂SO₄ and concentrated in vacuo. The resulting residue was purified using column chromatography (eluted with PE : EtOAc = 20 : 1) to provide 5-bromo-N-(4-methoxyphenyl)-2-nitroaniline (1.0 g, yield: 68.2%). ¹H-NMR (CDCl₃, 400 MHz) δ 9.34 (s, 1H), 7.98∼8.00 (m, 1H), 7.26∼7.35 (m, 2H), 6.73∼6.82 (m, 3H), 3.78 (s, 3H). (M+H)⁺: 323 / 325.

### Step 2 - Synthesis of 5-bromo-N¹-(4-methoxyphenyl)benzene-1,2-diamine

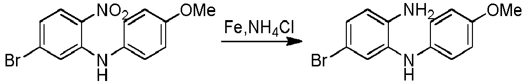

A mixture of 5-bromo-N-(4-methoxyphenyl)-2-nitroaniline (1.0 g, 3.1 mmol), Fe (518 mg, 9.3 mmol) and NH₄Cl (993 mg, 18.6 mmol) in THF:MeOH:H₂O (10 mL : 10 mL : 10 mL) was refluxed for 3 hours. After the reaction mixture was filtrated, the filtrate was concentrated, the resulting residue was purified using column chromatography eluted with PE : EtOAc = 20 : 1 to provide 5-bromo-N¹-(4-methoxyphenyl)benzene-1,2-diamine (0.6 g, yield: 66.2%). ¹H-NMR (CDCl₃, 400 MHz) δ 7.35 (s, 1H), 7.03 (s, 1H), 6.90∼6.92 (m, 1H), 6.76∼6.80 (m, 3H), 6.57∼6.59 (m, 1H), 4.96 (s, 1H), 3.72 (s, 3H), 4.96 (s, 2H). (M+H)⁺: 293 / 295.

### Step 3 - Synthesis of 6-bromo-1-(4-methoxyphenyl)-1H-benzo[d]imidazole

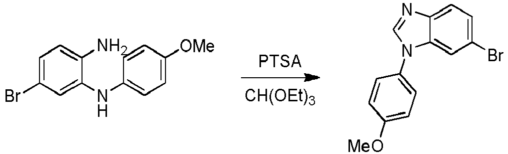

A mixture of 5-bromo-N¹-(4-methoxyphenyl)benzene-1,2-diamine (300 mg, 1.0 mmol) and p-TsOH (194 mg, 1.0 mmol) in CH(OCH₃)₃ (10 mL) was refluxed for 3 hours. After the reaction mixture was diluted with EtOAc, the mixture was washed with saturated NaHCO₃, dried (Na₂SO₄) and concentrated in vacuo. The resulting residue was purified using column chromatography (eluted with PE : EtOAc = 12 : 1) to provide 6-bromo-1-(4-methoxyphenyl)-1H-benzo[d]imidazole (180 g, yield: 58.1%). ¹H-NMR (CDCl₃, 400 MHz) δ 8.03 (s, 1H), 7.63∼7.65 (m, 2H), 7.32∼7.36 (m, 2H), 6.94∼6.99 (m, 3H), 3.81 (s, 3H). (M+H)⁺: 303 / 305.

### Step 4 - Synthesis of 2-(4-fluorophenyl)-5-(1-(4-methoxyphenyl)-1H-benzo[d]imidazol-6-yl)-N-methyl-6-(N-methylmethylsulfonamido)benzofuran-3-carboxamide

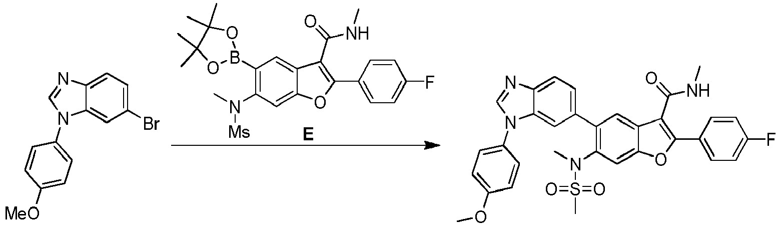

To a degassed solution of 6-bromo-1-(4-methoxyphenyl)-1H-benzo[d]imidazole (85 mg, 0.28 mmol) and 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (Compound E, 100 mg, 0.20 mmol) in 1, 4-dioxane (2 mL) was added Pd(dppf)Cl₂ (10 mg) and K₃PO₄ (85 mg, 0.4 mmol) under N₂. The mixture was heated to 100 °C overnight. The reaction mixture was cooled to room temperature and filtered. The filtrate was washed with H₂O, brine, dried over Na₂SO₄. After being concentrated in vacuo, the resulting residue was purified using prep-TLC to provide 2-(4-fluorophenyl)-5-(1-(4-methoxyphenyl)-1H-benzo[d]imidazol-6-yl)-N-methyl-6-(N-methylmethylsulfonamido)benzofuran-3-carboxamide (45 mg, yield: 26.8%). ¹H-NMR (CDCl₃, 400 MHz) δ 8.95 (s, 1H), 8.01 (d, *J* = 8.0 Hz, 1H), 7.76∼7.85 (m, 4H), 7.49∼7.56 (m, 4H), 7.13∼7.17 (m, 2H), 7.05 (d, *J* = 8.0 Hz, 2H), 5.81 (s, 1H), 3.83 (s, 3H), 2.97 (s, 3H), 2.90 (d, *J* = 4.4 Hz, 3H), 2.82 (s, 3H). (M+H)⁺: 599.

Compounds **2-38**, depicted in the table below, were prepared using the method described above and substituting the appropriate reactants and reagents.

| **Compound** | **Structure** | **NMR** | **MS (M+H)⁺** |
|---|---|---|---|
| **2** | 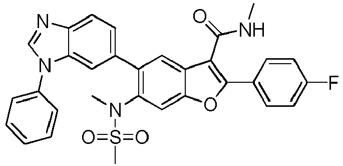 | ¹H-NMR (CDCl₃, 400 MHz) δ 9.19 (s, 1H), 8.01 (d, *J* = 8.4 Hz, 1H), 7.78∼7.84 (m, 4H), 7.55∼7.64 (m, 6H), 7.48 (s, 1H), 7.14 (t, *J* = 8.4 Hz, 2H), 5.95∼6.08 (br, 1H), 2.97 (s, 3H), 2.87 (d, *J =* 4.8 Hz, 3H), 2.85 (s, 3H). | **569** |
| **3** | 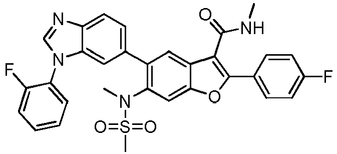 | ¹H-NMR (400 MHz, CDCl₃) δ 8.52 (s, 1H), 7.96 (d, *J* = 8.4 Hz, 1H), 7.84∼7.85 (m, 2H), 7.77 (s, 1H), 7.60∼7.63 (m, 1H), 7.56 (s, 1H), 7.52 (s, 1H), 7.45∼7.47 (m, 2H), 7.31∼7.36 (m, 2H), 7.12∼7.16 (m, 2H), 5.74 (d, *J =* 4.4 Hz, 1H), 3.01 (s, 3H), 2.90 (d, *J =* 4.8 Hz, 3H), 2.94 (s, 3H). | **587** |
| **4** | 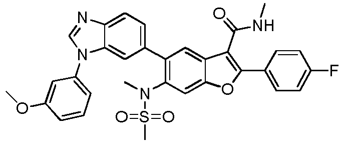 | ¹H-NMR (400 MHz, CDCl₃) δ 8.95 (s, 1H), 8.01 (d, *J* = 8.0 Hz, 1H), 7.76∼7.85 (m, 4H), 7.49∼7.56 (m, 4H), 7.13∼7.17 (m, 2H), 7.05 (d, *J* = 8.0 Hz, 2H), 5.81 (s, 1H), 3.83 (s, 3H), 2.97 (s, 3H), 2.90 (d, *J* = 4.4 Hz, 3H), 2.82 (s, 3H). | **599** |
| **5** | 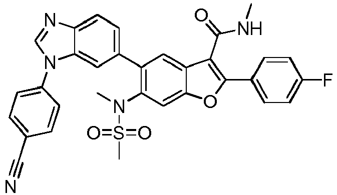 | ¹H-NMR (CDCl₃, 400 MHz) δ 8.54 (s, 1H), 7.47 (d, *J* = 8.4 Hz, 1H), 7.90∼7.77 (m, 8H), 7.48 (s, 1H), 7.45 (d, *J =* 7.6 Hz, 1H), 7.20∼7.13 (m, 2H), 5.75 (d, *J* = 5.2 Hz, 1H), 2.90∼2.96 (m, 9H). | **594** |
| **6** | 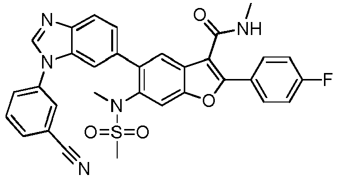 | ¹H-NMR (CDCl₃, 400 MHz) δ 8.68 (s, 1H), 8.04 (s, 1H), 7.99 (d, *J* = 8.4 Hz, 1H), 7.96∼7.73 (m, 7H), 7.52 (d, *J =* 10.0 Hz, 2H), 7.20 (t, *J* = 8.4 Hz, 2H), 6.00 (s, 1H), 2.99∼2.95 (m, 9H). | **594** |
| **7** | 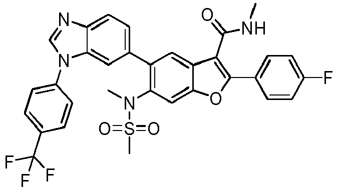 | ¹H-NMR (CDCl₃, 400 MHz) δ 8.49 (d, *J* = 7.6 Hz, 1H), 7.94 (d, *J* = 7.2 Hz, 1H), 7.81∼7.88 (m, 6H), 7.75 (d, *J* = 8.0 Hz, 2H), 7.50 (s, 1H), 7.43 (d, *J =* 8.4 Hz, 1H), 7.15 (t, *J =* 8.8 Hz, 2H), 5.74 (s, 1H), 2.97 (s, 3H), 2.90 (d, *J* = 4.8 Hz, 3H), 2.84 (s, 3H). | **637** |
| **8** | 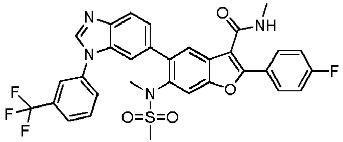 | ¹H-NMR (CDCl₃, 400 MHz) δ 8.14 (s, 1H), 7.90∼7.86 (m, 3H), 7.81 (d, *J* = 6.8 Hz, 1H), 7.77 (d, *J* = 6.0 Hz, 2H), 7.70∼7.66 (m, 3H), 7.51 (d, *J* = 4.0 Hz, 1H), 7.36 (d, *J* = 9.6 Hz, 1H), 7.16∼7.11 (m, 2H), 5.75 (s, 1H), 3.00 (s, 3H), 2.90 (d, *J* = 1.2 Hz, 3H), 2.70 (s, 3H). | **637** |
| **9** | 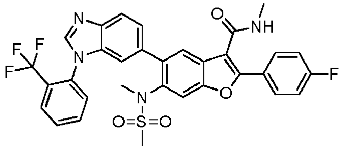 | ¹H-NMR (CDCl₃, 400 MHz) δ 8.05 (s, 1H), 7.95∼7.90 (m, 4H), 7.79 (s, 2H), 7.69 (s, 1H), 7.57 (d, *J* = 8.4 Hz, 2H), 7.38 (dd, *J₁* = 1.2 Hz, *J₂* = 1.6 Hz, 1H), 7.23∼7.17 (m, 3H), 5.82 (s, 1H), 3.04 (s, 3H), 2.96 (d, *J =* 5.2 Hz, 3H), 2.49 (s, 3H). | **637** |
| **10** | 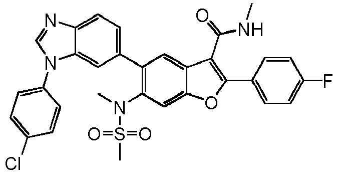 | ¹H-NMR (CDCl₃, 400 MHz) δ 8.86 (s, 1H), 8.02 (d, *J* = 8.4 Hz, 1H), 7.84∼7.90 (m, 4H), 7.53∼7.63 (m, 6H), 7.17∼7.22 (m, 2H), 5.86 (br s, 1H), 2.92∼2.99 (m, 9H). | **603** |
| **11** | 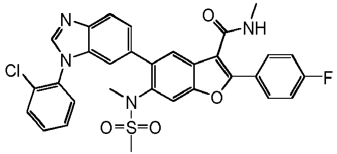 | ¹H-NMR (CDCl₃, 400 MHz) δ 8.85 (s, 1H), 8.01 (s, 1H), 7.78∼7.86 (m, 3H), 7.64 (s, 2H), 7.52∼7.54 (m, 5H), 7.14∼7.18 (m, 2H), 6.03 (s, 1H), 3.01 (s, 3H), 2.92 (d, *J* = 4.0 Hz, 3H), 2.73 (s, 3H). | **603** |
| **12** | 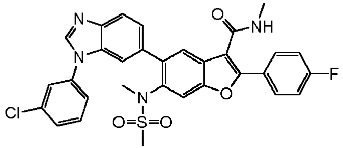 | ¹H-NMR (CDCl₃, 400 MHz) δ 8.46 (s, 1H), 7.93 (d, *J* = 8.0 Hz, 1H), 7.85∼7.88 (m, 2H), 7.79 (s, 1H), 7.74 (s, 1H), 7.56 (s, 1H), 7.48 (d, *J* = 8.0 Hz, 3H), 7.43 (d, *J* = 9.2 Hz, 2H), 7.15 (t, *J* = 8.4 Hz, 2H), 5.75 (t, *J* = 4.0 Hz, 1H), 3.00 (s, 3H), 2.90 (d, *J* = 4.8 Hz, 3H), 2.75 (s, 3H). | **603** |
| **13** | 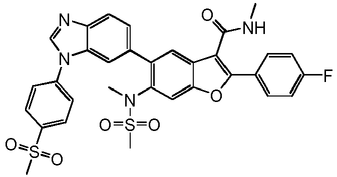 | ¹H-NMR (CDCl₃, 400 MHz) δ 8.24 (s, 1H), 8.18 (d, *J* = 8.4 Hz, 2H), 7.96-7.87 (m, 7H), 7.56 (s, 1H), 7.43 (d, *J* = 8.0 Hz, 1H), 7.24∼7.19 (m, 2H), 5.82 (s, 1H), 3.12 (s, 3H), 3.04 (s, 3H), 2.96 (d, *J* = 4.8 Hz, 3H), 2.90 (s, 3H). | **647** |
| **14** | 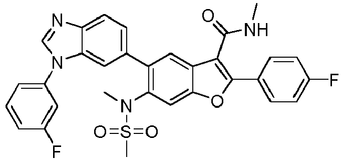 | ¹H-NMR (CDCl₃, 400 MHz) δ 8.02 (s, 1H), 7.80∼7.88 (m, 3H), 7.74 (s, 1H), 7.59 (s, 1H), 7.46∼7.50 (m, 3H), 7.30 (d, *J* = 1.2 Hz, 1H), 7.16∼7.20 (m, 2H), 7.09∼7.14 (m, 2H), 6.09 (s, 1H), 2.94 (s, 3H), 2.90 (d, *J* = 4.0 Hz, 3H), 2.66 (m, 3H). | **587** |
| **15** | 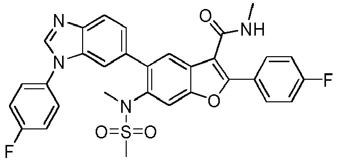 | ¹H-NMR (CDCl₃, 400 MHz) δ 8.09 (s, 1H), 7.82∼7.88 (m, 3H), 7.75 (s, 1H), 7.67 (s, 1H), 7.45∼7.50 (m, 2H), 7.31∼7.35 (m, 2H), 7.26 (d, *J* = 8.0 Hz, 1H), 7.07∼7.14 (m, 3H), 5.94 (s, 1H), 2.99 (s, 3H), 2.89 (d, *J* = 4.0 Hz, 3H), 2.66 (m, 3H). | **587** |
| **16** | 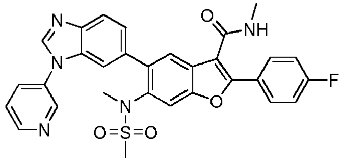 | ¹H-NMR (Methanol-*d*4, 400 MHz) δ 9.40 (s, 1H), 9.10 (s, 1H), 8.81 (d, *J* = 1.2 Hz, 1H), 8.39∼8.42 (m, 1H), 7.95∼7.98 (m, 4H), 7.88 (s, 1H), 7.79∼7.81 (m, 1H), 7.70∼7.77 (m, 2H), 7.27∼7.31 (m, 2H), 3.09 (s, 3H), 3.06 (s, 3H), 2.93 (m, 3H). | **570** |
| **17** | 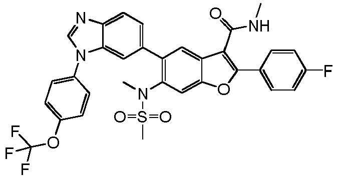 | ¹H-NMR (CDCl₃, 400 MHz) δ 8.61 (s, 1H), 7.96 (d, *J* = 8.4 Hz, 1H), 7.83∼7.87 (m, 2H), 7.78∼7.82 (m, 2H), 7.65 (d, *J* = 8.8 Hz, 2H), 7.49 (s, 1H), 7.46 (d, *J* = 8.8 Hz, 1H), 7.41 (d, *J* = 8.8 Hz, 2H), 7.15 (t, *J* = 8.4 Hz, 2H), 5.74 (d, *J* = 7.2 Hz, 1H), 2.96 (s, 3H), 2.90 (d, *J* = 4.8 Hz, 3H), 2.84 (s, 3H). | **653** |
| **18** | 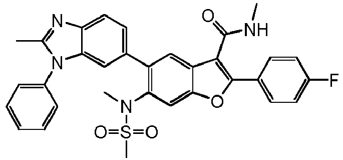 | ¹H-NMR (CDCl₃, 400 MHz) δ 7.84∼7.88 (m, 2H), 7.70 (t, *J* = 3.2 Hz, 2H), 7.49∼7.53 (m, 3H), 7.43 (t, *J* = 7.2 Hz, 1H), 7.34 (d, *J* = 7.2 Hz, 2H), 7.23 (d, *J* = 1.6 Hz, 1H), 7.13 (s, 1H), 7.09 (t, *J* = 6.8 Hz, 1H), 5.87 (s, 1H), 2.98 (s, 3H), 2.90 (d, *J* = 4.8 Hz, 3H), 2.50 (s, 3H), 2.48 (s, 3H). | **583** |
| **19** | 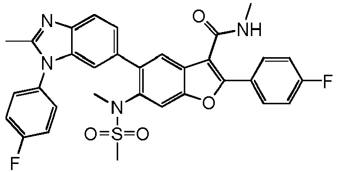 | ¹H-NMR (CDCl₃, 400 MHz) δ 7.85∼7.88 (m, 2H), 7.72 (s, 1H), 7.70 (s, 1H), 7.50 (s, 1H), 7.33∼7.36 (m, 2H), 7.22∼7.25 (m, 4H), 7.13 (t, *J* = 8.8 Hz, 2H), 5.77 (s, 1H), 2.96 (s, 3H), 2.90 (d, *J* = 4.8 Hz, 3H), 2.58 (s, 3H), 2.47 (s, 3H). | **601** |
| **20** | 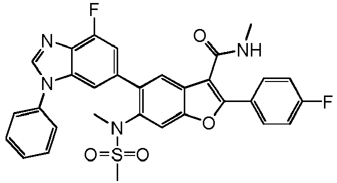 | ¹H-NMR (CDCl₃, 400 MHz) δ 8.29 (s, 1H), 7.85 (t, *J* = 8.8 Hz, 2H), 7.78 (s, 1H), 7.51∼7.54 (m, 5H), 7.49 (s, 1H), 7.44∼7.46 (m, 1H), 7.09∼7.16 (m, 3H), 5.74 (s, 1H), 3.02 (s, 3H), 2.91 (d, *J* = 4.8 Hz, 3H), 2.75 (s, 3H). | **587** |
| **21** | 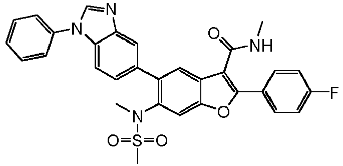 | ¹H-NMR (400 MHz, CDCl₃) δ 9.05 (s, 1H), 8.12 (s, 1H), 7.95-7.99 (m, 2H), 7.87 (s, 1H), 7.77-7.79 (m, 1H), 7.69∼7.73 (m, 4H), 7.61-7.62 (m, 3H), 7.20∼7.24 (m, 2H), 6.01∼6.02 (br, 1H), 3.19 (s, 3H), 3.01-3.02 (d, *J* = 4.0 Hz, 3H), 2.85 (s, 3H). | **569** |
| **22** | 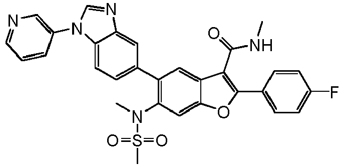 | ¹H-NMR (CDCl₃, 400 MHz) δ 8.99 (s, 1H), 8.89 (d, *J* = 4.0 Hz, 1H), 8.79 (s, 1H), 8.06 (s, 2H), 7.94∼7.97 (m, 2H), 7.87 (s, 1H), 7.72 (s, 1H), 7.70 (s, 1H), 7.64 (s, 1H), 7.62 (s, 1H), 7.20∼7.24 (m, 2H), 5.99 (s, 1H), 3.18 (s, 3H), 3.01 (d, *J* = 4.0 Hz, 3H), 2.81 (s, 3H). | **570** |
| **23** | 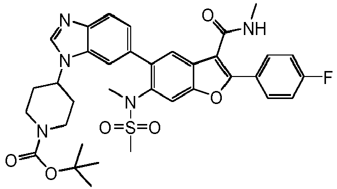 | ¹H-NMR (CDCl₃, 400 MHz) δ 7.98 (s, 1H), 7.86∼7.90 (m, 2H), 7.78∼7.82 (m, 2H), 7.65 (s, 1H), 7.51 (s, 1H), 7.27 (d, *J* = 8.4 Hz, 1H), 7.12∼7.19 (m, 2H), 5.84 (s, 1H), 4.31∼4.38 (m, 3H), 2.87∼2.94 (m, 8H), 2.68 (s, 3H), 2.16∼2.19 (m, 2H), 1.90∼1.93 (m, 2H), 1.42 (s, 9H). | 676 |
| **24** | 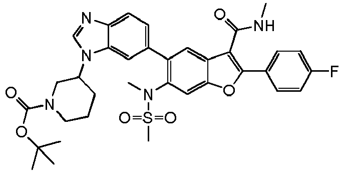 | ¹H-NMR (CDCl₃, 400 MHz) δ 9.08 (s, 1H), 7.96 (s, 2H), 7.85∼7.89 (m, 3H), 7.56 (d, *J* = 8.4 Hz, 1H), 7.51 (s, 1H), 7.14∼7.17 (m, 2H), 5.84 (br s, 1H), 4.51 (s, 1H), 4.32 (s, 1H), 3.88∼3.90 (m, 1H), 3.37 (s, 1H), 3.02∼3.04 (m, 1H), 2.86∼2.95 (m, 9H), 2.32∼2.35 (m, 1H), 2.15∼2.17 (m, 1H), 1.69∼1.82 (m, 1H), 1.67∼1.68 (m, 1H), 1.36 (s, 9H). | **676** |
| **25** | 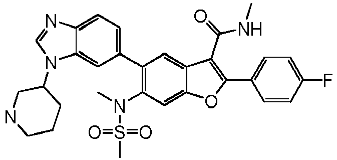 | ¹H-NMR (CDCl₃, 400 MHz) δ 8.00 (s, 1H), 7.86∼7.89 (m, 2H), 7.70∼7.75 (m, 3H), 7.44 (s, 1H), 7.23∼7.25 (m, 1H),7.11 (t, *J* = 8.4 Hz, 2H), 6.40 (s, 1H), 4.50∼4.53 (m, 1H), 3.49∼3.51 (m, 1H), 3.12∼3.15 (m, 2H), 2.96∼3.01 (m, 1H), 2.92 (s, 3H), 2.87 (d, *J* = 4.8 Hz, 3H), 2.70∼2.75 (m, 1H), 2.59 (s, 3H), 2.20∼2.23 (m, 1H), 2.00∼2.10 (m, 1H), 1.77∼1.89 (m, 2H). | **576** |
| **26** | 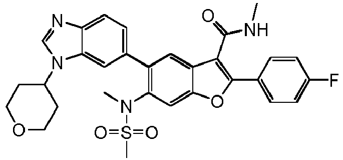 | ¹H-NMR (CDCl₃, 400 MHz) δ 8.95 (s, 1H), 8.05 (s, 1H), 7.88∼7.96 (m, 4H), 7.54∼7.55 (m, 2H), 7.18∼7.24 (m, 2H), 5.97 (br s, 1H), 4.68∼4.69 (m, 1H), 4.16∼4.19 (m, 2H), 3.59∼3.64 (m, 2H), 2.93∼2.96 (m, 9H), 2.15∼2.27 (m, 4H). | **577** |
| **27** | 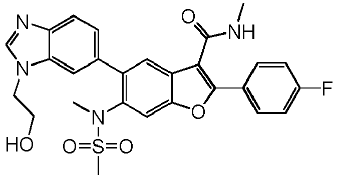 | ¹H-NMR (CDCl₃, 400 MHz) δ 9.14 (s, 1H), 7.94 (s, 1H), 7.85∼7.90 (m, 4H), 7.53 (d, *J* = 8.0 Hz, 1H), 7.14∼7.16 (m, 3H), 5.92 (s, 1H), 4.46 (s, 2H), 4.02 (s, 2H), 2.90∼2.91 (m, 9H). | 537 |
| **28** | 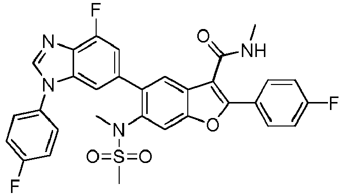 | ¹H-NMR (CDCl₃, 400 MHz) δ 8.07 (s, 1H), 7.90∼7.94 (m, 2H), 7.83 (s, 1H), 7.53∼7.56 (m, 3H), 7.46 (s, 1H), 7.23∼7.27 (m, 2H), 7.19 (t, *J* = 8.4 Hz, 2H), 7.09 (d, *J* = 10.6 Hz, 1H), 5.91 (d, *J* = 4.8 Hz, 1H), 3.03 (s, 3H), 2.96 (d, *J* = 4.8 Hz, 3H), 2.84 (s, 3H). | **605** |
| **29** | 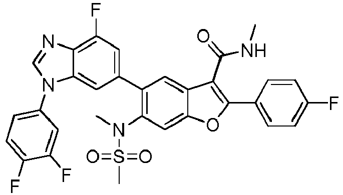 | ¹H-NMR (CDCl₃, 400 MHz) δ 7.88∼7.93 (m, 3H), 7.78 (s, 1H), 7.48 (s, 1H), 7.42 (s, 1H), 7.36∼7.40 (m, 1H), 7.27∼7.31 (m, 2H), 7.14 (t, *J* = 8.4 Hz, 2H), 7.02 (d, *J* = 10.8 Hz, 1H), 6.21 (s, 1H), 2.93 (s, 3H), 2.92 (s, 3H), 2.89 (s, 3H). | **623** |
| **30** | 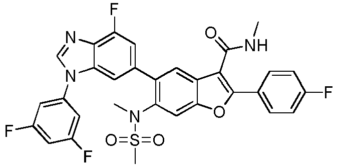 | ¹H-NMR (CDCl₃, 400 MHz) δ 8.10 (s, 1H), 7.90∼7.93 (m, 2H), 7.84 (s, 1H), 7.55 (d, *J* = 10.0 Hz, 2H), 7.17∼7.21 (m, 4H), 7.13 (d, *J* = 10.8 Hz, 1H), 6.89∼6.93 (m, 1H), 5.90 (d, *J* = 3.6 Hz, 1H), 3.05 (s, 3H), 2.96 (d, *J* = 4.8 Hz, 3H), 2.88 (s, 3H). | **623** |
| **31** | 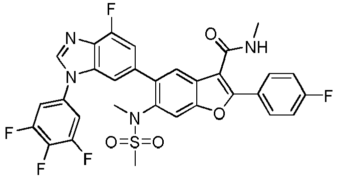 | ¹H-NMR (CDCl₃, 400 MHz) δ 8.11 (s, 1H), 7.84∼7.87 (m, 2H), 7.81 (s, 1H), 7.55 (s, 1H), 7.48 (s, 1H), 7.29 (t, *J* = 6.8 Hz, 2H), 7.10∼7.15 (m, 3H), 5.76 (d, *J* = 4.8 Hz, 1H), 2.97 (s, 3H), 2.91 (d, *J* = 3.6 Hz, 3H), 2.91 (s, 3H). | **641** |
| **32** | 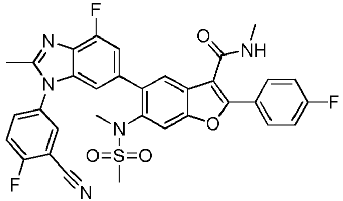 | 1H-NMR (CDCl₃, 400 MHz) δ 8.05∼8.08 (m, 3H), 7.69 (s, 2H), 7.53 (s, 1H), 7.40 (s, 1H), 7.27 (t, J = 8.0 Hz, 1H), 7.12 (t, *J* = 8.4 Hz, 2H), 6.95 (s, 1H), 6.87 (d, *J* = 10.8 Hz, 1H), 3.11 (s, 3H), 3.07 (d, *J* = 4.4 Hz, 3H), 2.75 (s, 3H), 1.92 (s, 3H). | **644** |
| **33** | 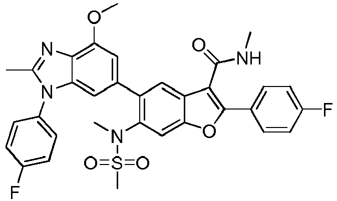 | ¹H-NMR (CDCl₃, 400 MHz) δ 7.80∼7.83 (m, 2H), 7.69 (s, 1H), 7.44 (s, 3H), 7.30 (t, *J* = 8.0 Hz, 2H), 7.15 (t, *J* = 8.4 Hz, 2H), 7.05 (s, 1H), 6.96 (s, 1H), 5.84 (s, 1H), 3.99 (s, 3H), 2.94 (s, 3H), 2.90 (d, *J* = 4.4 Hz, 3H), 2.85 (s, 3H), 2.72 (s, 3H). | **631** |
| **34** | 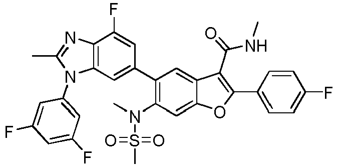 | ¹H-NMR (CDCl₃, 400 MHz) δ 7.91∼7.94 (m, 2H), 7.75 (s, 1H), 7.45 (s, 1H), 7.12 (t, *J* = 8.4 Hz, 2H), 7.07 (s, 1H), 6.86∼6.97 (m, 4H), 6.96 (s, 1H), 2.96 (d, *J* = 4.8 Hz, 3H), 2.92 (s, 3H), 2.84 (s, 3H), 2.30 (s, 3H). | **637** |
| **35** | 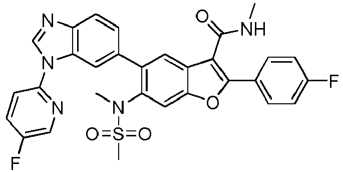 | ¹H-NMR (CDCl₃, 400 MHz) δ 9.10 (s, 1H), 8.86 (d, *J* = 4.0 Hz, 1H), 8.45 (d, *J* = 2.4 Hz, 1H), 8.28 (s, 1H), 8.03 (d, *J* = 8.4 Hz, 1H), 7.88∼7.93 (m, 2H), 7.82 (t, *J* = 7.6 Hz, 1H), 7.72 (s, 1H), 7.57 (s, 1H), 7.53 (d, *J* = 7.6 Hz, 1H), 7.20 (t, *J* = 8.4 Hz, 2H), 5.81 (s, 1H), 3.02 (s, 3H), 2.96 (d, *J* = 4.8 Hz, 3H), 2.90 (s, 3H). | **588** |
| **36** | 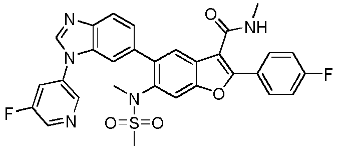 | ¹H-NMR (CDCl₃, 400 MHz) δ 8.77 (s, 1H), 8.64 (d, *J* = 1.6 Hz, 1H), 8.51 (s, 1H), 8.00 (d, *J* = 8.0 Hz, 1H), 7.89∼7.92 (m, 3H), 7.86 (s, 2H), 7.52 (s, 1H), 7.50 (d, *J* = 8.4 Hz, 1H), 7.20 (t, *J* = 8.8 Hz, 2H), 5.80 (d, *J* = 4.0 Hz, 1H), 2.99 (s, 3H), 2.95 (d, *J* = 4.8 Hz, 3H), 2.93 (s, 3H). | **588** |
| **37** | 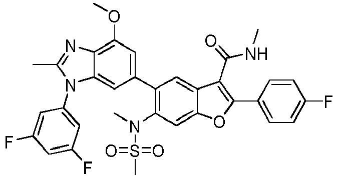 | ¹H-NMR (CDCl₃, 400 MHz) δ 7.86∼7.89 (m, 3H), 7.16 (s, 1H), 7.47 (s, 1H), 7.13 (t*, J* = 8.4 Hz, 2H), 6.88∼6.95 (m, 2H), 6.83 (d, *J* = 7.2 Hz, 2H), 5.83 (s, 1H), 4.00 (s, 3H), 2.93 (s, 3H), 2.91 (d*, J* = 4.8 Hz, 3H), 2.76 (s, 3H), 2.49 (s, 3H). | **649** |
| **38** | 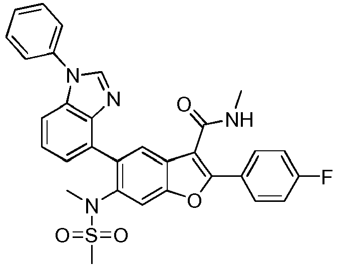 | ¹H NMR (CDCl₃, 400 MHz) δ 8.07 (s, 1H), 8.01∼8.05 (m, 2H), 7.90 (s, 1H), 7.69 (s, 1H), 7.54∼7.62 (m, 5H), 7.49 (t*, J* = 7.2 Hz, 1H), 7.40∼7.42 (m, 2H), 7.14∼7.19 (m, 2H), 6.58 (br s, 1H), 3.19 (s, 3H), 2.93 (d, *J* = 4.8 Hz, 3H), 2.51 (s, 3H). | **569** |

### Example 2

### Preparation of Compound 39

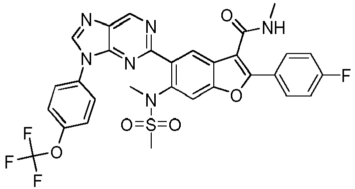

### Step 1 - Synthesis of 2-chloro-5-nitro-N-(4-(trifluoromethoxy)phenyl)pyrimidin-4-amine

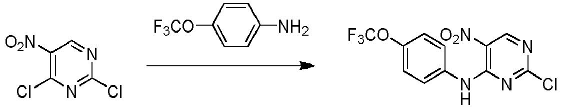

A mixture of 2, 4-dichloro-5-nitropyrimidine (3.0 g, 15.5 mmol) in anhydrous THF (5 mL) was added dropwise 4-fluoroaniline (2.7 g, 15.5 mmol) in THF at ice-bath, then the mixture was allowed to stir at room temperature for overnight. After the reaction mixture was concentrated, the resulting residue was purified using column chromatography eluted with PE : EtOAc = 40 : 1 to provide 2-chloro-5-nitro-N-(4- (trifluoromethoxy)phenyl)pyrimidin-4-amine (3.6 g, yield: 69.0%). ¹H-NMR (CDCl₃, 400 MHz) δ 9.58 (s, 1H), 9.09 (s, 1H), 7.50 (s, 2H), 7.05∼7.09 (m, 2H). MS (M+H)⁺: 345.

### Step 2 - Synthesis of 2-chloro-N-(4-(trifluoromethoxy)phenyl)pyrimidine-4,5-diamine

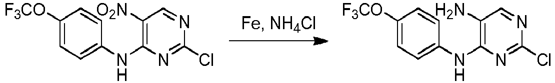

A mixture of 2-chloro-5-nitro-N-(4-(trifluoromethoxy)phenyl)pyrimidin-4-amine (5.0 g, 14.9 mmol), Fe (2.5 g, 44.8 mmol) and NH₄Cl (4.8 g, 89.6 mmol) in THF : MeOH : H₂O (20 mL : 20 mL : 20 mL) was refluxed for 3 hours. After the reaction mixture was filtrated, the filtrate was concentrated, the resulting residue was purified using column chromatography eluted with PE : EtOAc = 4 : 1 to provide 2-chloro-N⁴-(4-(trifluoromethoxy)phenyl)pyrimidine-4,5-diamine (3.8 g, yield: 70.2%). ¹H-NMR (CDCl₃, 400 MHz) δ 7.70 (s, 1H), 7.55∼7.57 (m, 2H), 7.19∼7.21 (m, 1H), 7.12∼7.14 (m, 2H). MS (M+H)⁺: 305.

### Step 3 - Synthesis of 2-chloro-9-(4-(trifluoromethoxy)phenyl)-9H-purine

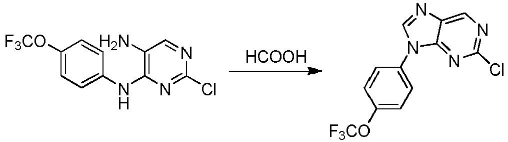

A mixture of 2-chloro-N⁴-(4-(trifluoromethoxy)phenyl)pyrimidine-4,5-diamine (1.0 g, 3.3 mmol) in HCOOH (10 mL) was refluxed for 3 hours. After the reaction mixture was diluted with EtOAc, the mixture was washed with saturated NaHCO₃, dried (Na₂SO₄), concentrated, the resulting residue was purified using column chromatography eluted with PE : EtOAc = 4 : 1 to provide 2-chloro-9-(4-(trifluoromethoxy)phenyl)-9H-purine (460 mg, yield: 44.5%). ¹H-NMR (CDCl₃, 400 MHz) δ 9.03 (s, 1H), 8.29 (s, 1H), 7.70∼7.72 (m, 2H), 7.39∼7.42 (m, 2H). MS (M+H)⁺: 315.

### Step 4 - Synthesis of 2-(4-fluorophenyl)-N-methyl-6-(N-methylmethylsulfonamido)-5-(9-(4-(trifluoromethoxy)phenyl)-9H-purin-2-yl)benzofuran-3-carboxamide

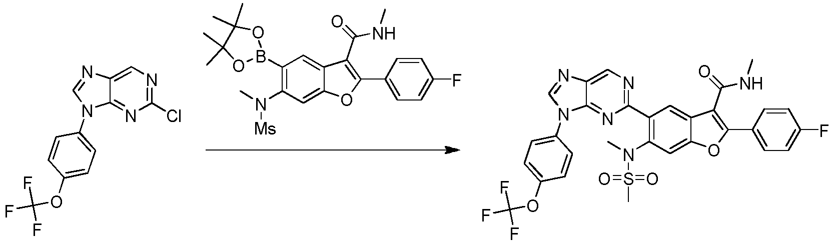

2-(4-fluorophenyl)-N-methyl-6-(N-methylmethylsulfonamido)-5-(9-(4-(trifluoromethoxy)phenyl)-9H-purin-2-yl)benzofuran-3-carboxamide was prepared using the method described in Example **1**. ¹H-NMR (CDCl₃, 400 MHz) δ 9.46 (s, 1H), 8.45 (s, 1H), 8.34 (s, 1H), 8.00∼8.04 (m, 2H), 7.85∼7.87 (m, 2H), 7.65 (s, 1H), 7.45∼7.47 (m, 2H), 7.17∼7.22 (m, 2H), 6.27 (s, 1H), 3.34 (s, 3H), 3.02 (d, *J* = 4.4 Hz, 3H), 2.82 (s, 3H). MS (M+H)⁺: 655.

Compounds **40-46**, depicted in the table below, prepared using the method described above and substituting the appropriate reactants and reagents.

| **Compound** | **Structure** | **NMR** | **MS (M+H)⁺** |
|---|---|---|---|
| **40** | 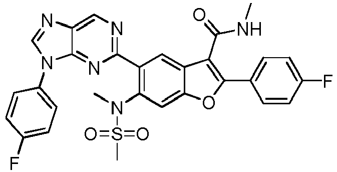 | ¹H-NMR (CDCl₃, 400 MHz) δ 9.37 (s, 1H), 8.99 (s, 1H), 8.58 (d*, J* = 4.4 Hz, 1H), 8.10 (s, 1H), 7.94∼8.02 (m, 5H), 7.98∼8.02 (m, 2H), 7.94∼7.96 (m, 2H), 3.30 (s, 3H), 2.86 (d*, J* = 4.4 Hz, 3H), 2.80 (s, 3H). | **589** |
| **41** | 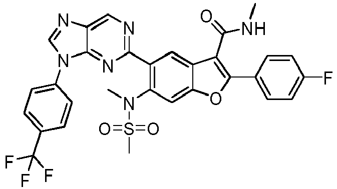 | ¹H-NMR (CDCl₃, 400 MHz) δ 9.46 (s, 1H), 8.45 (s, 1H), 8.34 (s, 1H), 8.00∼8.04 (m, 2H), 7.85∼7.87 (m, 2H), 7.65 (s, 1H), 7.45∼7.47 (m, 2H), 7.17∼7.22 (m, 2H), 6.27 (s, 1H), 3.34 (s, 3H), 3.02 (d, *J* = 4.4 Hz, 3H), 2.82 (s, 3H). | **639** |
| **42** | 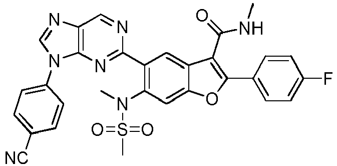 | ¹H-NMR (CDCl₃, 400 MHz) δ 9.30 (s, 1H), 8.38 (s, 1H), 8.35 (s, 1H), 8.01 (d, *J* = 8.4 Hz, 2H), 7.91∼7.94 (m, 2H), 7.83 (d*, J* = 8.4 Hz, 2H), 7.59 (s, 1H), 7.14 (t, *J* = 8.4 Hz, 2H), 5.93 (brs, 1H), 3.30 (s, 3H), 2.94 (d, *J* = 4.8 Hz, 3H), 2.79 (s, 3H). | **596** |
| **43** | 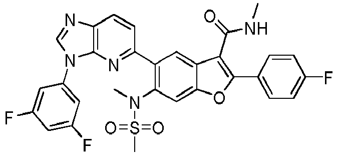 | ¹H-NMR (CDCl₃, 400 MHz) δ 8.35 (s, 1H), 8.19 (d, *J* = 8.0 Hz, 1H), 8.00 (s, 1H), 7.89∼7.92 (m, 2H), 7.61 (s, 1H), 7.59 (s, 1H), 7.47 (d*, J* = 5.2 Hz, 2H), 7.15 (t*, J* = 8.8 Hz, 2H), 6.81 (t*, J* = 8.4 Hz, 1H), 5.80 (s, 1H), 3.22 (s, 3H), 2.94 (d*, J* = 4.8 Hz, 3H), 2.63 (s, 3H). | **606** |
| **44** | 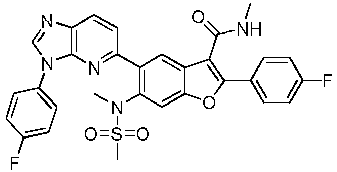 | ¹H-NMR (CDCl₃, 400 MHz) 8.30 (s, 1H), 8.22 (d*, J* = 8.0 Hz, 1H), 8.01 (s, 1H), 7.91∼7.94 (dd, *J* = 8.8 Hz, 2H), 7.71∼7.74 (m, 2H), 7.65 (s, 1H), 7.59 (d, *J* = 8.4 Hz, 1H), 7.17∼7.25 (m, 4H), 5.97 (brs, 1H), 3.12 (s, 3H), 2.96 (d*, J* = 4.8 Hz, 3H), 2.60 (s, 3H). | **588** |
| **45** | 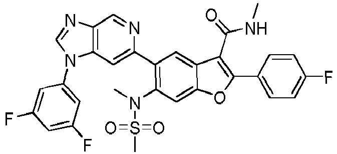 | ¹H-NMR (DMSO-*d*6, 400 MHz) δ 9.18 (s, 1H), 8.16 (s, 1H), 7.99 (s, 1H), 7.91∼7.94 (m, 3H), 7.53 (s, 1H), 7.16 (d*, J* = 4.4 Hz, 2H), 7.11 (d, *J* = 8.8 Hz, 2H), 6.88∼6.92 (t*, J* = 8.8 Hz, 1H), 5.94 (d, *J* = 2.8 Hz, 1H), 3.06 (s, 3H), 2.92 (d, *J* = 5.2 Hz, 3H), 2.90 (s, 3H). | **606** |
| **46** | 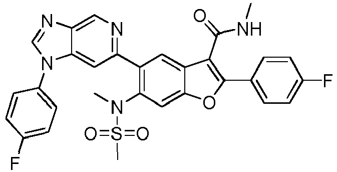 | ¹H-NMR (CDCl₃, 400 MHz) 9.21 (s, 1H), 8.17 (s, 1H), 8.01 (s, 1H), 7.94∼7.97 (m, 2H), 7.86 (s, 1H), 7.55∼7.59 (m, 3H), 7.26∼7.30 (t, *J* = 8.8 Hz, 2H), 7.15∼7.19 (t*, J* = 8.4 Hz, 2H), 5.94 (brs, 1H), 3.07 (s, 3H), 2.95∼2.96 (t, *J* = 4.8 Hz, 3H), 2.91 (s, 3H). | **588** |

### Example 3 (illustrative)

### Preparation of Compound 47

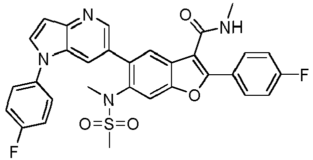

### Step 1 - Synthesis of diethyl 2-(5-bromo-3-nitropyridin-2-yl)malonate

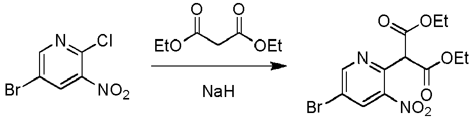

Sodium hydride (2.4 g, 95 mmol) was dissolved in DMF (6 mL) to this slowly added diethyl malonate (1.3 g, 79 mmol) under an inert atmosphere, once the addition was complete the reaction was allowed to stir for 10 minutes, 5-bromo-2-chloro-3-nitropyridine (1.0 g, 42 mmol) in DMF (2 mL) was slowly added to the anion, the reaction was maintained at approximately 40 °C for 3 hours before being quenched with waster (10 mL), extracted with EtOAc, dried (Na₂SO₄) and concentrated, the resulting residue was purified using column chromatography eluted with PE : EtOAc = 40 : 1 to provide diethyl 2-(5-bromo-3-nitropyridin-2-yl)malonate (1.0 g, yield: 67.8%). ¹H-NMR (CDCl₃, 400 MHz) δ 8.86 (s, 1H), 8.60 (s, 1H), 5.44 (s, 1H), 4.25∼4.30 (m, 4H), 1.25∼1.29 (m, 6H). (M+H)⁺: 361 / 363.

### Step 2 - Synthesis of 5-bromo-2-methyl-3-nitropyridine

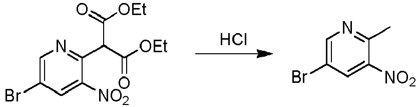

A mixture of diethyl 2-(5-bromo-3-nitropyridin-2-yl)malonate (1.2 g, 3.3 mmol) was added to 7.0 N HCl (10 mL) and heated to refluxed for 5 hours, the reaction was cooled to room temperature and extracted with DCM : MeOH = 10 : 1, the organic phase was dried (Na₂SO₄) and concentrated, the resulting residue was purified using column chromatography eluted with PE : EtOAc = 4 : 1 to provide 5-bromo-2-methyl-3-nitropyridine (0.7 g, yield: 89.7%). ¹H-NMR (CDCl₃, 400 MHz) δ 8.76 (s, 1H), 8.40 (s, 1H), 2.80 (s, 3H). (M+H)⁺: 217 / 219.

### Step 3 - Synthesis of 2-(5-bromo-3-nitropyridin-2-yl)-N,N-dimethylethenamine

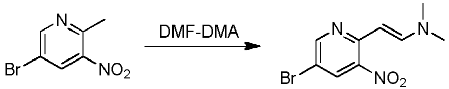

5-bromo-2-methyl-3-nitropyridine (1.0, 4.6 mmol) was dissolved in 10 mL of dry DMF and stirred under N₂, DMF-DMA (6.3 mL) was added dropwise. The reaction was heated to 90 °C, after 15 min of heating a deep reddish color began to appear, and it was allowed to react for 4 hours, the solvent was removed by evaporation, the resulting residue was purified using column chromatography eluted with PE : EtOAc = 30 : 1 to provide 2-(5-bromo-3-nitropyridin-2-yl)-N,N-dimethylethenamine (1.2 g, yield: 95.2%). ¹H-NMR (CDCl₃, 400 MHz) δ 8.35 (s, 1H), 8.27 (s, 1H), 7.99∼8.03 (m, 1H), 6.08∼6.11 (m, 1H), 2.98 (s, 6H). (M+H)⁺: 272 / 274.

### Step 4 - Synthesis of 6-bromo-1H-pyrrolo[3,2-b]pyridine

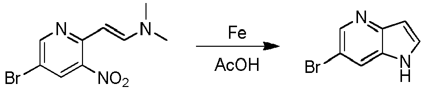

A mixture of 2-(5-bromo-3-nitropyridin-2-yl)-N,N-dimethylethenamine (1.0 g, 3.7 mmol) and Fe (1.8 g, 32.2 mmol) in AcOH (10 mL) was heated at 100 °C overnight. After the reaction mixture was filtrated, the filtrate was concentrated, the resulting residue was purified using column chromatography eluted with PE : EtOAc = 5 : 1 to provide 6-bromo-1H-pyrrolo[3, 2-b]pyridine (24 mg, yield: 33.3%). (M+H)⁺: 197 / 199.

### Step 5 - Synthesis of 6-bromo-1-(4-fluorophenyl)-1H-pyrrolo[3,2-b]pyridine

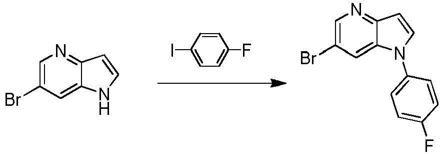

To a solution of 6-bromo-1H-pyrrolo[3, 2-b]pyridine (100 mg, 0.51 mmol), 1-fluoro-4-iodobenzene (225 mg, 1.02 mmol) and Cs₂CO₃ (496 mg, 1.52 mmol) in dioxane (2 mL) was allowed to stir at room temperature, the CuI (cat) and (IS, 2S)-N,N'-dimethylcyclohexane-1, 2-diamine (cat) were added under N₂, and then the mixture was allowed to stir at reflux for overnight. After the reaction mixture was cooled to room temperature and filtrated, the filtrate was concentrated, the resulting residue was purified using prep-TLC (PE : EtOAc = 1 : 1) to provide 6-bromo-1-(4-fluorophenyl)-1H-pyrrolo[3, 2-b]pyridine (80 mg, yield: 50.2%). ¹H-NMR (CDCl₃, 400 MHz) δ 8.54 (s, 1H), 7.65 (s, 1H), 7.90∼7.94 (m, 3H), 7.78∼7.80 (m, 1H), 7.65∼7.67 (m, 1H), 7.54∼7.57 (m, 1H). (M+H)⁺: 291 / 293.

### Step 5 - Synthesis of 2-(4-fluorophenyl)-5-(1-(4-fluorophenyl)-1H-pyrrolo[3,2-b]pyridin-6-yl)-N-methyl-6-(N-methylmethylsulfonamido)benzofuran-3-carboxamide

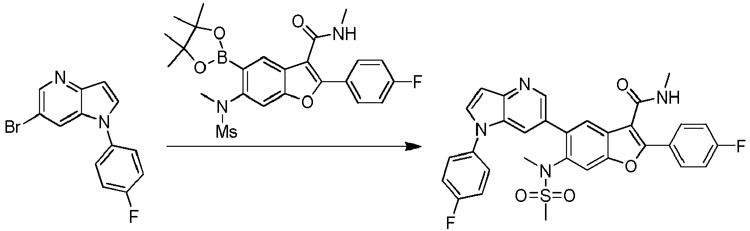

The procedure of 2-(4-fluorophenyl)-5-(1-(4-fluorophenyl)-1H-pyrrolo[3,2-b]pyridin-6-yl)-N-methyl-6-(N-methylmethylsulfonamido)benzofuran-3-carboxamide was similar to that of Example **1**. ¹H-NMR (CDCl₃, 400 MHz) δ 8.84 (s, 1H), 8.55 (s, 1H), 7.90∼7.94 (m, 3H), 7.85∼7.86 (m, 1H), 7.54∼7.57 (m, 3H), 7.26∼7.31 (m, 3H), 7.16∼7.21 (m, 2H), 5.99∼6.00 (br, 1H), 3.13 (s, 3H), 2.98 (d, *J* = 4.2 Hz, 3H), 2.96 (s, 3H). (M+H)⁺: 587.

### Example 4 (illustrative)

### Preparation of Compound 67

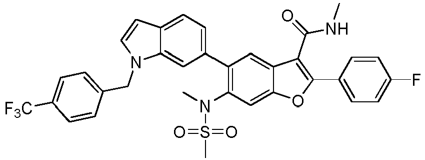

### Step 1 - Synthesis of 6-chloro-1-(4-(trifluoromethyl)benzyl)-1H-indole

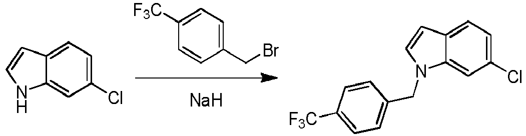

NaH (65 mg, 1.63 mmol) was added to a solution of 6-chloro-1H-indole (200 mg, 1.32 mmol) in dry DMF (5 mL) under N₂. The mixture was allowed to stir at room temperature for 1 hour. Then 1-(bromomethyl)-4-(trifluoromethyl)benzene (500 mg, 2.09 mmol) was added to the reaction mixture, and the mixture was allowed to stir at room temperature for 30mins. Ice cold NH₄Cl (sat. aq.) was added and the mixture was extracted with EtOAc. The organic layer was washed with brine, dried over Na₂SO₄, filtered and the solvent was evaporated under reduced pressure. The crude product was purified using column chromatography to provide product 6-chloro-1-(4-(trifluoromethyl)benzyl)-1H-indole (400 mg, yield: 97%). ¹H-NMR (CDCl₃, 400 MHz) δ 7.55∼7.58 (m, 3H), 7.22 (s, 1H), 7.09∼7.17 (m, 4H), 6.57 (br s, 1H), 5.33 (s, 2H). (M+H)⁺: 310.

### Step 2 - Synthesis of 6-(4,4,5,5-tetramethyl-1,3,2-dioxaborolan-2-yl)-1-(4-(trifluoromethyl)benzyl)-1H-indole

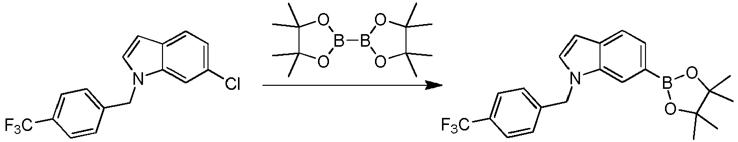

To a solution of 6-chloro-1-(4-(trifluoromethyl)benzyl)-1H-indole (0.4 g, 1.29 mmol) in dioxane (5 mL), KOAc (0.4 g, 4.08 mmol), 4,4,4',4',5,5,5',5'-octamethyl-2,2'-bi(1,3,2-dioxaborolane) (0.5 g, 1.97 mmol), Pd₂(dba)₃ (20 mg), X-Phos (20 mg) were added under N₂ protection. The mixture was heated at 100 °C for overnight. The mixture was concentrated in vacuo. The resulting residue was purified using column chromatography (PE : EtOAc = 10 : 1) to provide 6-(4,4,5,5-tetramethyl-1,3,2-dioxaborolan-2-yl)-1-(4-(trifluoromethyl)benzyl)-1H-indole (400 mg, yield: 77%). ¹H-NMR (CDCl₃, 400 MHz) δ 7.79 (s, 1H), 7.68 (d, *J =* 8.0 Hz, 1H), 6.59 (d, *J =* 8.0 Hz, 1H), 7.53∼7.56 (m, 2H), 7.14∼7.17 (m, 3H), 6.59 (d, *J=* 3.2 Hz, 1H), 5.44 (s, 2H), 1.35 (s, 12H). (M+H)⁺: 402.

### Step 3 - Synthesis of 2-(4-fluorophenyl)-N-methyl-6-(N-methylmethylsulfonamido)-5-(1-(4-(trifluoromethyl)benzyl)-1H-indol-6-yl)benzofuran-3-carboxamide

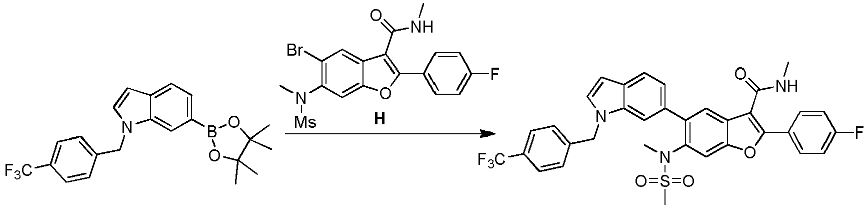

To a mixture of 5-bromo-2-(4-fluorophenyl)-N-methyl-6-(N-methylmethylsulfonamido)benzofuran-3-carboxamide (Compound **H**, 60 mg, 0.13 mmol), 6-(4,4,5,5-tetramethyl-1,3,2-dioxaborolan-2-yl)-1-(4-(trifluoromethyl)benzyl)-1H-indole (70 mg, 0.17 mmol) and K₃PO₄·3H₂O (70 mg, 0.26 mmol) in DMF (3 mL), Pd(dppf)Cl₂ (5 mg) was added under N₂ protection. The mixture was heated at 80 °C for 4 hours. The reaction mixture was concentrated in vacuo, suspended in water and extracted with EtOAc. The organic layer was washed with brine, dried over Na₂SO₄ and concentrated in vacuo. The resulting residue was purified using prep-TLC to provide 2-(4-fluorophenyl)-N-methyl-6-(N-methylmethylsulfonamido)-5-(1-(4-(trifluoromethyl)benzyl)-1H-indol-6-yl)benzofuran-3-carboxamide (50 mg, yield: 58%). ¹H-NMR (CDCl₃, 400 MHz) δ 7.92∼7.97 (m, 2H), 7.76 (s, 1H), 7.70 (d, *J* = 8.0 Hz, 1H), 7.52∼7.55 (m, 3H), 7.41 (s, 1H), 7.16∼7.25 (m, 6H), 6.62 (d, *J* = 2.4 Hz, 1H), 5.89 (br s, 1H), 5.42 (s, 2H), 2.95 (br s, 6H), 2.51 (s, 3H). (M+H)⁺: 650.

Compounds **68-69**, depicted in the table below, prepared using the method described above and substituting the appropriate reactants and reagents.

### Example 5 (illustrative)

Preparation of Compound **70**

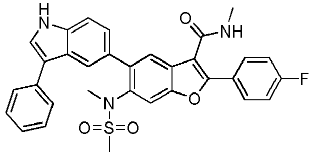

### Step 1 - Synthesis of 5-chloro-3-phenyl-1H-indole

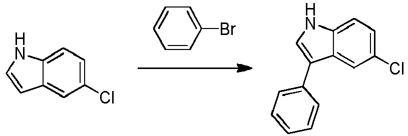

In a screw-cap vial under air, a solution of 5-chloro-1H-indole (200 mg, 1.32 mmol) and bromobenzene (249 mg, 1.58 mmol) in water (4 mL) was added Pd(OAc)₂ (15 mg), dppm (25 mg) and LiOH (166 mg, 2.96mmol) under N₂. The mixture was heated to 110 °C and then stirred 24 hours. The reaction mixture was cooled to room temperature and partitioned between IN HCl (5 mL) and EtOAc (10 mL). The layers were separated and the aqueous layer was further extracted with EtOAc (100 mL). The combined organic layer was washed with H₂O, brine, dried over Na₂SO₄. After being concentrated in vacuo, the resulting residue was purified using prep-TLC (PE : EtOAc = 5 : 1) to provide 5-chloro-3-phenyl-1H-indole (150 mg, yield: 50.0%). ¹H-NMR (CDCl₃, 400 MHz) δ 8.10 (s, 1H), 7.80 (d, *J=* 4.0 Hz, 1H), 7.51∼7.53 (m, 2H), 7.34∼7.38 (m, 2H), 7.20∼7.25 (m, 3H), 7.09∼7.11 (m, 1H). MS (M+H)⁺: 228 / 230.

### Step 2 - Synthesis of 2-(4-fluorophenyl)-N-methyl-6-(N-methylmethylsulfonamido)-5-(3-phenyl-1H-indol-5-yl)benzofuran-3-carboxamide

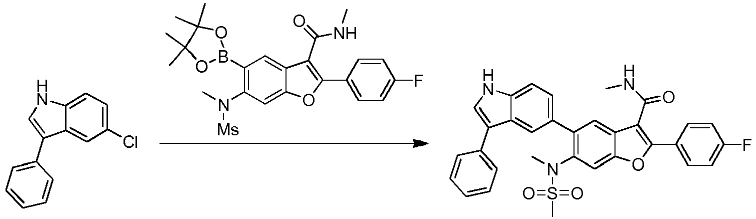

The procedure of 2-(4-fluorophenyl)-N-methyl-6-(N-methylmethylsulfonamido)-5-(3-phenyl-1H-indol-5-yl)benzofuran-3-carboxamide was similar to that of Example **1**. ¹H-NMR (CDCl₃, 400 MHz) δ 8.40 (s, 1H), 7.94∼7.98 (m, 3H), 7.79 (s, 1H), 7.66 (d, *J* = 8.0 Hz, 2H), 7.61 (s, 1H), 7.47 (d, *J* = 8.0 Hz, 1H), 7.40∼7.44 (m, 3H), 7.24∼7.33 (m, 2H), 7.15∼7.17 (m, 2H), 5.86 (d, *J* = 4.0 Hz, 1H), 3.11 (s, 3H), 2.96 (d*, J* = 4.0 Hz, 3H), 2.51 (s, 3H). MS (M+H)⁺: 568.

### Example 6

### Preparation of Compound 72 & 73

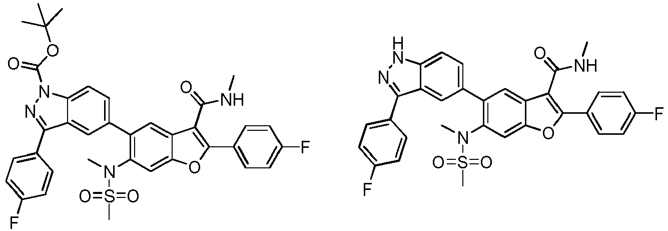

### Step 1 - Synthesis of 5-bromo-3-iodo-1H-indazole

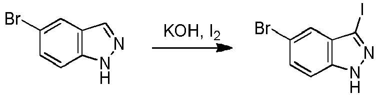

KOH (4.27 g, 76.13 mmol) was added into a solution of 5-bromo-1H-indazole (5.0 g, 25.38 mmol) and I₂ (12.9 g, 50.75 mmol) in DMF (10 mL) and stirred at room temperature for 12 hours. After being diluted with ice-water and extracted with EtOAc (50 mL x 3), and the combined organic phase was washed with brine, dried over Na₂SO₄ and concentrated to provide the desired product of 5-bromo-3-iodo-1H-indazole (8 g, yield: 90.0%). And it was used for the next step without further purification.

### Step 2 - Synthesis of tert-butyl 5-bromo-3-iodo-1H-indazole-1-carboxylate

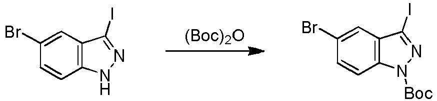

To a solution of 5-bromo-3-iodo-1H-indazole (8.0 g, 24.7 mmol), Et₃N (3.76 g, 37.2 mmol) and DMAP (151 mg, 1.24 mmol) in dry DCM (70 mL) was allowed to stir at 25 °C. Boc₂O (5.95 g, 27.3 mmol) was added. The mixture was allowed to stir to at 25 °C for overnight. The solvent was removed in vacuo and the resulting residue was purified using column chromatography (PE : EtOAc = 50 : 1) to provide tert-butyl 5-bromo-3-iodo-1H-indazole-1-carboxylate (7.0 g, yield: 77.8 %). ¹H-NMR (CDCl₃, 400 MHz) δ 7.94 (d, *J* = 8.0 Hz, 1H), 7.58∼7.61 (m, 2H), 1.64 (s, 9H). MS (M+H)⁺: 423 / 425.

### Step 3 - Synthesis of tert-butyl 5-bromo-3-(4-fluorophenyl)-1H-indazole-1-carboxylate

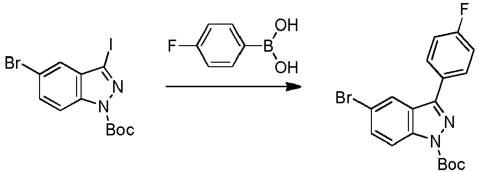

To a degassed solution of tert-butyl 5-bromo-3-iodo-1H-indazole-1-carboxylate (423 mg, 1.0 mmol) and 4-fluorophenylboronic acid (168 mg, 1.2 mmol) in dry ethanol: toluene (1 : 10 mL) was added Pd(dppf)Cl₂ (3 mg) and Na₂CO₃(4 mL, 2.0 mmol) under N₂. The mixture was heated to 100 °C and then stirred overnight. The reaction mixture was cooled to room temperature and filtered. The filtrate was washed with EtOAc, brine, dried over Na₂SO₄. After being concentrated in vacuo, the resulting residue was purified using prep-HPLC to provide tert-butyl 5-bromo-3-(4-fluorophenyl)-1H-indazole-1-carboxylate (20 mg, yield: 29%). ¹H-NMR (CDCl₃, 400 MHz) δ 8.03 (d, *J* = 8.0 Hz, 1H), 7.99 (d, *J* = 4.0 Hz, 1H), 7.84∼7.88 (m, 2H), 7.56∼7.59 (m, 1H), 7.13∼7.19 (m, 2H), 1.67 (s, 9H). MS (M+H)⁺: 391 / 393.

### Step 4 - Synthesis of tert-butyl 3-(4-fluorophenyl)-5-(2-(4-fluorophenyl)-3-(methylcarbamoyl)-6-(N-methylmethylsulfonamido)benzofuran-5-yl)-1H-indazole-1-carboxylate

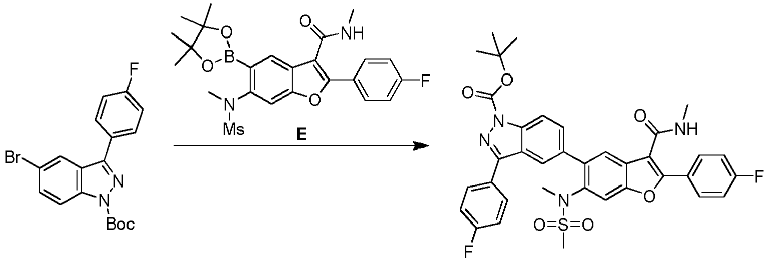

To a degassed solution of tert-butyl 5-bromo-3-(4-fluorophenyl)-1H-indazole-1-carboxylate (100 mg, 0.26 mmol) and 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (Compound E, 128 mg, 0.25 mmol) in dry dioxane (5 mL) was added Pd(dppf)Cl₂ (5 mg) and K₃PO₄ (204 mg, 0.77 mmol) under N₂. The mixture was heated to 100 °C and stirred overnight. The reaction mixture was cooled to room temperature and filtered. The filtrate was diluted with EtOAc, washed with brine, dried over Na₂SO₄. After being concentrated in vacuo, the resulting residue was purified using prep-HPLC to provide tert-butyl 3-(4-fluorophenyl)-5-(2-(4-fluorophenyl)-3-(methylcarbamoyl)-6-(N-methylmethylsulfonamido)benzofuran-5-yl)-1H-indazole-1-carboxylate (100 mg, yield: 56.8 %). ¹H-NMR (CDCl₃, 400 MHz) δ 8.23 (d, *J* = 8.0 Hz, 1H), 8.00∼8.05 (m, 3H), 7.90∼7.94 (m, 2H), 7.88 (s, 1H), 7.63∼7.65 (m, 1H), 7.59 (s, 1H), 7.17∼7.22 (m, 4H), 5.85 (d, *J =* 4.0 Hz, 1H), 3.07 (s, 3H), 2.96 (d, *J =* 4.8 Hz, 3H), 2.73 (s, 3H), 1.79 (s, 9H). MS (M+H)⁺: 687.

### Step 5 - Synthesis of 2-(4-fluorophenyl)-5-(3-(4-fluorophenyl)-1H-indazol-5-yl)-N-methyl-6-(N-methylmethylsulfonamido)benzofuran-3-carboxamide

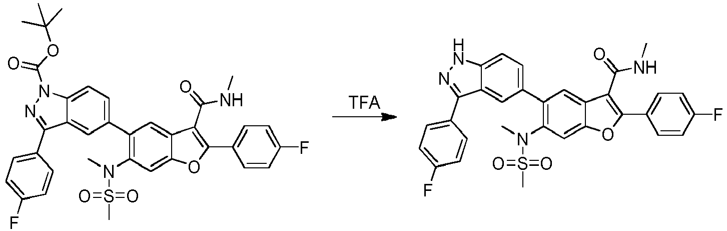

TFA (1 mL) was added into a solution of tert-butyl 3-(4-fluorophenyl)-5-(2-(4-fluorophenyl)-3-(methylcarbamoyl)-6-(N-methylmethylsulfonamido)benzofuran-5-yl)-1H-indazole-1-carboxylate (50 mg, 0.07 mmol) in DCM (10 mL) under N₂ dropwise at 0 °C and the mixture was allowed to stir at room temperature for 6 hours. After being diluted with NaHCO₃ (a.q) and extracted with EtOAc (50 mL x 3), the combined organic phase was washed with brine, dried over Na₂SO₄ and concentrated to provide the desired product of 2-(4-fluorophenyl)-5-(3-(4-fluorophenyl)-1H-indazol-5-yl)-N-methyl-6-(N-methylmethylsulfonamido)benzofuran-3-carboxamide (30 mg, yield: 69.7%). ¹H-NMR (CDCl₃, 400 MHz) δ 7.99 (s, 1H), 7.84∼7.91 (m, 4H), 7.80 (s, 1H), 7.53 (s, 1H), 7.42∼7.48 (m, 2H), 7.03∼7.16 (m, 4H), 5.85 (d, *J* = 4.8 Hz, 1H), 3.01 (s, 3H), 2.90 (d, *J* = 4.8 Hz, 3H), 2.64 (s, 3H). MS (M+H)⁺: 587.

Compounds **74-76**, depicted in the table below, prepared using the method described above and substituting the appropriate reactants and reagents.

| **Compound** | **Structure** | **NMR** | **MS (M+H)⁺** |
|---|---|---|---|
| **74** | 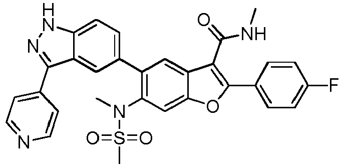 | ¹H-NMR (CDCl₃, 400 MHz) δ 8.48 (s, 2H), 8.19 (s, 2H), 8.09 (s, 1H), 7.82∼7.85 (m, 2H), 7.76 (s, 1H), 7.57 (d*, J* = 8.0 Hz, 1H), 7.49 (s, 1H), 7.44 (d*, J* = 8.0 Hz, 1H), 7.08∼7.13 (m, 2H), 2.97 (s, 3H), 2.92 (s, 3H), 2.90 (s, 3H). | **570** |
| **75** | 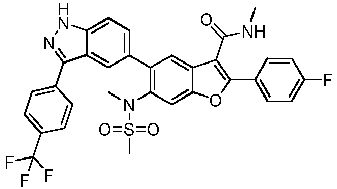 | ¹H-NMR (CDCl₃, 400 MHz) δ 8.03 (d*, J* = 8.0 Hz, 3H), 7.81∼7.86 (m, 3H), 7.65 (d, *J* = 8.0 Hz, 2H), 7.52 (s, 1H), 7.40∼7.48 (m, 2H), 7.11∼7.19 (m, 2H), 5.89 (d, *J* = 4.0 Hz, 1H), 3.00 (s, 3H), 2.90 (d, *J* = 4.0 Hz, 3H), 2.68 (s, 3H). | **637** |
| **76** | 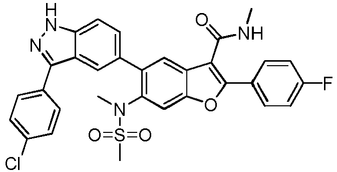 | ¹H-NMR (CDCl₃, 400 MHz) δ 7.97 (s, 1H), 7.81∼7.84 (m, 4H), 7.77 (s, 1H), 7.51 (s, 1H), 7.39∼7.41 (m, 1H), 7.34∼7.36 (m, 3H), 7.09∼7.18 (m, 2H), 5.98 (d, *J* = 4.8 Hz, 1H), 2.98 (s, 3H), 2.88 (d, *J* = 4.8 Hz, 3H), 2.63 (s, 3H). | **603** |

### Example 7 (illustrative)

### Preparation of Compound 77

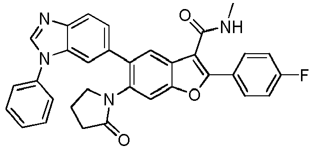

### Step 1 - Synthesis of ethyl 5-bromo-2-(4-fluorophenyl)-6-(2-oxopyrrolidin-1-yl)benzofuran-3-carboxylate

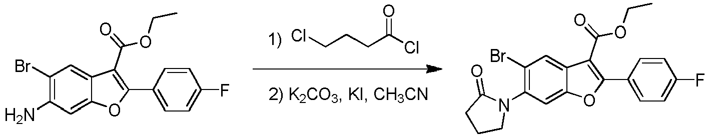

4-chlorobutanoyl chloride (670 mg, 4.76 mmol) was added dropwise at 0°C to a solution of ethyl 6-amino-5-bromo-2-(4-fluorophenyl)benzofuran-3-carboxylate and Et₃N (1.0 mL) in CH₂Cl₂ (10 mL) under N₂ protection and the mixture was allowed to stir at room temperature for 16 hours. The reaction mixture was concentrated to provide the resulting residue. A mixture of the resulting residue, K₂CO₃ (658 mg, 4.76 mmol) and KI (263 mg, 1.59 mmol) in CH₃CN (10 mL) was refluxed for 16 hours. After cooled, the mixture was diluted with water and extracted with EtOAc. The combined organic phases were washed with brine, dried over Na₂SO₄, filtered and evaporated. The crude product was purified using column chromatography (PE : EtOAc = 2 : 1) to provide pure ethyl 5-bromo-2-(4-fluorophenyl)-6-(2-oxopyrrolidin-1-yl)benzofuran-3-carboxylate (280 mg, yield: 40%). ¹H-NMR (CDCl₃, 400 MHz) δ 8.32 (s, 1H), 8.04∼8.07 (m, 2H), 7.48 (s, 1H), 7.17∼7.21 (m, 2H), 4.42∼4.43 (m, 2H), 3.82∼3.86 (m, 2H), 2.61∼2.65 (m, 2H), 2.27∼2.31 (m, 2H), 1.40∼1.44 (m, 3H). MS (M+H)⁺: 446 / 448.

### Step 2 - Synthesis of 5-bromo-2-(4-fluorophenyl)-6-(2-oxopyrrolidin-1-yl)benzofuran-3-carboxylic acid

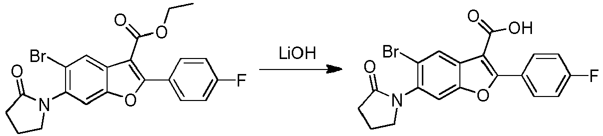

A solution of ethyl 5-bromo-2-(4-fluorophenyl)-6-(2-oxopyrrolidin-1-yl)benzofuran-3-carboxylate (2.5 g, 5.80 mmol) and LiOH (0.5 g, 21.0 mmol) in dioxane (30 mL) and water (10 mL) was allowed to stir at 90 °C for 1 hour. The mixture was quenched with ice and extracted with DCM, the organic layer was washed with brine, dried over Na₂SO₄ and concentrated to provide 5-bromo-2-(4-fluorophenyl)-6-(2-oxopyrrolidin-1-yl)benzofuran-3-carboxylic acid (2.2 g, yield: 91%). ¹H-NMR (CDCl₃, 400 MHz) δ 8.08 (s, 1H), 7.81∼7.84 (m, 2H), 7.34 (s, 1H), 6.89∼6.93 (m, 2H), 3.79∼3.82 (m, 2H), 2.66∼2.70 (m, 2H), 2.26∼2.31 (m, 2H). MS (M+H)⁺: 418 / 420.

### Step 3 - Synthesis of 5-bromo-2-(4-fluorophenyl)-N-methyl-6-(2-oxopyrrolidin-1-yl)benzofuran-3-carboxamide

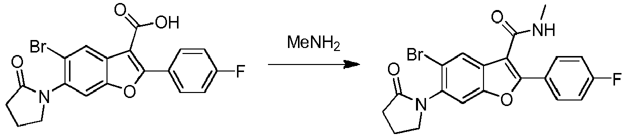

A solution of 5-bromo-2-(4-fluorophenyl)-6-(2-oxopyrrolidin-1-yl)benzofuran-3-carboxylic acid (280 mg, 0.67 mmol), HOBT (150 mg, 1.11 mmol) and EDCI (280 mg, 1.47 mmol) in dry DMF (2 mL) was allowed to stir at room temperature for 1 hour. Then Et₃N (0.2 mL) and CH₃NH₂ (HCl salt, 100 mg, 1.48 mmol) was added to the mixture, the resultant mixture was allowed to stir overnight. After the solvent was removed, water was added and the mixture was extracted with ethyl acetate. The combined organic layer was washed with brine, dried and concentrated in vacuo. The resulting residue was purified using column chromatography (PE : EtOAc = 1 : 1) to provide 5-bromo-2-(4-fluorophenyl)-N-methyl-6-(2-oxopyrrolidin-1-yl)benzofuran-3-carboxamide (220 mg, yield: 73%). ¹H-NMR (CDCl₃, 400 MHz) δ 7.94 (s, 1H), 7.82∼7.86 (m, 2H), 7.32 (s, 1H), 7.09∼7.14 (m, 2H), 6.29 (s, 1H), 3.75∼3.78 (m, 2H), 2.97 (d, *J* = 4.8 Hz, 3H), 2.56∼2.60 (m, 2H), 2.24∼2.26 (m, 2H). MS (M+H)⁺: 431 / 433.

### Step 4 - Synthesis of 2-(4-fluorophenyl)-N-methyl-6-(2-oxopyrrolidin-1-yl)-5-(1-phenyl-1H-benzo[d]imidazol-6-yl)benzofuran-3-carboxamide

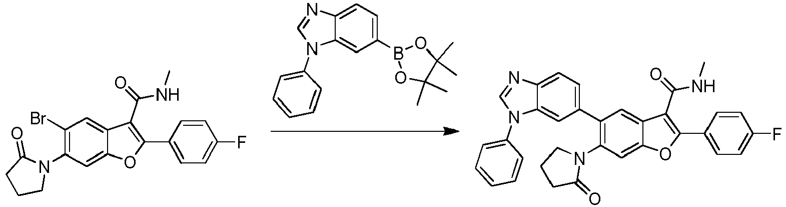

To a mixture of 5-bromo-2-(4-fluorophenyl)-N-methyl-6-(2-oxopyrrolidin-1-yl)benzofuran-3-carboxamide (70 mg, 0.16 mmol), K₃PO₄·3H₂O (108 mg, 0.41 mmol) and 1-phenyl-6-(4,4,5,5-tetramethyl-1,3,2-dioxaborolan-2-yl)-1H-benzo[d]imidazole (40 mg, 0.16 mmol, prepared from corresponding bromide intermediate of Compound **2**, using the method described in step 2 of Example **4**) in DMF (1 mL), Pd(dppf)Cl₂ (10 mg) was added under N₂ protection. The mixture was allowed to stir at 100 °C for 16 hours. After the mixture was filtered and concentrated, the resulting residue was purified using prep-HPLC to provide 2-(4-fluorophenyl)-N-methyl-6-(2-oxopyrrolidin-1-yl)-5-(1-phenyl-1H-benzo[d]imidazol-6-yl)benzofuran-3-carboxamide (10 mg, yield: 11%). ¹H-NMR (CDCl₃, 400 MHz) δ 8.11 (s, 1H), 7.81∼7.89 (m, 3H), 7.76 (s, 1H), 7.51∼7.54 (m, 3H), 7.40∼7.48 (m, 4H), 7.29∼7.31 (m, 1H), 7.10∼7.14 (m, 2H), 5.83∼5.85 (br s, 1H), 3.12∼3.15 (m, 2H), 2.89 (d, *J* = 4.8 Hz, 3H), 2.27∼2.31 (m, 2H), 1.73∼1.77 (m, 2H). MS (M+H)⁺: 545.

### Example 8

### Preparation of Compound 78

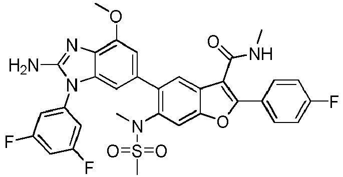

### Step 1 - Synthesis of 6-bromo-1-(3,5-difluorophenyl)-4-methoxy-1H-benzo[d]imidazol-2-amine

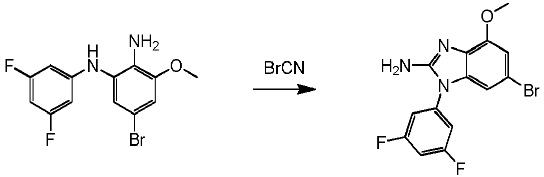

To a solution of 5-bromo-N¹-(3,5-difluorophenyl)-3-methoxybenzene-1,2-diamine (500 mg, 0.32 mmol, prepared using similar method described in the Example 1) in MeOH (15 mL) was added BrCN (750 mg, 0.63 mmol) and the mixture was heated at 60 °C for 10 hours. Then the mixture was concentrated, diluted with water, and extracted with DCM. The organics were dried over Na₂SO₄ and concentrated to give crude product of 6-bromo-1-(3,5-difluorophenyl)-4-methoxy-1H-benzo[d]imidazol-2-amine (500 mg, yield: 93%), which was used for next step without further purification. ¹H-NMR (CDCl₃, 400 MHz) δ 6.92∼7.00 (m, 3H), 6.78 (s, 2H), 4.98 (s, 2H), 3.91 (s, 3H). (M+H)⁺: 354 / 356.

### Step 2 - Synthesis of 5-(2-amino-1-(3,5-difluorophenyl)-4-methoxy-1H-benzo[d]imidazol-6-yl)-2-(4-fluorophenyl)-N-methyl-6-(N-methylmethylsulfonamido)benzofuran-3-carboxamide

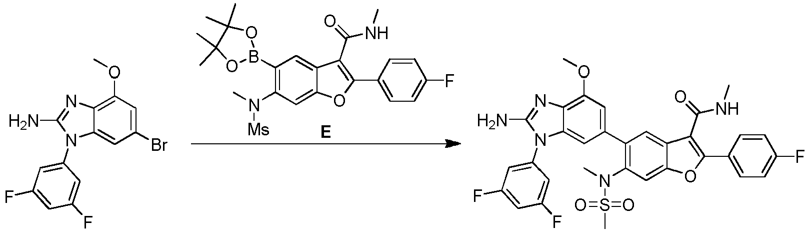

To a degassed solution of 6-bromo-1-(3,5-difluorophenyl)-4-methoxy-1H-benzo[d]imidazol-2-amine (77 mg, 0.22 mmol) and 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (Compound **E**, 100 mg, 0.2 mmol) in 1,4-dioxane (2 mL) were added Pd(dppf)Cl₂ (10 mg) and K₂CO₃ (41 mg, 0.3 mmol) under N₂. The mixture was heated at 100 °C overnight. The reaction mixture was cooled to room temperature, filtered and washed with EtOAc. The filtrate was washed with H₂O, brine, dried over Na₂SO₄ and concentrated, the residue was purified by prep-TLC (DCM : EtOAc = 1 : 1) to give 5-(2-amino-1-(3,5-difluorophenyl)-4-methoxy-1H-benzo[d]imidazol-6-yl)-2-(4-fluorophenyl)-N-methyl-6-(N-methylmethylsulfonamido)benzofuran-3-carboxamide (20 mg, yield: 18%). ¹H-NMR (CDCl₃, 400 MHz) δ 7.85∼7.89 (m, 2H), 7.73 (s, 1H), 7.45 (s, 1H), 7.12 (t, *J* = 8.4 Hz, 2H), 7.06∼7.08 (m, 2H), 6.85∼6.89 (m, 1H), 6.79 (d, *J* = 4.4 Hz, 2H), 5.90 (d, *J* = 4.8 Hz, 1H), 4.84 (s, 2H), 3.95 (s, 3H), 2.93 (s, 3H), 2.91 (d, *J* = 5.2 Hz, 3H), 2.75 (s, 3H). (M+H)⁺: 650.

Compound **79**, depicted in the table below, was prepared using the method described above and substituting the appropriate reactants and reagents.

| **Compound** | **Structure** | **NMR** | **MS (M+H)⁺** |
|---|---|---|---|
| 79 | 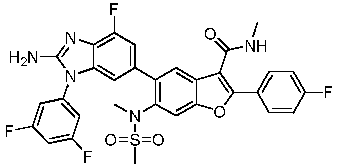 | ¹H-NMR (Methanol-*d*4, 400 MHz) δ 8.46∼8.47 (m, 1H), 7.91∼7.95 (m, 2H), 7.83 (s, 1H), 7.66 (d*, J* = 4.8 Hz, 1H), 7.42 (d*, J* = 6.0 Hz, 2H), 7.23∼7.34 (m, 3H), 7.13 (s, 1H), 3.07 (s, 3H), 3.03 (s, 3H), 2.91 (d, *J* = 4.0 Hz, 3H). | **638** |

### Example 9 (illustrative)

### Preparation of Compound 80

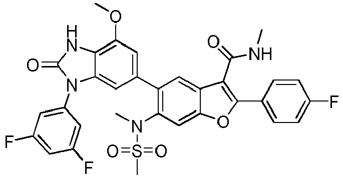

### Step 1 - Synthesis of 6-bromo-1-(3,5-difluorophenyl)-4-methoxy-1H-benzo[d]imidazol-2(3H)-one

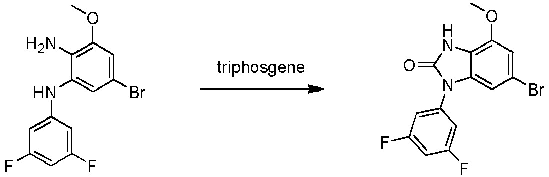

To a solution of 5-bromo-N¹-(3,5-difluorophenyl)-3-methoxybenzene-1,2-diamine (500 mg, 0.32 mmol) in DCM (13 mL) was added triphosgene (835 mg, 0.63 mmol) and the mixture was heated at 40 °C for 8 hours under N₂. Then the mixture was concentrated and diluted with water. After extracted with DCM, the organics were dried over Na₂SO₄ and concentrated to give crude product of 6-bromo-1-(3,5-difluorophenyl)-4-methoxy-1H-benzo[d]imidazol-2(3H)-one (500 mg, yield: 93%), which was used for next step without further purification. ¹H-NMR (CDCl₃, 400 MHz) δ 7.92 (s, 1H), 7.06 (d, *J* = 5.6 Hz, 2H), 6.86 (s, 1H), 6.79∼6.82 (m, 2H), 3.87 (s, 3H). (M+H)⁺: 355 / 357.

### Step 2 - Synthesis of 5-(3-(3,5-difluorophenyl)-7-methoxy-2-oxo-2,3-dihydro-1H-benzo[d]imidazol-5-yl)-2-(4-fluorophenyl)-N-methyl-6-(N-methylmethylsulfonamido)benzofuran-3-carboxamide

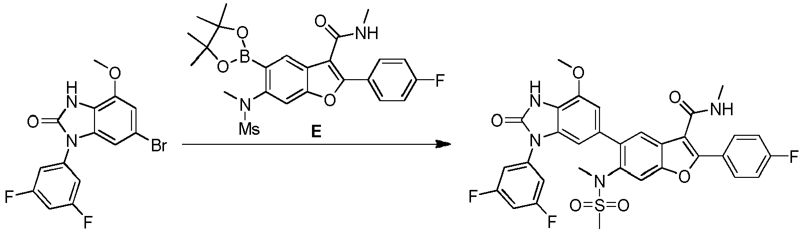

To a degassed solution of 6-bromo-1-(3,5-difluorophenyl)-4-methoxy-1H-benzo[d]imidazol-2(3H)-one (77 mg, 0.22 mmol) and 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (Compound **E**, 100 mg, 0.2 mmol) in 1,4-dioxane (2 mL) were added Pd(dppf)Cl₂ (10 mg) and K₂CO₃ (41 mg, 0.3 mmol) under N₂. The mixture was heated at 100 °C overnight. The reaction mixture was cooled to room temperature, filtered and washed with EtOAc. The filtrate was washed with H₂O, brine, dried over Na₂SO₄ and concentrated, the residue was purified by prep-TLC (DCM : EtOAc = 1 : 1) to give 5-(3-(3,5-difluorophenyl)-7-methoxy-2-oxo-2,3-dihydro-1H-benzo[d]imidazol-5-yl)-2-(4-fluorophenyl)-N-methyl-6-(N-methylmethylsulfonamido)benzofuran-3-carboxamide (30 mg, yield: 21%). ¹H-NMR (CDCl₃, 400 MHz) δ 7.84∼7.88 (m, 2H), 7.75 (d, *J=* 4.4 Hz, 1H), 7.45 (s, 1H), 7.12∼7.18 (m, 4H), 7.12 (s, 1H), 6.89 (s, 1H), 6.74∼6.80 (m, 1H), 5.74 (s, 1H), 3.91 (s, 3H), 2.97 (s, 3H), 2.91 (d, *J=* 5.6 Hz, 6H). (M+H)⁺: 651.

### Example 10 (illustrative)

### Preparation of Compound 83

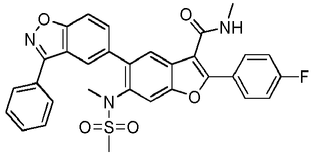

### Step 1 - Synthesis of (5-bromo-2-hydroxyphenyl)(phenyl)methanone

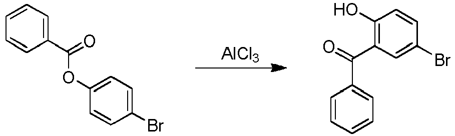

To a melt of 4-bromophenyl benzoate (4 g, 14.4 mmol) was added powdered aluminum trichloride (3.84 g, 28.8 mmol) and the mixture was heated at 150 °C for 3 h. To the residue was added 6 M of HCl and EtOAc. The organic phase was washed with brine, dried over sodium sulfate and concentrated in vacuo to give (5-bromo-2-hydroxyphenyl)(phenyl)methanone (3.2 g, yield: 80%) through column chromatography (PE : EtOAc = 10 : 1). ¹H-NMR (DMSO-*d*₆, 400 MHz) δ 10.38 (s, 1H), 7.70∼7.72 (m, 2H), 7.63∼7.67 (m, 1H), 7.44∼7.57 (m, 3H), 7.43 (s, 1H), 6.91 (d, *J* = 8.8 Hz, 1H). (M+H)⁺: 277 / 279.

### Step 2 - Synthesis of (E)-(5-bromo-2-hydroxyphenyl)(phenyl)methanone oxime

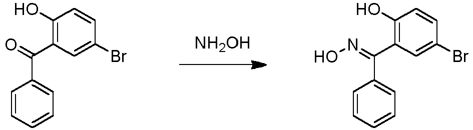

A solution of (5-bromo-2-hydroxyphenyl)(phenyl)methanone (2.0 g, 7.2 mmol) and hydroxylamine hydrochloride (3.52 g, 50.6 mmol) in 10 mL of pyridine and 60 mL of EtOH was heated to reflux for 16 h. The mixture was concentrated in vacuo and the residue was suspended in 1 M of HCl, then extracted with EtOAc. The organic phase was washed with brine, dried over sodium sulfate and concentrated in vacuo to give the pure (E)-(5-bromo-2-hydroxyphenyl)(phenyl)methanone oxime (670 mg, yield: 32%) through column chromatography (PE : EtOAc = 10 : 1). ¹H-NMR (DMSO-*d*₆, 400 MHz) δ 11.8 (s, 1H), 11.2 (s, 1H), 7.45∼7.51 (m, 3H), 7.34∼7.41 (m, 3H), 6.89∼6.91 (m, 2H). (M+H)⁺: 292 / 294.

### Step 3 - Synthesis of 5-bromo-3-phenylbenzo[d]isoxazole

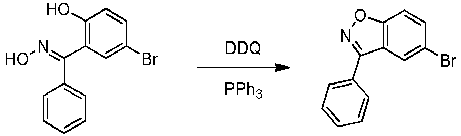

DDQ (116 mg, 0.51 mmol) and PPh₃ (135 mg, 0.51 mmol) were dissolved in DCM (5 mL) and the mixture was stirred for 10 minutes. Then (E)-(5-bromo-2-hydroxyphenyl)(phenyl)methanone oxime (100 mg, 0.34 mmol) was added and the mixture was stirred at room temperature for 2 h. Finally the mixture was concentrated in vacuo to give 5-bromo-3-phenylbenzo[d]isoxazole (90 mg, yield: 95%) through column chromatography (PE : EtOAc = 10 : 1). ¹H-NMR (DMSO-*d*6, 400 MHz) δ 8.19 (d, *J* = 6.8 Hz, 2H), 8.06 (s, 1H), 7.78 (d, *J =* 8.8 Hz, 1H), 7.58∼7.67 (m, 4H). (M+H)⁺: 274 / 276.

### Step 4 - Synthesis of 2-(4-fluorophenyl)-N-methyl-6-(N-methylmethylsulfonamido)-5-(3-phenylbenzo[d]isoxazol-5-yl)benzofuran-3-carboxamide

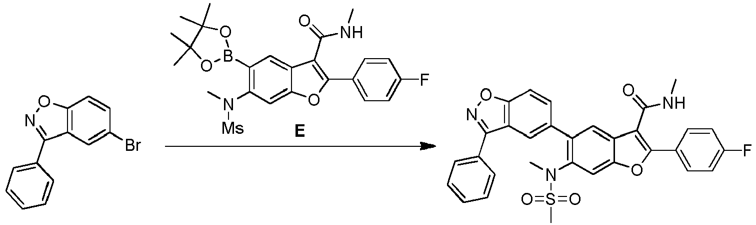

To a solution of 5-bromo-3-phenylbenzo[d]isoxazole (90 mg, 0.33 mmol), 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (Compound **E**, 150 mg, 0.30 mmol) and K₃PO₄·3H₂O (159 mg, 0.60 mmol) in 2 mL of dioxane and 0.2 mL of water was added Pd(dppf)Cl₂ (10 mg) under nitrogen. The mixture was heated at 100 °C for 4 h, then concentrated in vacuo and the residue was suspended in water. The mixture was extracted with EtOAc and the organic phase was washed with brine, dried over sodium sulfate. 2-(4-fluorophenyl)-N-methyl-6-(N-methylmethylsulfonamido)-5-(3-phenylbenzo[d]isoxazol-5-yl)benzofuran-3-carboxamide (120 mg, yield: 70%) was obtained after the prep-TLC (DCM : MeOH = 30 : 1). ¹H-NMR (CDCl₃, 400 MHz) δ 8.27∼8.29 (m, 2H), 7.94∼7.97 (m, 2H), 7.84 (s, 1H), 7.81 (s, 1H), 7.63∼7.67 (m, 2H), 7.52∼7.56 (m, 3H), 7.46∼7.48 (m, 1H), 7.20 (t*, J* = 8.8 Hz, 2H), 5.88 (brs, 1H), 3.18 (s, 3H), 2.98 (d, *J* = 4.8 Hz, 3H), 2.60 (s, 3H). (M+H)⁺: 570.

### Example 11 (illustrative)

### Preparation of Compound 84

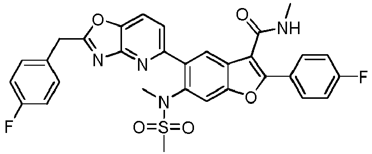

### Step 1 - Synthesis of 5-bromo-2-(4-fluorobenzyl)oxazolo[4,5-b]pyridine

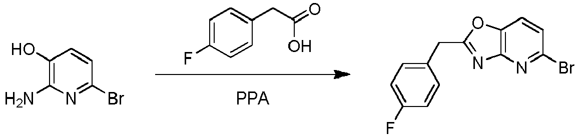

PPA (2 mL) was heated at 130 °C, and then 2-(4-fluorophenyl)acetic acid (400 mg, 2.6 mmol) and 2-amino-6-bromopyridin-3-ol (589 mg, 3.1 mmol) were added under N₂ protection. After stirred at 130 °C for 4 hours, the mixture was quenched with ice and NaOH (aq). Then the mixture was extracted with EtOAc, dried over Na₂SO₄, filtrated and concentrated. The residue was purified by column chromatography (PE : EtOAc = 15 : 1 to 10 : 1) to give the product of 5-bromo-2-(4-fluorobenzyl)oxazolo[4,5-b]pyridine (200 mg, yield: 25%). ¹H-NMR (CDCl₃, 400 MHz) δ 7.61 (d, *J* = 8.4 Hz, 1H), 7.41 (d, *J* = 8.4 Hz, 1H), 7.34 (dd, *J* = 8.0, 5.6 Hz, 2H), 7.02 (t, *J* = 8.0 Hz, 2H), 4.28 (s, 2H). MS (M+H)⁺: 307 / 309.

### Step 2 - Synthesis of 5-(2-(4-fluorobenzyl)oxazolo[4,5-b]pyridin-5-yl)-2-(4-fluorophenyl)-N-methyl-6-(N-methylmethylsulfonamido)benzofuran-3-carboxamide

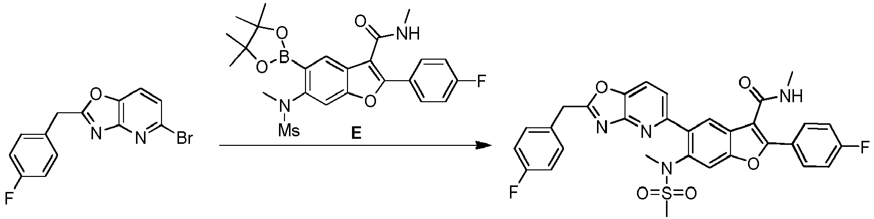

To a degassed solution of 5-bromo-2-(4-fluorobenzyl)oxazolo[4,5-b]pyridine (100 mg, 0.32 mmol), 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (Compound **E**, 196 mg, 0.39 mmol) and K₃PO₄·3H₂O (174 mg, 0.65 mmol) in 1,4-dioxane (2 mL) was added Pd(dppf)Cl₂ (20 mg) under N₂ protection. The reaction mixture was stirred at 100 °C for 16 hours. After filtered through a celite pad, the filtrate was concentrated. The residue was purified by basic prep-HPLC to give the product of 5-(2-(4-fluorobenzyl)oxazolo[4,5-b]pyridin-5-yl)-2-(4-fluorophenyl)-N-methyl-6-(N-methylmethylsulfonamido)benzofuran-3-carboxamide (20 mg, yield: 10%). ¹H-NMR (CDCl₃, 400 MHz) δ 7.89∼7.93 (m, 3H), 7.78 (d, *J* = 8.4 Hz, 1H), 7.59 (s, 1H), 7.54 (d, *J* = 8.4 Hz, 1H), 7.35 (dd, *J* = 8.8, 5.6 Hz, 2H), 7.13 (t, *J* = 8.8 Hz, 2H), 7.00 (t, *J* = 8.8 Hz, 2H), 5.87 (d, *J* = 4.8 Hz, 1H), 4.27 (s, 2H), 3.13 (s, 3H), 2.92 (d, *J* = 4.8 Hz, 3H), 2.78 (s, 3H). MS (M+H)⁺: 603.

### Example 12 (illustrative)

### Preparation of Compound 88

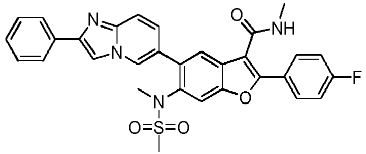

### Step 1 - Synthesis of 6-bromo-2-phenylimidazo[1,2-a]pyridine

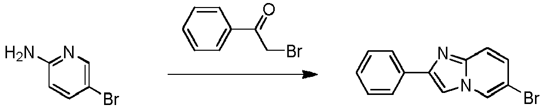

A solution of 5-bromopyridin-2-amine (1.0 g, 5.78 mmol), 2-bromoacetophenone (1.4 g, 7.03 mmol) in MeOH (20 mL) was heated at 80 °C overnight. The reaction mixture was concentrated in vacuo. The residue was suspended with water, and extracted with EtOAc. The organic layer was washed with brine, dried over Na₂SO₄, filtered and concentrated in vacuo. The residue was purified by column chromatography (PE : EtOAc = 5 : 1 to 2 : 1) to give product of 6-bromo-2-phenylimidazo[1,2-a]pyridine (800 mg, yield: 50%). ¹H-NMR (CDCl₃, 400 MHz) δ 8.27 (d, *J* = 0.8 Hz, 1H), 7.93∼7.95 (m, 2H), 7.84 (s, 1H), 7.53 (d, *J=* 9.6 Hz, 1H), 7.42∼7.47 (m, 2H), 7.33∼7.37 (m, 1H), 7.22∼7.25 (m, 1H). MS (M+H)⁺: 273 / 275.

### Step 2 - Synthesis of 2-(4-fluorophenyl)-N-methyl-6-(N-methylmethylsulfonamido)-5-(2-phenylimidazo[1,2-a]pyridin-6-yl)benzofuran-3-carboxamide

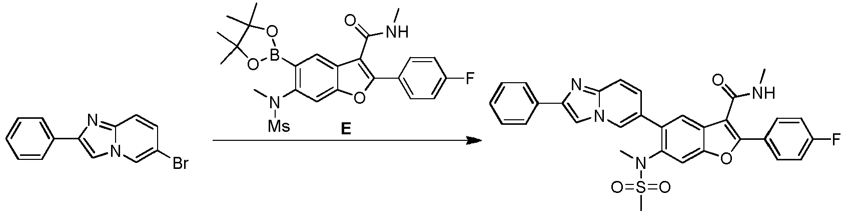

The procedure of 2-(4-fluorophenyl)-N-methyl-6-(N-methylmethylsulfonamido)-5-(2-phenylimidazo[1,2-a]pyridin-6-yl)benzofuran-3-carboxamide (30 mg, yield: 24%) was similar to step 4 of Example **1**. ¹H NMR (CDCl₃, 400 MHz) δ 8.38 (s, 1H), 7.89∼7.93 (m, 2H), 7.87 (s, 1H), 7.81 (d, *J* = 5.6 Hz, 2H), 7.55∼7.58 (m, 2H), 7.47 (s, 1H), 7.27∼7.32 (m, 3H), 7.14∼7.22 (m, 4H), 3.10 (d, *J* = 4.8 Hz, 3H), 3.04 (s, 3H), 2.98 (s, 3H). MS (M+H)⁺: 569.

### Example 13 (illustrative)

### Preparation of Compound 90

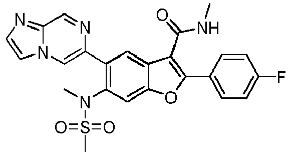

### Step 1 - Synthesis of 6-bromoimidazo[1,2-a]pyrazine

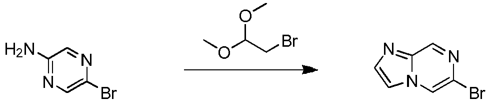

To a degassed solution of 5-bromopyrazin-2-amine (2.0 g, 0.13 mmol) and HBr (aq, 0.5 mL) in propan-2-ol (50 mL) was added 2-bromo-1,1-dimethoxyethane (3.9 g, 22.9 mmol). The reaction was heated to 80 °C for 10 hours. The reaction mixture was cooled to RT and extract with DCM. The organics were washed with NaHCO₃ (aq), brine and dried over Na₂SO₄. After concentrated, the crude product of 6-bromoimidazo[1,2-a]pyrazine (2.1 g, yield: 75%), which was used for next step without purification. ¹H-NMR (CDCl₃, 400 MHz) δ 8.86 (s, 1H), 8.24 (s, 1H), 7.79 (s, 1H), 7.65 (s, 1H). MS (M+H)⁺: 198 / 200.

### Step 2 - Synthesis of 2-(4-fluorophenyl)-5-(imidazo[1,2-a]pyrazin-6-yl)-N-methyl-6-(N-methylmethylsulfonamido)benzofuran-3-carboxamide

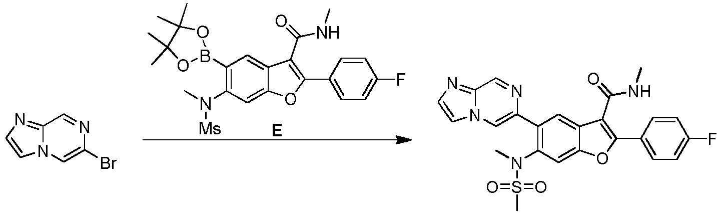

A solution of 6-bromoimidazo[1,2-a]pyrazine (80 mg, 0.4 mmol) and 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (Compound **E**, 200 mg, 0.2 mmol) in dry DMF (10 mL) was added Pd(dppf) Cl₂ (10 mg) and K₃PO₄ (80 mg, 0.38 mmol) under N₂. The mixture was heated to 80 °C and then stirred overnight. The reaction mixture was cooled to room temperature and filtered. After concentrated, the residue was purified by prep-HPLC to give the product of 2-(4-fluorophenyl)-5-(imidazo[1,2-a]pyrazin-6-yl)-N-methyl-6-(N-methylmethylsulfonamido)benzofuran-3-carboxamide (66 mg, yield: 34%). ¹H-NMR (CDCl₃, 400 MHz) δ 9.61 (s, 1H), 8.71 (s, 1H), 8.10 (s, 1H), 8.05 (s, 1H), 7.91∼7.95 (m, 3H), 7.61 (s, 1H), 7.21∼7.25 (m, 2H), 6.21 (s, 1H), 3.25 (s, 3H), 3.01 (s, 3H), 3.00 (s, 3H). MS (M+H)⁺: 494.

### Example 14 (illustrative)

### Preparation of Compound 91

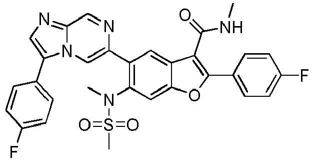

### Step 1 - Synthesis of 6-bromo-3-iodoimidazo[1,2-a]pyrazine

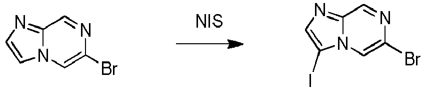

A degassed solution of 6-bromoimidazo[1,2-a]pyrazine (500 mg, 2.52 mmol) and NIS (852 mg, 3.79 mmol) in DMF (10 mL) was heated to 60 °C for 10 hours. The reaction mixture was cooled to RT and extract with DCM. The organics were washed with Na₂S₂O₃ (aq), brine and dried over Na₂SO₄. After concentrated, the crude product was purified by column (PE : EtOAc = 5 : 1) to obtain 6-bromo-3-iodoimidazo[1,2-a]pyrazine (530 mg, yield: 65%). ¹H-NMR (CDCl₃, 400 MHz) δ 8.75 (s, 1H), 8.18 (s, 1H), 7.82 (s, 1H). MS (M+H)⁺: 324 / 326.

### Step 2 - Synthesis of 6-bromo-3-(4-fluorophenyl)imidazo[1,2-a]pyrazine

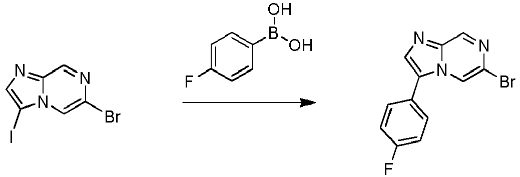

To a degassed solution of 6-bromo-3-iodoimidazo[1,2-a]pyrazine (100 mg, 0.31 mmol) and 4-fluorophenylboronic acid (43 mg, 0.31 mmol) in 1,4-dioxane (3 mL) were added Pd(dppf)Cl₂ (10 mg) and K₂CO₃ (77 mg, 0.56 mmol) under N₂. The mixture was heated to 100 °C overnight. After cooled to RT and filtered, the filtrate was washed with brine, dried over Na₂SO₄ and concentrated, the residue was purified by prep-TLC (PE : EtOAc = 5 : 1) to give 6-bromo-3-(4-fluorophenyl)imidazo[1,2-a]pyrazine (50 mg, yield: 22%). ¹H-NMR (CDCl₃, 400 MHz) δ 8.06 (t, *J =* 6.8 Hz, 2H), 7.45∼7.48 (m, 1H), 7.23 (t, *J =* 8.8 Hz, 1H), 7.07 (t, *J =* 8.4 Hz, 3H). MS (M+H)⁺: 292 / 294.

### Step 3 - Synthesis of 2-(4-fluorophenyl)-5-(3-(4-fluorophenyl)imidazo[1,2-a]pyrazin-6-yl)-N-methyl-6-(N-methylmethylsulfonamido)benzofuran-3-carboxamide

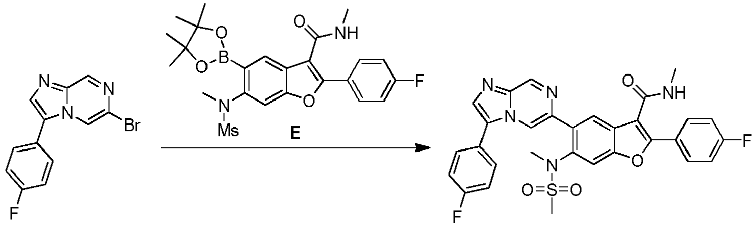

To a degassed solution of 6-bromo-3-(4-fluorophenyl)imidazo[1,2-a]pyrazine (78 mg, 0.27 mmol) and 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (Compound **E**, 150 mg, 0.3 mmol) in 1, 4-dioxane (3.0 mL) were added Pd(dppf)Cl₂ (10 mg) and K₃PO₄ (126 mg, 0.6 mmol) under N₂. The mixture was heated to 100 °C overnight. The reaction mixture was cooled to RT and filtered. The filtrate was washed with H₂O, brine, dried over Na₂SO₄. After concentrated, the residue was purified by prep-TLC (PE : EtOAc = 1 : 1) to give 2-(4-fluorophenyl)-5-(3-(4-fluorophenyl)imidazo[1,2-a]pyrazin-6-yl)-N-methyl-6-(N-methylmethylsulfonamido)benzofuran-3-carboxamide (30 mg, yield: 18%). ¹H-NMR (CDCl₃, 400 MHz) δ 9.27 (s, 1H), 8.64 (s, 1H), 8.03 (s, 1H), 7.89∼7.92 (m, 3H), 7.59∼7.62 (m, 2H), 7.52 (s, 1H), 7.20 (t, *J=* 8.8 Hz, 2H), 7.14 (t*, J* = 8.8 Hz, 2H), 5.99 (d*, J* = 4.0 Hz, 1H), 3.17 (s, 3H), 2.95 (s, 3H), 2.92 (d, *J=* 4.8 Hz, 3H). MS (M+H)⁺: 588.

### Example 15 (illustrative)

### Preparation of Compound 94

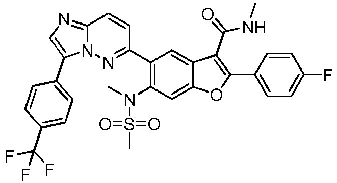

### Step 1 - Synthesis of 6-chloroimidazo[1,2-b]pyridazine

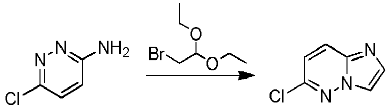

To a degassed solution of 6-chloropyridazin-3-amine (5.0 g, 38.6 mmol) and HBr (aq, 0.5 mL) in 2-bromo-1,1-dimethoxyethane (15.2 g, 77.2 mmol) in propan-2-ol (50 mL) was heated to 90 °C for 10 hours. The reaction mixture was cooled to RT and extract with DCM. The organics were washed with NaHCO₃ (aq), brine and dried over Na₂SO₄. After concentrated, the crude product of 6-chloroimidazo[1,2-b]pyridazine (4.7 g, yield: 80%), which was used to next step with no purification. ¹H-NMR (CDCl₃, 400 MHz) δ 7.90 (s, 1H), 7.85 (d, *J* = 9.6 Hz, 1H), 7.76 (s, 1H), 7.00 (d, *J* = 9.6 Hz, 1H). MS (M+H)⁺: 154.

### Step 2 - Synthesis of 6-chloro-3-iodoimidazo[1,2-b]pyridazine

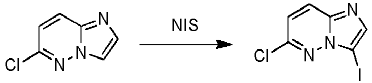

To a degassed solution of 6-chloroimidazo[1,2-b]pyridazine (1.0 g, 6.51 mmol) and NIS (2.2 g, 9.77 mmol) in MeCN (30 mL) was heated to 60 °C for 10 hours. The reaction mixture was cooled to RT and extract with DCM. The organics were washed with Na₂S₂O₃ (aq), brine and dried over Na₂SO₄. After concentrated, the crude product was purified by column (PE : EA = 5 : 1) to obtain 6-chloro-3-iodoimidazo[1,2-b]pyridazine (1.4 g, yield: 83%). ¹H-NMR (CDCl₃, 400 MHz) δ 7.83 (s, 1H), 7.80 (d, *J* = 4.8 Hz, 1H), 7.04 (d, *J* = 9.6 Hz, 1H). MS (M+H)⁺: 280.

### Step 3 - Synthesis of 6-chloro-3-(4-(trifluoromethyl)phenyl)imidazo[1,2-b]pyridazine

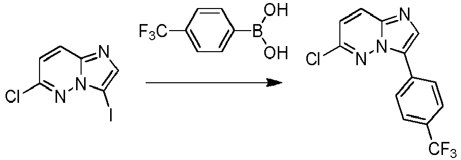

To a degassed solution of 6-chloro-3-iodoimidazo[1,2-b]pyridazine (100 mg, 0.36 mmol) and 4-(trifluoromethyl)phenylboronic acid (82 mg, 0.43 mmol) in 1, 4-dioxane (10.0 mL) was added Pd(dppf)Cl₂ (10 mg) and K₃PO₄ (98 mg, 0.72 mmol) under N₂. The mixture was heated to 100 °C overnight. The reaction mixture was cooled to RT and filtered. The filtrate was washed with H₂O, brine, dried over Na₂SO₄. After concentrated, the residue was purified by Prep-TLC to give 6-chloro-3-(4-(trifluoromethyl)phenyl)imidazo[1,2-b]pyridazine (95 mg, yield: 84%). ¹H-NMR (CDCl₃, 400 MHz) δ 8.12 (s, 2H), 8.09 (d, *J* = 6.8 Hz, 1H), 7.98 (d, *J* = 9.2 Hz, 1H), 7.70 (d, *J* = 8.0 Hz, 2H), 7.11 (d, *J* = 9.2 Hz, 1H). MS (M+H)⁺: 298.

### Step 4 - Synthesis of 2-(4-fluorophenyl)-N-methyl-6-(N-methylmethylsulfonamido)-5-(3-(4-(trifluoromethyl)phenyl)imidazo[1,2-b]pyridazin-6-yl)benzofuran-3-carboxamide

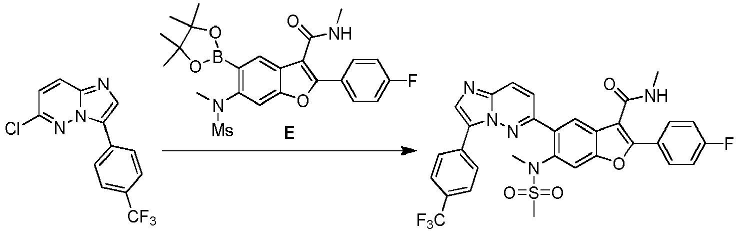

To a degassed solution of 6-chloro-3-(4-(trifluoromethyl)phenyl)imidazo[1,2-b]pyridazine (71 mg, 0.24 mmol) and 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (Compound E, 100 mg, 0.2 mmol) in 1, 4-dioxane (2.0 mL) was added Pd(dppf)Cl₂ (10 mg) and K₃PO₄ (63 mg, 0.3 mmol) under N₂. The mixture was heated to 100 °C overnight. The reaction mixture was cooled to RT and filtered. The filtrate was washed with H₂O, brine, dried over Na₂SO₄. After concentrated, the residue was purified by prep-TLC to give 2-(4-fluorophenyl)-N-methyl-6-(N-methylmethylsulfonamido)-5-(3-(4-(trifluoromethyl)phenyl)imidazo[1,2-b]pyridazin-6-yl)benzofuran-3-carboxamide (40 mg, yield: 30%). ¹H-NMR (CDCl₃, 400 MHz) δ 8.33 (d, *J* = 9.6 Hz, 1H), 8.05∼8.11 (m, 4H), 7.83∼7.87 (m, 2H), 7.66 (d, *J* = 8.4 Hz, 2H), 7.62 (s, 1H), 7.57 (t, *J* = 5.6 Hz, 2H), 7.16 (d, *J* = 8.8 Hz, 1H), 5.75 (d, *J* = 6.8 Hz, 1H), 3.21 (s, 3H), 2.91 (d, *J* = 4.8 Hz, 3H), 2.80 (s, 3H). MS (M+H)⁺: 638.

### Example 16 (illustrative)

### Preparation of Compound 96

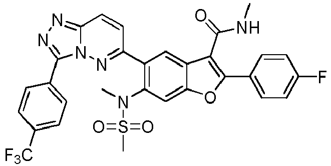

### Step 1 - Synthesis of 3-chloro-6-hydrazinylpyridazine

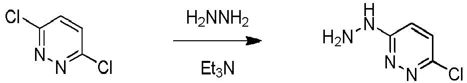

To a degassed solution of 3,6-dichloropyridazine (18 g, 120 mmol) and hydrazine (6.4 mL) in 1, 4-dioxane (80 mL) was added Et₃N (16.8 mL) under N₂. The mixture was heated to 100 °C overnight. The reaction mixture was concentrated and then it was poured into ice-water. After filtrated, the solid was collected and dried as the product of 3-chloro-6-hydrazinylpyridazine (14.7 g, yield: 84%). ¹H-NMR (DMSO-*d*₆, 400 MHz) δ 8.19 (s, 1H), 7.36 (d, *J* = 9.2 Hz, 1H), 7.05 (d, *J* = 9.2 Hz, 1H), 4.34 (s, 2H). MS (M+H)⁺: 145 / 147.

### Step 2 - Synthesis of N'-(6-chloropyridazin-3-yl)-4-(trifluoromethyl)benzohydrazide

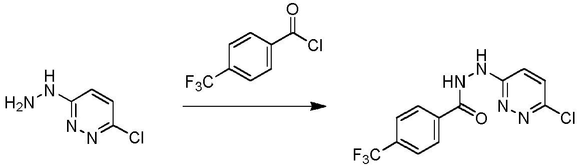

A degassed solution of 4-(trifluoromethyl)benzoic acid (1.0 g, 5.26 mmol) in SOCl₂ (10 mL) was heated at 100 °C for 2 hours. After concentrated and dissolved in DCM (10 mL), the resulting solution was added into a solution of 3-chloro-6-hydrazinylpyridazine (836 mg, 5.79 mmol) and Et₃N (1.14 mL) in DCM (10 mL) under N₂ dropwise. Then it was stirred at 25 °C for 8 hours and then the mixture was diluted with H₂O. After extracted with DCM, the organics were concentrated to afford the product of N'-(6-chloropyridazin-3-yl)-4-(trifluoromethyl)benzohydrazide (1.6 g, yield: 96%). ¹H-NMR (DMSO-*d*₆, 400 MHz) δ 10.83 (s, 1H), 9.35 (s, 1H), 8.08 (d, *J* = 8.0 Hz, 2H), 7.88 (d, *J* = 8.0 Hz, 2H), 7.54 (d, *J* = 9.2 Hz, 1H), 7.10 (d, *J* = 9.2 Hz, 1H). MS (M+H)⁺: 317 / 319.

### Step 3 - Synthesis of 6-chloro-3-(4-(trifluoromethyl)phenyl)-[1,2,4]triazolo[4,3-b]pyridazine

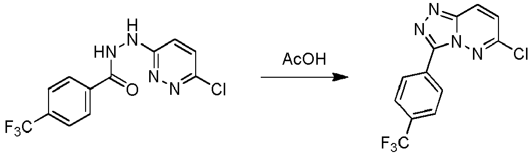

A stirred solution of N'-(6-chloropyridazin-3-yl)-4-(trifluoromethyl)benzohydrazide (200 mg, 0.63 mmol) in HCOOH (2 mL) under nitrogen was stirred at 100 °C for 5 hours and then the solvent was removed in vacuo to give a brown residue. The crude product was washed with NaHCO₃ (aq) and concentrated to obtained the product of 6-chloro-3-(4-(trifluoromethyl)phenyl)-[1,2,4]triazolo[4,3-b]pyridazine (100 mg, 53%) as a brown solid. ¹H-NMR (CDCl₃, 400 MHz) δ 8.57 (d, *J* = 8.0 Hz, 2H), 8.14 (d, *J* = 9.6 Hz, 1H), 7.77 (d, *J* = 8.0 Hz, 2H), 7.15 (d, = 9.6 Hz, 1H). MS (M+H)⁺: 299 / 301.

### Step 4 - Synthesis of 2-(4-fluorophenyl)-N-methyl-6-(N-methylmethylsulfonamido)-5-(3-(4-(trifluoromethyl)phenyl)-[1,2,4]triazolo[4,3-b]pyridazin-6-yl)benzofuran-3-carboxamide

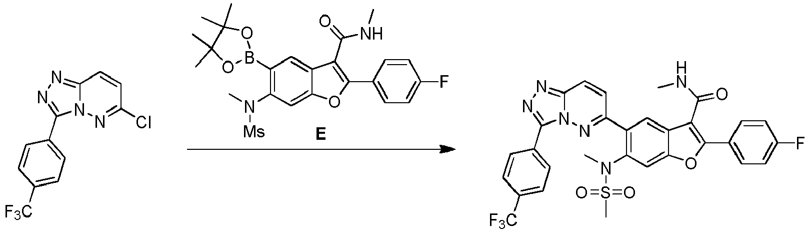

To a degassed solution of 6-chloro-3-(4-(trifluoromethyl)phenyl)-[1,2,4]triazolo[4,3-b]pyridazine (80 mg, 0.3 mmol) and 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (Compound E, 100 mg, 0.2 mmol) in 1, 4-dioxane (2.0 mL) were added Pd(dppf)Cl₂ (10 mg) and K₃PO₄ (70 mg, 0.4 mmol) under N₂. The mixture was heated to 100 °C overnight. The reaction mixture was cooled to RT and filtered. The filtrate was washed with H₂O, brine, dried over Na₂SO₄. After concentrated, the residue was purified by prep-HPLC to give 2-(4-fluorophenyl)-N-methyl-6-(N-methylmethylsulfonamido)-5-(3-(4-(trifluoromethyl) phenyl)-[1,2,4]triazolo[4,3-b]pyridazin-6-yl)benzofuran-3-carboxamide (20 mg, yield: 17%). ¹H-NMR (CDCl₃, 400 MHz) δ 8.59 (d, *J* = 8.0 Hz, 2H), 8.13∼8.22 (m, 3H), 7.84∼7.88 (m, 2H), 7.71 (d, *J* = 8.4 Hz, 2H), 7.60 (s, 1H), 7.42∼7.45 (m, 1H), 7.16∼7.20 (m, 2H), 5.80 (s, 1H), 3.25 (s, 3H), 2.92 (d, *J* = 4.8 Hz, 3H), 2.86 (s, 3H). MS (M+H)⁺: 639.

### Example 17 (illustrative)

### Preparation of Compound 97

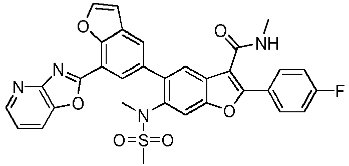

### Step 1 - Synthesis of 2-(5-bromo-2,3-dihydrobenzofuran-7-yl)oxazolo[4,5-b]pyridine

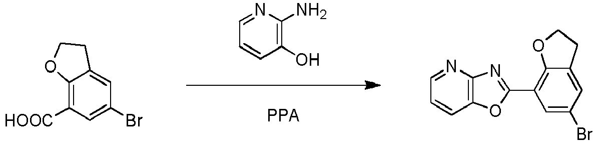

A mixture of 5-bromo-2,3-dihydrobenzofuran-7-carboxylic acid (0.80 g, 3.29 mmol), 2-aminopyridin-3-ol (0.40 g, 3.63 mmol) in PPA (6 mL) was stirred at 140 °C for 2 h. The reaction mixture was added to water and basified to pH = 8, then extracted with ethyl acetate and washed with brine, dried over Na₂SO₄. After concentrated, the residue was purified by column chromatography (PE : EtOAc = 1 : 2) to give the product of 2-(5-bromo-2,3-dihydrobenzofuran-7-yl)oxazolo[4,5-b]pyridine (600 mg, yield: 58%). ¹H-NMR (CDCl₃, 400 MHz) δ 8.59 (s, 1H), 8.18 (s, 1H), 7.90 (d, *J* = 6.4 Hz, 1H), 7.45 (s, 1H), 7.28∼7.30 (m, 1H), 4.84∼4.88 (m, 2H), 3.32∼3.34 (m, 2H). MS (M+H)⁺: 317 / 319.

### Step 2 - Synthesis of 2-(5-bromobenzofuran-7-yl)oxazolo[4,5-b]pyridine

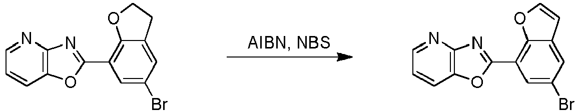

To a solution of 2-(5-bromo-2,3-dihydrobenzofuran-7-yl)oxazolo[4,5-b]pyridine (400 mg, 1.26 mmol) and AIBN (20 mg, 0.12 mmol) in CCl₄ (2 mL), NBS (250 mg, 1.4 mmol) was added. The reaction mixture was refluxed for 2 hours. The reaction mixture was quenched with water and the mixture was extracted with CH₂Cl₂. The organic layer was washed with brine, dried over Na₂SO₄ and concentrated in vacuo. The residue was purified by column chromatography (PE : EtOAc = 5 : 1) to give the product of 2-(5-bromobenzofuran-7-yl)oxazolo[4,5-b]pyridine (300 mg, yield: 76%). ¹H-NMR (CDCl₃, 400 MHz) δ 8.58 (d, *J* = 4.0 Hz, 1H), 8.33 (d, *J* = 1.6 Hz, 1H), 7.88∼7.91 (m, 2H), 7.81∼7.83 (m, 1H), 7.28∼7.31 (m, 1H), 6.79 (d, *J* = 2.0 Hz, 1H). MS (M+H)⁺: 315 / 317.

### Step 3 - Synthesis of 2-(4-fluorophenyl)-N-methyl-6-(N-methylmethylsulfonamido)-7'-(oxazolo[4,5-b]pyridin-2-yl)-[5,5'-bibenzofuran]-3-carboxamide

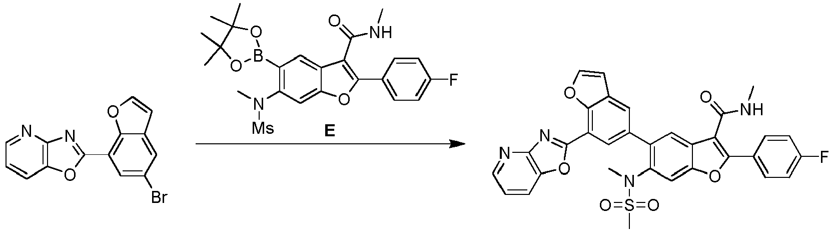

To a stirring solution of 2-(5-bromobenzofuran-7-yl)oxazolo[4,5-b]pyridine (80 mg, 0.25 mmol) in dioxane (2 mL), K₃PO₄·3H₂O (160 mg, 0.6 mmol) and 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (Compound **E,** 100 mg, 0.20 mmol) were added, then Pd(dppf) Cl₂ (5 mg) was added under N₂ protection, and the mixture was stirred at 80 °C overnight. The mixture was concentrated in vacuo. The residue was purified by prep-HPLC to give the product of 2-(4-fluorophenyl)-N-methyl-6-(N-methylmethylsulfonamido)-7'-(oxazolo[4,5-b]pyridin-2-yl)-[5,5'-bibenzofuran]-3-carboxamide (35 mg, yield: 30%). ¹H-NMR (CDCl₃, 400 MHz) δ 8.23∼8.51 (m, 1H), 8.23 (d, *J* = 1.2 Hz, 1H), 7.83∼7.88 (m, 6H), 7.55 (s, 1H), 7.24∼7.27 (m, 1H), 7.08∼7.13 (m, 2H), 6.23∼6.24 (br s, 1H), 3.08 (s, 3H), 2.94 (d, *J* = 4.8 Hz, 3H), 2.65 (s, 3H). MS (M+H)⁺: 611.

### Example 18 (illustrative)

### Preparation of Compound 99

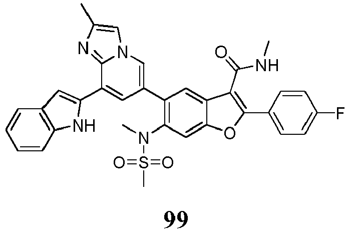

### Step 1 - Synthesis of 6,8-dibromo-2-methylimidazo[1,2-a]pyridine

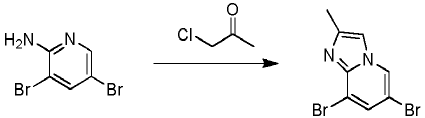

A mixture of 3,5-dibromopyridin-2-amine (1.8 g, 7 mmol) and 1-chloropropan-2-one (1.5 g, 16 mmol) in EtOH (20 mL) was stirred at 100 °C for 48 h. The mixture was then concentrated in vacuum. The residue was purified by chromatography (DCM : MeOH = 10 : 1) to give the desired product of 6,8-dibromo-2-methylimidazo[1,2-a]pyridine (1 g, yield: 48.3%). ¹H-NMR (DMSO-*d*₆, 400 MHz) δ 8.84 (s, 1H), 7.76 (s, 1H), 7.69 (s, 1H), 2.31 (s, 3H). MS (M+H)⁺: 289 / 291 / 293.

### Step 2 - Synthesis of 6-bromo-8-(1H-indol-2-yl)-2-methylimidazo[1,2-a]pyridine

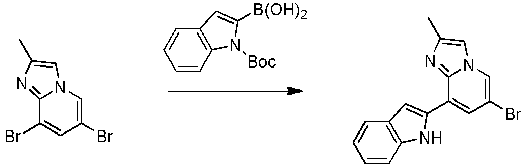

To a degassed solution of (1-(tert-butoxycarbonyl)-1H-indol-2-yl) boronic acid (300 mg, 1.15 mmol), 6,8-dibromo-2-methylimidazo[1,2-a]pyridine (1 g, 3.45 mmol) and K₃PO₄ (917 mg, 3.45 mmol) in dry DMF (5 mL) was added Pd(dppf)Cl₂ (84 mg, 0.12 mmol) under N₂. The mixture was heated to 80 °C and then stirred overnight. The reaction mixture was cooled to RT, diluted with EtOAc and filtered. The filtrate was washed with H₂O, brine, dried over Na₂SO₄. After concentrated, the residue was purified by prep-TLC (PE : EtOAc = 2 : 1) to give the product of 6-bromo-8-(1H-indol-2-yl)-2-methylimidazo[1,2-a]pyridme (150 mg, yield: 30.7%). ¹H-NMR (CDCl₃, 400 MHz) δ 11.86 (s, 1H), 8.02 (d, *J* = 1.6 Hz, 1H), 7.66 (d, *J* = 1.6 Hz, 1H), 7.58∼7.60 (m, 1H), 7.47∼7.50 (m, 1H), 7.33∼7.35 (m, 1H), 7.27 (s, 1H), 7.03∼7.17 (m, 1H), 6.49 (t, *J* = 2.0 Hz, 1H), 2.46 (s, 3H). MS (M+H)⁺: 326 / 328.

### Step 3 - Synthesis of 5-(8-(1H-indol-2-yl)-2-methylimidazo[1,2-a]pyridin-6-yl)-2-(4-fluorophenyl)-N-methyl-6-(N-methylmethylsulfonamido)benzofuran-3-carboxamide

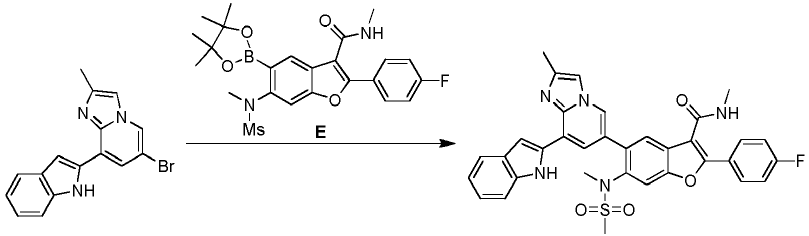

To a degassed solution of 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (Compound **E,** 177 mg, 0.35 mmol), 6-bromo-8-(1H-indol-2-yl)-2-methylimidazo[1,2-a]pyridine (150 mg, 0.46 mmol) and K₃PO₄ (281 mg, 1.06 mmol) in dioxane / H₂O (1.5 mL / 0.4 mL) were added Pd₂(dba)₃ (20 mg, 0.02 mmol) and X-phos (17 mg, 0.04 mmol) under N₂. The mixture was heated to 80 °C and then stirred overnight. The reaction mixture was cooled to RT, diluted with EtOAc and filtered. The filtrate was washed with H₂O, brine, dried over Na₂SO₄. After concentrated, the residue was purified by prep-TLC (PE : EtOAc = 1 : 1) to give the desired product of 5-(8-(1H-indol-2-yl)-2-methylimidazo[1,2-a]pyridin-6-yl)-2-(4-fluorophenyl)-N-methyl-6-(N-methylmethylsulfonamido)benzofuran-3-carboxamide (120 mg, yield: 42%). ¹H-NMR (CDCl₃, 400 MHz) δ 11.97 (s, 1H), 8.13 (d, *J* = 1.2 Hz, 1H), 7.89∼7.93 (m, 3H), 7.90 (d, *J* = 1.2 Hz, 1H), 8.62 (d, *J* = 7.2 Hz, 2H), 7.55 (d, *J* = 8.4 Hz, 1H), 7.42 (s, 1H), 7.08∼7.22 (m, 5H), 5.85 (d, *J* = 4.8 Hz, 1H), 3.10 (s, 3H), 2.96 (d, *J* = 4.8 Hz, 3H), 2.88 (s, 3H), 2.56 (s, 3H). MS (M+H)⁺: 622.

### Example 19

### Preparation of Compound 100

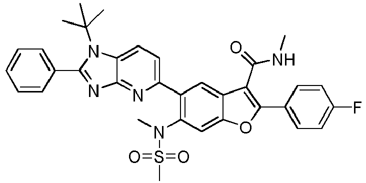

### Step 1 - Synthesis of N³-(tert-butyl)-6-chloropyridine-2,3-diamine and 6-chloropyridine-2,3-diamine

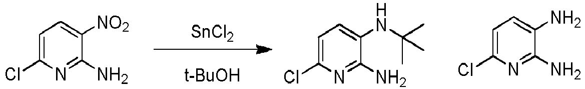

A mixture of 6-chloro-3-nitropyridin-2-amine (15 g, 86.4 mmol) and SnCl₂·2H₂O (98 g, 0.43 mol) in ethyl acetate / 2-methylpropan-2-ol (450 mL / 90 mL) was stirred at 60 °C for 1 h. Then NaBH₄ (1.6 g, 43.2 mmol) was added at 60 °C and the resultant mixture was stirred at the same temperature for another 3 h. Water was added and the mixture was extracted. The organic layer was washed with NaHCO₃ solution and brine, dried over Na₂SO₄ and concentrated in vacuo. The residue was purified by column chromatography (EA : DCM = 1 : 1) to give the product of N³-(tert-butyl)-6-chloropyridine-2,3-diamine (4.0 g, yield: 30%) and 6-chloropyridine-2,3-diamine (5.0 g, yield: 43%). ¹H-NMR (DMSO-*d*₆, 400 MHz) δ 6.84 (d, *J* = 8.0 Hz, 1H), 6.37 (d, *J* = 8.0 Hz, 1H), 5.99 (s, 2H), 3.98 (s, 1H), 1.20 (s, 9H), MS (M+H)⁺: 200 / 202. ¹H-NMR (DMSO-*d*₆, 400 MHz) δ 6.65 (d, *J* = 8.0 Hz, 1H), 6.31 (d, *J* = 8.0 Hz, 1H), 5.76 (s, 2H), 4.73 (s, 2H), MS (M+H)⁺: 144 / 146.

### Step 2 - Synthesis of 1-(tert-butyl)-5-chloro-2-phenyl-1H-imidazo[4,5-b]pyridine

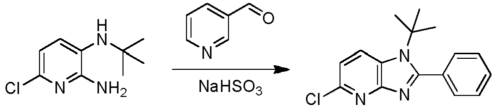

A mixture of benzaldehyde (266 mg, 2.5 mmol) in MeOH (5 mL) was added dropwise to a mixture of N³-(tert-butyl)-6-chloropyridine-2,3-diamine (500 mg, 2.5 mmol) and NaHSO₃ (313 mg, 3.0 mmol) in MeOH (30 mL). The mixture was stirred at 100 °C for 2 h. The mixture was filtered. The filtrate was concentrated and the residue was washed with water and CH₂Cl₂, then the residue was dried to give the product of 1-(tert-butyl)-5-chloro-2-phenyl-1H-imidazo[4,5-b]pyridine (300 mg, yield: 42%) which was directly in the next step without further purification.

### Step 3 - Synthesis of 5-(1-(tert-butyl)-2-phenyl-1H-imidazo[4,5-b]pyridin-5-yl)-2-(4-fluorophenyl)-N-methyl-6-(N-methylmethylsulfonamido)benzofuran-3-carboxamide

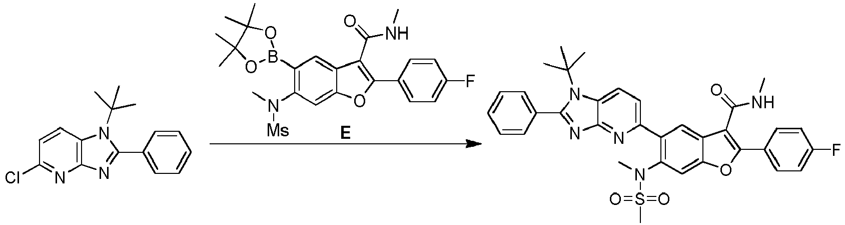

To a degassed solution of 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (Compound **E,** 176 mg, 0.35 mmol), 1-(tert-butyl)-5-chloro-2-phenyl-1H-imidazo[4,5-b]pyridine (150 mg, 0.52 mmol) and K₃PO₄·3H₂O (280 mg, 1.10 mmol) in dioxane / H₂O (1 mL / 0.1 mL) were added Pd₂(dba)₃ (20 mg, 0.02 mmol) and X-phos (17 mg, 0.04 mmol) under N₂. The mixture was heated to 80 °C and then stirred overnight. The reaction mixture was cooled to RT, diluted with EtOAc and filtered. The filtrate was washed with H₂O, brine, dried over Na₂SO₄. After concentrated, the residue was purified by prep-TLC (PE : EtOAc = 1 : 1) to give the desired product of 5-(1-(tert-butyl)-2-phenyl-1H-imidazo[4,5-b]pyridin-5-yl)-2-(4-fluorophenyl)-N-methyl-6-(N-methylmethylsulfonamido)benzofuran-3-carboxamide (100 mg, yield: 46%). ¹H-NMR (CDCl₃, 400 MHz) δ 8.06∼8.08 (m, 1H), 7.92∼7.99 (m, 3H), 7.52∼7.57 (m, 2H), 7.33∼7.40 (m, 5H), 7.08∼7.12 (m, 2H), 6.68 (s, 1H), 3.11 (s, 3H), 2.93 (s, 3H), 2.84 (s, 3H), 1.58 (s, 9H). MS (M+H)⁺: 626.

Compounds **101** and **102,** depicted in the table below, were prepared using the method described above and substituting the appropriate reactants and reagents.

| **Compound** | **Structure** | **NMR** | **MS (M+H)⁺** |
|---|---|---|---|
| **101** | 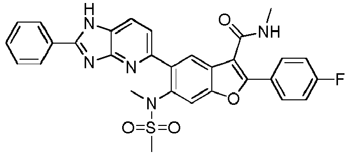 | ¹H-NMR (CDCl₃, 400 MHz) δ 8.16∼8.18 (s, 2H), 8.09∼8.11 (m, 2H), 7.94∼8.00 (m, 3H), 7.44∼7.51 (m, 5H), 7.10∼7.18 (m, 3H), 3.31 (s, 3H), 3.02 (d, *J* = 4.4 Hz, 3H), 2.83 (s, 3H). | **570** |
| **102** | 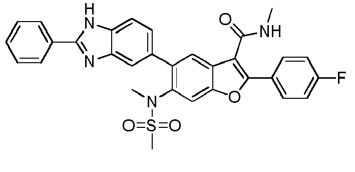 | ¹H-NMR (Methanol-*d*4, 400 MHz) δ 8.12∼8.14 (m, 2H), 7.89∼7.97 (m, 4H), 7.82∼7.85 (m, 1H), 7.67∼7.73 (m, 5H), 7.24∼7.29 (m, 2H), 3.15 (s, 3H), 2.96 (s, 3H), 2.93 (s, 3H). | **569** |

### Example 20 (illustrative)

### Preparation of Compound 103

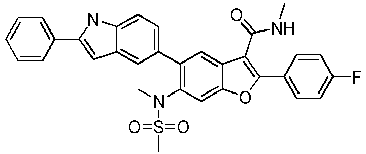

### Step 1 - Synthesis of 5-bromo-2-phenyl-1H-indole

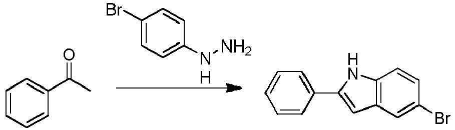

To a suspension of acetophenone (1.03 g, 5.0 mmol) and 4-bromophenylhydrazine hydrochloride (1.12 g, 5.0 mmol) in ethanol (5 mL) was added a few drops of glacial acetic acid. The reaction was stirred at 80 °C for 2 h. Solvent was evaporated to yield the phenylhydrazone intermediate, which was added to polyphosphoric acid (20 g). The reaction mixture was stirred at 120 °C for 2 h. The mixture was poured into crashed ice and then neutralized with 1 M NaOH and extracted with CH₂Cl₂. The combined organic extracts were washed with water, dried over anhydrous Na₂SO₄, and evaporated to give 5-bromo-2-phenyl-1H-indole (0.92 g, 68%) as a pale yellow solid. ¹H-NMR (CDCl₃, 400 MHz) δ 8.29 (s, 1H), 7.68 (s, 1H), 7.57∼7.62 (m, 2H), 7.36∼7.40 (m, 2H), 7.23∼7.29 (m, 1H), 7.18∼7.20 (m, 2H), 6.69 (s, 1H). MS (M+H)⁺: 272 / 274.

### Step 2 - Synthesis of 2-(4-fluorophenyl)-N-methyl-6-(N-methylmethylsulfonamido)-5-(2-phenyl-1H-indol-5-yl)benzofuran-3-carboxamide

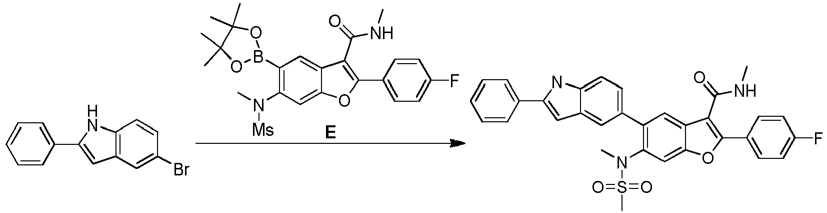

To a degassed solution of 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (Compound **E,** 147 mg, 0.29 mmol), 5-bromo-2-phenyl-1H-indole (80 mg, 0.29 mmol) and K₃PO₄·3H₂O (124 mg, 0.58 mmol) in DMF (3 mL) were added Pd(dppf) Cl₂ (10 mg) under N₂. The mixture was heated to 90 °C for 3 hours. The reaction mixture was cooled to RT, diluted with EtOAc and filtered. The filtrate was washed with H₂O, brine, dried over Na₂SO₄. After concentrated, the residue was purified by prep-HPLC to give the desired product of 2-(4-fluorophenyl)-N-methyl-6-(N-methylmethylsulfonamido)-5-(2-phenyl-1H-indol-5-yl)benzofuran-3-carboxamide (30 mg, yield: 17.9%). ¹H-NMR (CDCl₃, 400 MHz) δ 8.49 (s, 1H), 7.92∼7.95 (m, 2H), 7.78 (s, 1H), 7.62 (s, 1H), 7.53∼7.55 (m, 2H), 7.30∼7.36 (m, 3H), 7.20∼7.24 (m, 2H), 7.14∼7.18 (m, 2H), 7.04∼7.06 (m, 1H), 6.52 (s, 1H), 5.82 (d, *J* = 4.8 Hz, 1H), 3.00 (s, 3H), 2.88 (d, *J* = 4.8 Hz, 3H), 2.29 (s, 3H). MS (M+H)⁺: 568.

### Example 21 (illustrative)

### Preparation of Compound 104

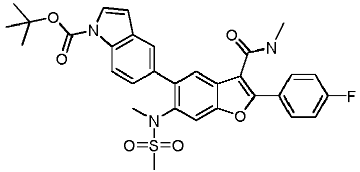

### Step 1 - Synthesis of tert-butyl 5-chloro-1H-indole-1-carboxylate

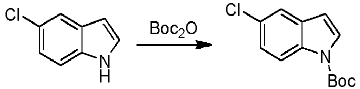

To a solution of 5-chloro-1H-indole (1.5 g, 9.9 mmol) and DMAP (120 mg, 0.99 mmol) in dry DCM (20 mL) Boc₂O (3.24 g) was added dropwise, and then the mixture was stirred at 25 °C. The mixture was stirred to at 25 °C for overnight. The solvent was removed by vacuum. The residue was purified by column chromatography (PE : EA = 50 : 1) to give the product of tert-butyl 5-chloro-1H-indole-1-carboxylate (2.4 g, yield: 96%). ¹H-NMR (CDCl₃, 400 MHz) δ 7.98 (d, *J* = 8.0 Hz, 1H), 7.51 (d, *J* = 4.0 Hz, 1H), 7.44 (d, *J* = 4.0 Hz, 1H), 7.16∼7.19 (m, 1H), 6.41 (d, *J* = 4.0 Hz, 1H), 1.58 (s, 12H). MS (M+H)⁺: 252.

### Step 2 - Synthesis of tert-butyl 5-(4,4,5,5-tetramethyl-1,3,2-dioxaborolan-2-yl)-1H-indole-1-carboxylate

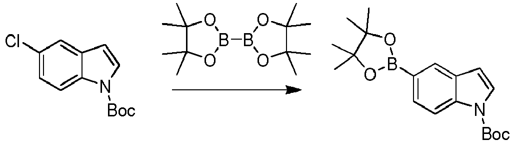

To a degassed solution of tert-butyl 5-chloro-1H-indole-1-carboxylate (1.0 g, 3.97 mmol) and 4,4,4',4',5,5,5',5'-octamethyl-2,2'-bi(1,3,2-dioxaborolane) (1.11 g, 4.37 mmol) in dry DMF (10 mL) was added Pd(dba)₃ (10 mg), x-Phos (10 mg) and AcOK (780 mg, 7.95 mmol) under N₂. The mixture was heated to 100 °C and then stirred overnight. The reaction mixture was cooled to RT and filtered. The filtrate was washed with EA, brine, dried over Na₂SO₄. After concentrated, the residue was purified by column (PE : EA = 50 : 1) to give the product of tert-butyl 5-(4,4,5,5-tetramethyl-1,3,2-dioxaborolan-2-yl)-1H-indole-1-carboxylate (1.0 g, yield: 73%). ¹H-NMR (CDCl₃, 400 MHz) δ 8.12 (d, *J* = 8.0 Hz, 1H), 8.04 (s, 1H), 7.74 (d, *J* = 12.0 Hz, 1H), 7.54 (d, *J* = 4.0 Hz, 1H), 6.54 (d, *J* = 4.0 Hz, 1H), 1.65 (s, 9H), 1.35 (s, 12H). MS (M+H)⁺: 344.

### Step 3 - Synthesis of tert-butyl 5-(2-(4-fluorophenyl)-3-(methylcarbamoyl)-6-(N-methylmethylsulfonamido)benzofuran-5-yl)-1H-indole-1-carboxylate

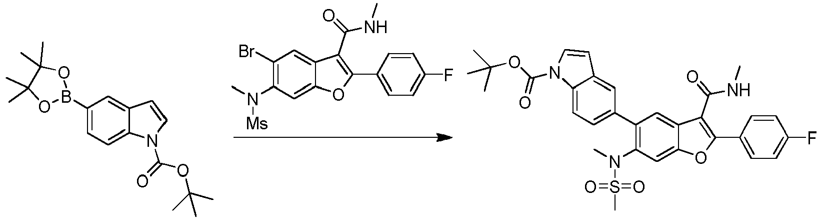

To a degassed solution of tert-butyl 5-(4,4,5,5-tetramethyl-1,3,2-dioxaborolan-2-yl)-1H-indole-1-carboxylate (0.5 g, 0.88 mmol) and 5-bromo-2-(4-fluorophenyl)-N-methyl-6-(N-methylmethylsulfonamido)benzofuran-3-carboxamide (332 mg, 0.96 mmol) in dry DMF (10 mL) was added Pd(dppf)Cl₂ (10 mg) and K₃PO₄ (468 mg, 1.76 mmol) under N₂. The mixture was heated to 100 °C and then stirred overnight. The reaction mixture was cooled to RT and filtered. The filtrate was washed with EA, brine, dried over Na₂SO₄. After concentrated, the residue was purified by column (PE : EA = 2 : 1) to give the product of tert-butyl 5-(2-(4-fluorophenyl)-3-(methylcarbamoyl)-6-(N-methylmethylsulfonamido) benzofuran-5-yl)-1H-indole-1-carboxylate (400 mg, yield: 76.9%). ¹H-NMR (CDCl₃, 400 MHz) 8.17 (d, *J* = 8.0 Hz, 1H), 7.92∼7.96 (m, 2H), 7.78 (s, 1H), 7.60∼7.63 (m, 3H), 7.36 (d, *J* = 1.6 Hz, 1H), 7.17 (t, *J* = 8.0 Hz, 2H), 6.58 (d, *J* = 4.0 Hz, 1H), 5.88 (d, *J* = 4.0 Hz, 1H), 3.13 (s, 3H), 2.96 (d, *J* = 4.0 Hz, 3H), 2.49 (s, 3H), 1.68 (s, 9H). (M+H)⁺: 592.

### Example 22

### Preparation of Compound 105

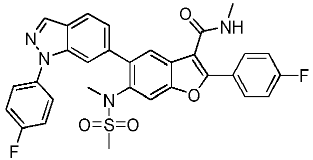

### Step 1 - Synthesis of 6-bromo-1-(4-fluorophenyl)-1H-indazole

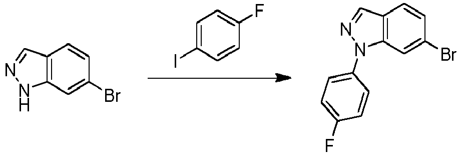

To a solution of 6-bromo-1H-indazole (500 mg, 2.54 mmol) and 1-fluoro-4-iodobenzene (845 mg, 3.81 mmol) in Dioxane (10 mL), Cs₂CO₃ (2.07 g, 6.35 mmol), CuI (48 mg, 0.25 mmol) and trans - N,N'-dimethyl-1,2-cyclohexanediamine (50 mg, 0.35 mmol) were added. The reaction mixture was stirred at 100 °C overnight. The reaction mixture was filtered and the filtrate was concentrated. The residue was purified by column chromatography (PE : EtOAc = 20 : 1) to provide 6-bromo-1-(4-fluorophenyl)-1H-indazole (400 mg, yield: 55%).

### Step 2 - Synthesis of 2-(4-fluorophenyl)-5-(1-(4-fluorophenyl)-1H-indazol-6-yl)-N-methyl-6-(N-methylmethylsulfonamido)benzofuran-3-carboxamide

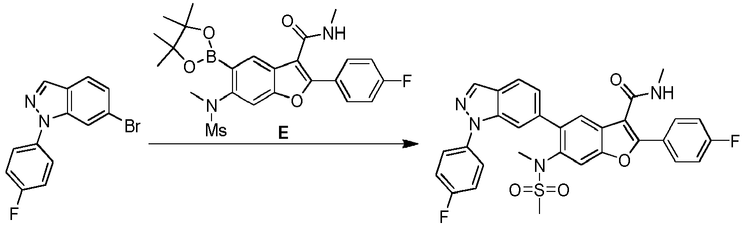

The procedure of 2-(4-fluorophenyl)-5-(1-(4-fluorophenyl)-1H-indazol-6-yl)-N-methyl-6-(N-methylmethylsulfonamido)benzofuran-3-carboxamide (150 mg, yield 78%) was similar to that of Example **1.** ¹H-NMR (CDCl₃, 400 MHz) δ 8.23 (s, 1H), 7.95∼7.92 (m, 2H), 7.85 (t, 3H), 7.77∼7.73 (m, 2H), 7.60 (s, 1H), 7.30 (dd, *J₁* = *J₂* = 1.2 Hz, 1H), 7.24∼7.19 (m, 4H), 5.82 (s, 1H), 3.08 (s, 3H), 2.96 (d, *J=* 5.2 Hz, 3H), 2.74 (s, 3H). (M+H)⁺: 587.

Compounds **106-108,** depicted in the table below, were prepared using the method described above and substituting the appropriate reactants and reagents.

| **Compound** | **Structure** | **NMR** | **MS (M+H)⁺** |
|---|---|---|---|
| **106** | 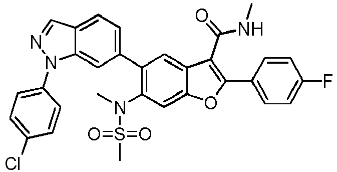 | ¹H-NMR (CDCl₃, 400 MHz) δ 8.21 (s, 1H), 7.81∼7.92 (m, 5H), 7.73 (d, *J* = 8.0 Hz, 2H), 7.57 (s, 1H), 7.47 (d, *J* = 8.0 Hz, 2H), 7.29 (d, *J* = 8.0 Hz, 1H), 7.16∼7.24 (m, 2H), 5.87 (d, *J* = 4.0 Hz, 1H), 3.05 (s, 3H), 2.93 (d, *J* = 4.0 Hz, 3H), 2.74 (s, 3H). | **604** |
| **107** | 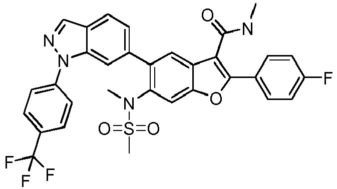 | ¹H-NMR (CDCl₃, 400 MHz) δ 8.27 (s, 1H), 7.97∼8.00 (m, 3H), 7.90∼7.94 (m, 2H), 7.85∼7.88 (m, 2H), 7.77∼7.80 (m, 2H), 7.58 (s, 1H), 7.34 (d, *J* = 8.0 Hz, 1H), 7.18∼7.23 (m, 2H), 5.87 (br s, 1H), 3.07 (s, 3H), 2.95 (d, *J* = 4.8 Hz, 3H), 2.81 (s, 3H). | **637** |
| **108** | 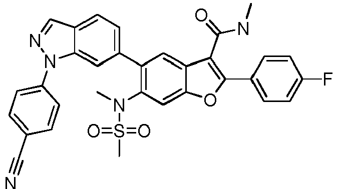 | ¹H-NMR (CDCl₃, 400 MHz) δ 8.49∼8.52 (m, 2H), 7.95∼8.06 (m, 9H), 7.68 (s, 1H), 7.37∼7.41 (m, 3H), 3.05 (s, 3H), 3.01 (s, 3H), 2.77 (d, *J* = 4.0 Hz, 3H). | **594** |

### Example 23

### Preparation of Compound 109

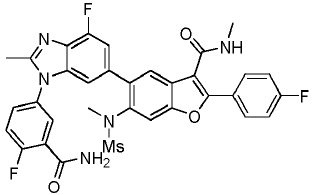

### Step 1 - Synthesis of 5-(1-(3-carbamoyl-4-fluorophenyl)-4-fluoro-2-methyl-1H-benzo[d]imidazol-6-yl)-2-(4-fluorophenyl)-N-methyl-6-(N-methylmethylsulfonamido)benzofuran-3-carboxamide

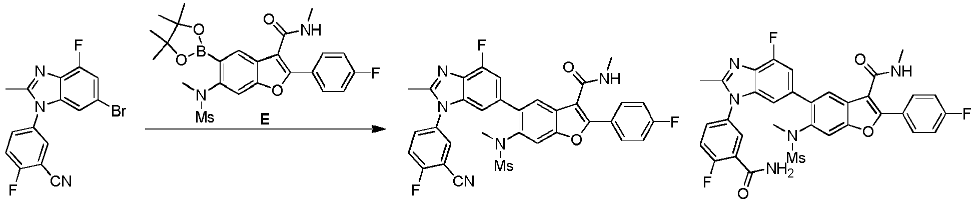

To a degassed solution of 5-(6-bromo-4-fluoro-2-methyl-1H-benzo[d]imidazol-1-yl)-2-fluorobenzonitrile (90 mg, 0.28 mmol) (prepared using similar method described in Example **1**) and 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (Compound **E,** 144 mg, 0.29 mmol) in 1,4-dioxane and H₂O (3.0 mL) were added Pd(dppf)Cl₂ (20 mg) and K₂CO₃ (64 mg, 0.4 mmol) under N₂. The mixture was heated at 110 °C overnight. The reaction mixture was cooled to room temperature and filtered. The filtrate was washed with H₂O, brine, dried over Na₂SO₄. After concentrated, the residue was purified by prep-TLC to give Compound **32** and 5-(1-(3-carbamoyl-4-fluorophenyl)-4-fluoro-2-methyl-1H-benzo[d]imidazol-6-yl)-2-(4-fluorophenyl)-N-methyl-6-(N-methylmethylsulfonamido)benzofuran-3-carboxamide (20 mg / 30 mg, yield: 12% / 17%). ¹H-NMR (CDCl₃, 400 MHz) δ 8.05∼8.08 (m, 3H), 7.69 (s, 2H), 7.53 (s, 1H), 7.40 (s, 1H), 7.27 (t, *J* = 8.0 Hz, 1H), 7.12 (t, *J* = 8.4 Hz, 2H), 6.95 (s, 1H), 6.87 (d, *J*= 10.8 Hz, 1H), 3.11 (s, 3H), 3.07 (d, *J* = 4.4 Hz, 3H), 2.75 (s, 3H), 1.92 (s, 3H). MS (M+H)⁺: 644. ¹H-NMR (CDCl₃, 400 MHz) δ 7.92∼7.97 (m, 1H), 7.89∼7.91 (m, 2H), 7.71 (s, 1H), 7.51∼7.55 (m, 1H), 7.45 (s, 1H), 7.27∼7.32 (m, 1H), 7.11 (t, *J* = 6.4 Hz, 2H), 6.95∼6.98 (m, 2H), 6.76 (d, *J* = 8.0 Hz, 1H), 6.42 (s, 1H), 6.03 (s, 1H), 2.94 (d, *J* = 4.8 Hz, 3H), 2.93 (s, 3H), 2.79 (s, 3H), 2.35 (s, 3H). MS (M+H)⁺: 662.

Compound **110,** depicted in the table below, was prepared using the method described above and substituting the appropriate reactants and reagents.

| **Compound** | **Structure** | **NMR** | **MS (M+H)⁺** |
|---|---|---|---|
| **110** | 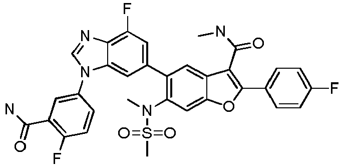 | ¹H-NMR (CDCl₃, 400 MHz) δ 8.16 (s, 1H), 8.05 (s, 1H), 7.86∼7.89 (m, 2H), 7.77 (s, 2H), 7.49 (s, 1H), 7.42 (s, 1H), 7.32 (t, *J* = 8.8 Hz, 1H), 7.13 (t, *J* = 8.8 Hz, 2H), 7.06 (d, *J* = 10.8 Hz, 1H), 6.72 (d, *J* = 10.6 Hz, 1H), 6.00 (s, 1H), 5.91 (s, 1H), 2.98 (s, 3H), 2.92 (d, *J* = 4.8 Hz, 3H), 2.82 (s, 3H). | **648** |

### Example 24 (illustrative)

### Preparation of Compound 111

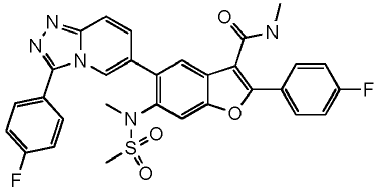

### Step 1 - Synthesis of 5-([1,2,4]triazolo[4,3-a]pyridin-6-yl)-2-(4-fluorophenyl)-N-methyl-6-(N-methylmethylsulfonamido)benzofuran-3-carboxamide

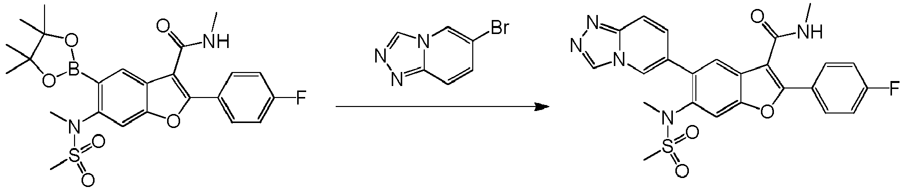

To a degassed solution of 6-bromo-[1,2,4]triazolo[4,3-a]pyridine (177 mg, 0.90 mmol) and 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (300 mg, 0..60 mmol) in 1,4-dioxane (8 mL) and water (200 µl) was added K₃PO₄ (380 mg, 1.79 mmol) and 1,1'-Bis(di-tert-butylphosphino)ferrocene palladium dichloride (39 mg, 0.06 mmol) under N₂ protection. The resulting mixture was heated to 80 °C and stirred at this temperature for 2 hours, then heated to 100 °C and stirred for 2 hours. The reaction was cooled, filtered through a pad of the celite and washed with ethyl acetate. The combined filtrate was evaporated *in vacuo.* The resulting residue was purified using column chromatography (eluted with 0-3% MeOH / DCM) to provide 5-([1,2,4]triazolo [4,3-a pyridin-6-yl)-2-(4-fluorophenyl)-N-methyl-6-(N-methylmethylsulfonamido) benzofuran-3-carboxamide] (230 mg, yield: 78%).

### Step 2 Synthesis of 5-(3-bromo-[1,2,4]triazolo[4,3-a]pyridin-6-yl)-2-(4-fluorophenyl)-N-methyl-6-(N-methylmethylsulfonamido)benzofuran-3-carboxamide

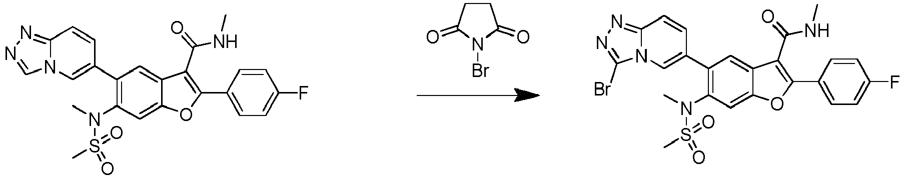

To a solution of 2-(4-fluorophenyl)-5-(11-fluoropyrido[3',2':4,5] pyrimido[1,6-a]indol-2-yl)-N-methyl-6-(N-methylmethylsulfonamido)benzofuran-3-carboxamide (210 mg, 0.43 mmol) in DCM (10 ml) and MeOH (200 µl) was added NBS (151 mg, 0.85 mmol). The resulting mixture was stirred at RT for 2 hours, then heated to 40 °C and stirred for 2 hours. Concentrated *in vacuo.* The resulting residue was purified using column chromatography (eluted with 0-3% MeOH / DCM) to provide 5-(3-bromo-[1,2,4]triazolo[4,3-a]pyridin-6-yl)-2-(4-fluorophenyl)-N-methyl-6-(N-methylmethyl sulfonamido)benzofuran-3-carboxamide (210 mg, yield: 86%).

### Step 3 Synthesis of compound 111

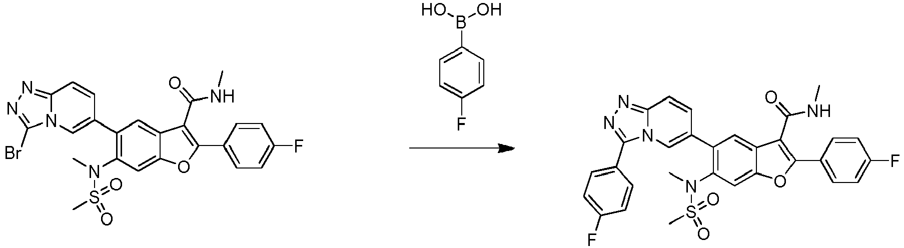

To a degassed solution of 5-(3-bromo-[1,2,4]triazolo[4,3-a]pyridin-6-yl)-2-(4-fluorophenyl)-N-methyl-6-(N-methylmethyl sulfonamido)benzofuran-3-carboxamide (80 mg, 0.14 mmol) and (4-fluorophenyl)boronic acid (29 mg, 0.21 mmol) in 1,4- dioxane (1.5 mL) and water (100 µl) was added K₃PO₄ (89mg, 0.42 mmol) and 1,1'-Bis(di-*tert*-butylphosphino) ferrocene palladium dichloride (14 mg, 0.02 mmol) under N₂ protection. The resulting mixture was heated to 85 °C and stirred at this temperature for 6 hours. The reaction was cooled, filtered through a pad of the celite and washed with ethyl acetate. The combined filtrate was evaporated *in vacuo.* The resulting residue was purified using preparartive TLC (eluted with 3% MeOH / DCM) to provide 2-(4-fluorophenyl)-5-(3-(4-fluorophenyl)-[1,2,4]triazolo[4,3-a]pyridin-6-yl)-N-methyl-6-(N-methylmethylsulfonamido) benzofuran-3-carboxamide (40 mg, yield: 49%).

### Example 25 (illustrative)

### Preparation of Compound 112

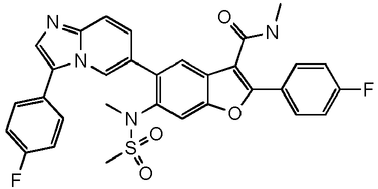

### Step 1 - Synthesis of 2-(4-fluorophenyl)-5-(imidazo[1,2-a]pyridin-6-yl)-N-methyl-6-(N-methylmethylsulfonamido)benzofuran-3-carboxamide

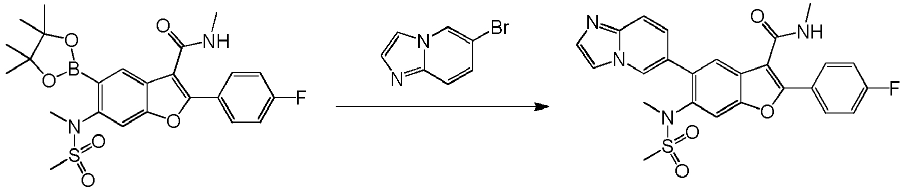

To a degassed solution 6-bromoimidazo[1,2-a]pyridine (118 mg, 0.60 mmol) and 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (300 mg, 0..60 mmol) in 1,4- dioxane (8 mL) and water (200 µl) was added K₃PO₄ (380 mg, 1.79 mmol) and 1,1'-1,1'-Bis(di-*tert*-butylphosphino) ferrocene palladium dichloride (39 mg, 0.06 mmol) under N₂ protection. The resulting mixture was heated to 80 °C and stirred at this temperature for 2 hours, then heated to 100 °C and stirred for 2 hours. The reaction was cooled, filtered through a pad of the celite and washed with ethyl acetate. The combined filtrate was evaporated *in vacuo.* The resulting residue was purified using column chromatography (eluted with 0-3% MeOH / DCM) to provide 2-(4-fluorophenyl) -5-(imidazo[1,2-a]pyridin-6-yl)-N-methyl-6-(N-methylmethylsulfonamido)benzofuran-3-carboxamide (220 mg, yield: 75%).

### Step 2 Synthesis of 5-(3-bromoimidazo[1,2-a]pyridin-6-yl)-2-(4-fluorophenyl)-N-methyl-6-(N-methylmethylsulfonamido)benzofuran-3-carboxamide

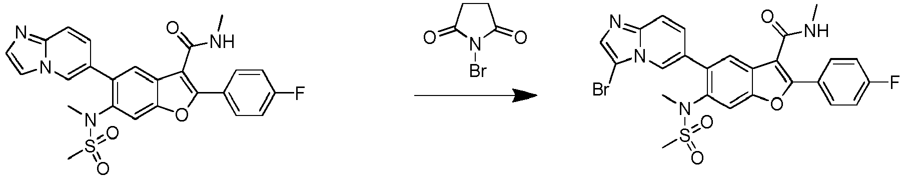

To a solution of 2-(4-fluorophenyl) -5-(imidazo[1,2-a]pyridin-6-yl)-N-methyl-6-(N-methylmethylsulfonamido)benzofuran-3-carboxamide (200 mg, 0.41 mmol) in DMF (5 ml) was added NBS (94 mg, 0.53 mmol). The resulting mixture was stirred at RT for 2 hours, then treated with water and extracted with EtOAc. The organic layer was washed with brine, dried (Na₂SO₄), filtered and concentrated *in vacuo.* The resulting residue was purified using column chromatography (eluted with 0-3% MeOH / DCM) to provide 5-(3-bromoimidazo[1,2-a]pyridin-6-yl)-2-(4-fluorophenyl)-N-methyl-6-(N-methylmethylsulfonamido)benzofuran-3-carboxamide (198 mg, yield: 85%).

### Step 3 Synthesis of compound 112

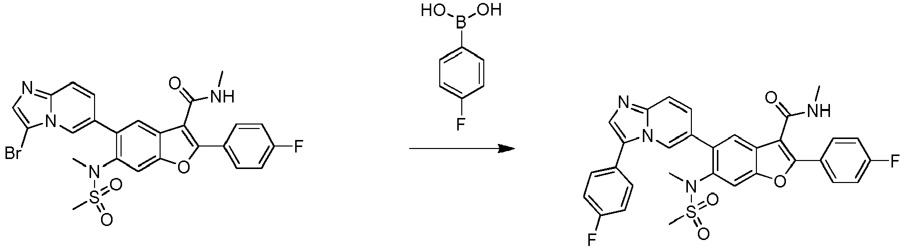

To a degassed solution of 5-(3-bromoimidazo[1,2-a]pyridin-6-yl)-2-(4-fluorophenyl)-N-methyl-6-(N-methylmethylsulfonamido)benzofuran-3-carboxamide (50 mg, 0.09 mmol) and (4-fluorophenyl)boronic acid (24 mg, 0.18 mmol) in 1,4- dioxane (1.5 mL) and water (100 µl) was added K₃PO₄ (37 mg, 0.18 mmol) and 1,1'-Bis(di-*tert*-butylphosphino) ferrocene palladium dichloride (5.7 mg, 0.009 mmol) under N₂ protection. The resulting mixture was heated to 85 °C and stirred at this temperature for 6 hours. The reaction was cooled, filtered through a pad of the celite and washed with ethyl acetate. The combined filtrate was evaporated *in vacuo.* The resulting residue was purified using preparartive TLC (eluted with 3% MeOH / DCM) to provide 2-(4-fluorophenyl)-5-(3-(4-fluorophenyl)imidazo[1,2-a]pyridin-6-yl)-N-methyl-6-(N-methylmethylsulfonamido)benzofuran-3-carboxamide (20 mg, yield: 39%).

### Example 26 (illustrative)

### Preparation of Compound 113

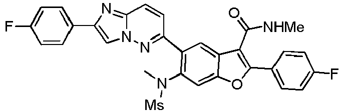

### Step 1

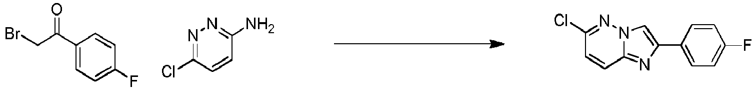

A mixture of 2-bromo-1-(4-fluorophenyl)ethanone (1 g, 4.61 mmol) and 6-chloropyridazin-3-amine (0.597 g, 4.61 mmol) in Ethanol (20 mL) was heated to reflux for 1.5 h. The reaction was cooled down. Filtered and give 6-chloro-2-(4-fluorophenyl)imidazo[1,2-b]pyridazine (400 mg, 1.615 mmol, 35.1 % yield).

### Step 2

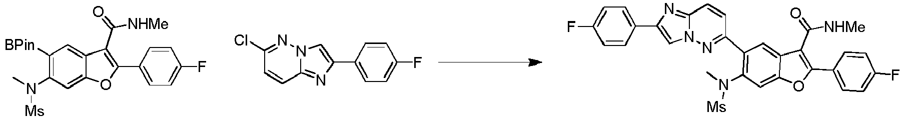

A mixture of 6-chloro-2-(4-fluorophenyl)imidazo[1,2-b]pyridazine (79 mg, 0.32 mmol), 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (100 mg, 0.26 mmol), K₃PO₄·3H₂O (113 mg, 0.53 mmol), 1,1'-bis(di-t-butylphosphino)-ferrocene palladium dichloride (12mg, 0.027 mmol) was allowed to stir in dioxane / H₂O (5 mL, 4 / 1) at 100 °C overnight. The reaction mixture was cooled to 25 °C, diluted with water and extracted with ethyl acetate. The organic extract was washed with brine, dried over Na₂SO₄, filtered and concentrated *in vacuo.* The resulting residue was purified using prep-HPLC to provide Compound **113** (45 mg, yield: 29%).

### Example 27 (illustrative)

### Preparation of Compound 114

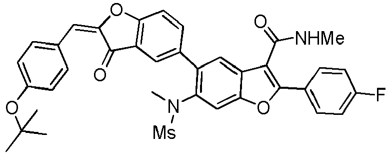

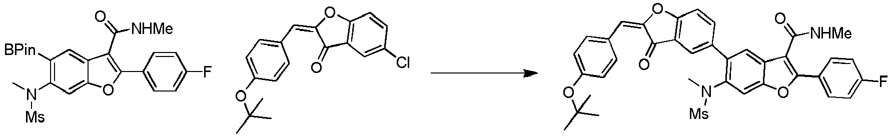

A mixture of (E)-2-(4-(tert-butoxy)benzylidene)-5-chlorobenzofuran-3(2H)-one (131 mg, 0.4 mmol), 2-(4-fluorophenyl)-N-methyl-6-(N-methylmethylsulfonamido)-5-(4,4,5,5-tetramethyl-1,3,2-dioxaborolan-2-yl)benzofuran-3-carboxamide (100 mg, 0.26 mmol), K₃PO₄·3H₂O (113 mg, 0.53 mmol), 1,1'-bis(di-t-butylphosphino)-ferrocene palladium dichloride (12mg, 0.027 mmol) was allowed to stir in dioxane / H₂O (5 mL, 4 / 1) at 100 °C overnight. The reaction mixture was cooled to 25 °C, diluted with water and extracted with ethyl acetate. The organic extract was washed with brine, dried over Na₂SO₄, filtered and concentrated *in vacuo.* The resulting residue was purified using prep-HPLC to provide Compound **114** (35 mg, yield: 20%).

### Example 28

### Measuring Compound Inhibitory Potency

Measurement of inhibition by compounds was performed using the HCV replicon system. Several different replicons encoding different HCV genotypes or mutations were used. In addition, potency measurements were made using different formats of the replicon assay, including different ways of measurements and different plating formats. *See* Jan M. Vrolijk et al., A replicons-based bioassay for the measurement of interferons in patients with chronic hepatitis C, 110 J. VIROLOGICAL METHODS 201 (2003); Steven S. Carroll et al., Inhibition of Hepatitis C Virus RNA Replication by 2'-Modified Nucleoside Analogs, 278(14) J. BIOLOGICAL CHEMISTRY 11979 (2003). However, the underlying principles are common to all of these determinations, and are outlined below.

Stable neomycin phosphotransferase encoding replicons-harboring cell lines were used, so all cell lines were maintained under G418 selection prior to the assay. Potency was deteremined using a cell ELISA assay with an antibody to the replicons encoded NS3/4a protease. *See* Caterina Trozzi et al., In Vitro Selection and Characterization of Hepatitis C Virus Serine Protease Variants Resistant to an Active-Site Peptide Inhibitor, 77(6) J. Virol. 3669 (2003). To initiate an assay, replicon cells were plated in the presence of a dilution series of test compound in the absence of G418. Typically, the assays were performed in a 96-well plate formate for manual operation, or a 384-well plate format for automated assay. Replicon cells and compound were incubated for 96 hours. At the end of the assay, cells were washed free of media and compound, and the cells were then lysed. RNA was quantified indirectly through detection of replicon-encoded NS3/4A protein levels, through an ELISA-based assay with an antibody specific for NS3/4A. IC₅₀ determinations were calculated as a percentage of a DMSO control by fitting the data to a four-parameter fit function and the data obtained is provided in the table below.

Data for selected compounds of the present invention was obtained for genotypes 1a and 1b using this method and is provided in the table below:

| **Compound** | **1a IC₅₀ (nM)** | **1b IC₅₀ (nM)** | **Compound** | **1a IC₅₀ (nM)** | **1b IC₅₀ (nM)** |
|---|---|---|---|---|---|
| **1** | 16.5 | 4.6 | **58** | 7.2 | 2.6 |
| **2** | 13.2 | 2.5 | **59** | 15.3 | 5.2 |
| **3** | 68.0 | 5.8 | **60** | 45.4 | 4.4 |
| **4** | 26.5 | 2.5 | **61** | 34.2 | 10.1 |
| **5** | 2.1 | 0.8 | **62** | 182.8 | 22.9 |
| **6** | 10.6 | 1.2 | **63** | 23.5 | 7.2 |
| **7** | 2.6 | 2.2 | **64** | 408.8 | 76.1 |
| **8** | 27.7 | 3.9 | **65** | 1525 | 114.9 |
| **9** | 75.8 | 7.1 | **66** | 2270 | 225.4 |
| **10** | 4.0 | 3.6 | **67** | 131.8 | 49.8 |
| **11** | 38.9 | 3.5 | **68** | 54.2 | 18.6 |
| **12** | 11.7 | 2.6 | **69** | 146.8 | 17.4 |
| **13** | 18.2 | 3.8 | **70** | 17.8 | 11.3 |
| **14** | 2.7 | 1.2 | **71** | 21.5 | 4.5 |
| **15** | 5.5 | 1.2 | **72** | 4.9 | 4.5 |
| **16** | 37.2 | 4.6 | **73** | 3.9 | 3.8 |
| **17** | 37.6 | 12.8 | **74** | 20.6 | 5.4 |
| **18** | 16.6 | 2.1 | **75** | 8.0 | 7.3 |
| **19** | 12.8 | 2.2 | **76** | 6.8 | 5.8 |
| **20** | 6.1 | 2.4 | **77** | 235.3 | 32.6 |
| **21** | 25.3 | 2.9 | **78** | 19.0 | 5.3 |
| **22** | 14.4 | 3.3 | **79** | 31.4 | 3.1 |
| **23** | 36.7 | 8.2 | **80** | 9.0 | 6.6 |
| **24** | 760.5 | 82.6 | **81** | 30.3 | 5.2 |
| **25** | 585.2 | 51.1 | **82** | 23.0 | 4.9 |
| **26** | 64.7 | 12.0 | **83** | 23.6 | 14.7 |
| **27** | 755 | 75.7 | **84** | 53.7 | 8.6 |
| **28** | 4.2 | 3.3 | **85** | 459.8 | 235.4 |
| **29** | 5.0 | 3.3 | **86** | 714.5 | 303.7 |
| **30** | 6.3 | 2.8 | **87** | 51.5 | 10.8 |
| **31** | 3.4 | 3.1 | **88** | 50.1 | 7.0 |
| **32** | 5.3 | 3.1 | **89** | 7.0 | 2.7 |
| **33** | 13.7 | 8.8 | **90** | 2182 | 120.3 |
| **34** | 24.8 | 4.3 | **91** | 48.2 | 8.1 |
| **35** | 35.2 | 5.9 | **92** | 12.9 | 5.1 |
| **36** | 36.2 | 6.5 | **93** | 47.9 | 7.9 |
| **37** | 6.8 | 4.3 | **94** | 75.6 | 12.3 |
| **38** | 629 | 155 | **95** | 85.9 | 44.0 |
| **39** | 1825 | 82.4 | **96** | 34.5 | 16.5 |
| **40** | 338.5 | 20.2 | **97** | 2.7 | 1.3 |
| **41** | 118.7 | 6.7 | **98** | 3.7 | 4.1 |
| **42** | 1518 | 299.2 | **99** | 3.1 | 5.2 |
| **43** | 158.1 | 17.3 | **100** | 297.2 | 73.0 |
| **44** | 56.5 | 7.7 | **101** | 79.1 | 17.0 |
| **45** | 94.8 | 14.4 | **102** | 14.6 | 5.1 |
| **46** | 74.5 | 10.7 | **103** | 1097 | 100.7 |
| **47** | 2.1 | 1.5 | **104** | 179.1 | 18.9 |
| **48** | 2.6 | 3.3 | **105** | 5.2 | 2.8 |
| **49** | 8.3 | 3.3 | **106** | 6.0 | 6.9 |
| **50** | 3.2 | 4.1 | **107** | 8.3 | 6.1 |
| **51** | 34.6 | 7.8 | **108** | 27.6 | 12.3 |
| **52** | 42.9 | 7.1 | **109** | 110.0 | 21.1 |
| **53** | 82.0 | 12.5 | **110** | 111.4 | 13.8 |
| **54** | 109.3 | 14.1 | **111** | 4.8 | 1.9 |
| **55** | 152.1 | 23.8 | **112** | 12.4 | 7.1 |
| **56** | 223.8 | 30.8 | **113** | 186.0 | 14.9 |
| **57** | 13.9 | 3.0 | **114** | 54.4 | 37.0 |

### Uses of the Substituted Benzofuran Compounds

The Substituted Benzofuran Compounds are useful in human and veterinary medicine for treating or preventing a viral infection in a patient. In one embodiment, the Substituted Benzofuran Compounds can be inhibitors of viral replication. In another embodiment, the Substituted Benzofuran Compounds can be inhibitors of HCV replication. Accordingly, the Substituted Benzofuran Compounds are useful for treating viral infections, such as HCV. In accordance with the invention, the Substituted Benzofuran Compounds can be administered to a patient in need of treatment or prevention of a viral infection.

Accordingly, in one embodiment, the invention provides at least one Substituted Benzofuran Compound or a pharmaceutically acceptable salt thereof for use treating a viral infection in a patient comprising administering to the patient an effective amount of at least one Substituted Benzofuran Compound or a pharmaceutically acceptable salt thereof.

### Treatment or Prevention of a Flaviviridae Virus

The Substituted Benzofuran Compounds may be useful for treating or preventing a viral infection caused by the Flaviviridae family of viruses.

Examples of Flaviviridae infections include but are not limited to, Japanese encephalitis, Kyasanur Forest disease, Murray Valley encephalitis, St. Louis encephalitis, Tick-borne encephalitis, West Nile encephalitis, yellow fever and Hepatitis C Virus (HCV) infection.

In one embodiment, the Flaviviridae infection being treated is hepatitis C virus infection.

### Treatment or Prevention of HCV Infection

The Substituted Benzofuran Compounds are useful in the inhibition of HCV (e.g., HCV NS5B), the treatment of HCV infection and/or reduction of the likelihood or severity of symptoms of HCV infection and the inhibition of HCV viral replication and/or HCV viral production in a cell-based system. For example, the Substituted Benzofuran Compounds are useful in treating infection by HCV after suspected past exposure to HCV by such means as blood transfusion, exchange of body fluids, bites, accidental needle stick, or exposure to patient blood during surgery or other medical procedures.

In one embodiment, the hepatitis C infection is acute hepatitis C. In another embodiment, the hepatitis C infection is chronic hepatitis C.

Accordingly, in one embodiment, the invention provides at least one Substituted Benzofuran Compound or a pharmaceutically acceptable salt thereof for use in treating HCV infection in a patient. In a specific embodiment, the amount administered is effective to treat or prevent infection by HCV in the patient. In another specific embodiment, the amount administered is effective to inhibit HCV viral replication and/or viral production in the patient.

The Substituted Benzofuran Compounds are also useful in the preparation and execution of screening assays for antiviral compounds. For example the Substituted Benzofuran Compounds are useful for identifying resistant HCV replicon cell lines harboring mutations within NS5B, which are excellent screening tools for more powerful antiviral compounds. Furthermore, the Substituted Benzofuran Compounds are useful in establishing or determining the binding site of other antivirals to the HCV replicase.

The compositions and combinations of the present invention can be useful for treating a patient suffering from infection related to any HCV genotype. HCV types and subtypes may differ in their antigenicity, level of viremia, severity of disease produced, and response to interferon therapy as described in Holland et al., Pathology, 30(2):192-195 (1998). The nomenclature set forth in Simmonds et al., J Gen Virol, 74(Pt11):2391-2399 (1993) is widely used and classifies isolates into six major genotypes, 1 through 6, with two or more related subtypes, *e.g*., 1a and 1b. Additional genotypes 7-10 and 11 have been proposed, however the phylogenetic basis on which this classification is based has been questioned, and thus types 7, 8, 9 and 11 isolates have been reassigned as type 6, and type 10 isolates as type 3 (see Lamballerie et al., J Gen Virol, 78(Pt1):45-51 (1997)). The major genotypes have been defined as having sequence similarities of between 55 and 72% (mean 64.5%), and subtypes within types as having 75%-86% similarity (mean 80%) when sequenced in the NS-5 region (see Simmonds et al., J Gen Virol, 75(Pt 5):1053-1061 (1994)).

### Combination Therapy

In another embodiment, the present methods for treating or preventing HCV infection can further comprise the administration of one or more additional therapeutic agents which are not Substituted Benzofuran Compounds.

In one embodiment, the additional therapeutic agent is an antiviral agent.

In another embodiment, the additional therapeutic agent is an immunomodulatory agent, such as an immunosuppressive agent.

Accordingly, in one embodiment, the present invention provides at least one Substituted Benzofuran Compound or a pharmaceutically acceptable salt thereof for use in treating a viral infection in a patient, wherein the at least one Substituted Benzofuran Compound, or a pharmaceutically acceptable salt thereof, is administered along with at least one additional therapeutic agent that is other than a Substituted Benzofuran Compound, wherein the amounts administered are together effective to treat or prevent a viral infection.

When administering a combination therapy of the invention to a patient, therapeutic agents in the combination, or a pharmaceutical composition or compositions comprising therapeutic agents, may be administered in any order such as, for example, sequentially, concurrently, together, simultaneously and the like. The amounts of the various actives in such combination therapy may be different amounts (different dosage amounts) or same amounts (same dosage amounts). Thus, for illustration purposes, a Substituted Benzofuran Compound and an additional therapeutic agent may be present in fixed amounts (dosage amounts) in a single dosage unit (*e.g.,* a capsule, a tablet and the like).

In one embodiment, the at least one Substituted Benzofuran Compound is administered during a time when the additional therapeutic agent(s) exert their prophylactic or therapeutic effect, or *vice versa.*

In another embodiment, the at least one Substituted Benzofuran Compound and the additional therapeutic agent(s) are administered in doses commonly employed when such agents are used as monotherapy for treating a viral infection.

In another embodiment, the at least one Substituted Benzofuran Compound and the additional therapeutic agent(s) are administered in doses lower than the doses commonly employed when such agents are used as monotherapy for treating a viral infection.

In still another embodiment, the at least one Substituted Benzofuran Compound and the additional therapeutic agent(s) act synergistically and are administered in doses lower than the doses commonly employed when such agents are used as monotherapy for treating a viral infection.

In one embodiment, the at least one Substituted Benzofuran Compound and the additional therapeutic agent(s) are present in the same composition. In one embodiment, this composition is suitable for oral administration. In another embodiment, this composition is suitable for intravenous administration. In another embodiment, this composition is suitable for subcutaneous administration. In still another embodiment, this composition is suitable for parenteral administration.

Viral infections and virus-related disorders that can be treated or prevented using the combination therapy methods of the present invention include, those listed above.

In one embodiment, the viral infection is HCV infection.

The at least one Substituted Benzofuran Compound and the additional therapeutic agent(s) can act additively or synergistically. A synergistic combination may allow the use of lower dosages of one or more agents and/or less frequent administration of one or more agents of a combination therapy. A lower dosage or less frequent administration of one or more agents may lower toxicity of therapy without reducing the efficacy of therapy.

In one embodiment, the administration of at least one Substituted Benzofuran Compound and the additional therapeutic agent(s) may inhibit the resistance of a viral infection to these agents.

Examples of additional therapeutic agents useful in the present compositions and methods include an interferon, an immunomodulator, a viral replication inhibitor, an antisense agent, a therapeutic vaccine, a viral polymerase inhibitor, a nucleoside inhibitor, a viral protease inhibitor, a viral helicase inhibitor, a virion production inhibitor, a viral entry inhibitor, a viral assembly inhibitor, an antibody therapy (monoclonal or polyclonal), and any agent useful for treating an RNA-dependent polymerase-related disorder.

In one embodiment, the additional therapeutic agent is a viral protease inhibitor.

In another embodiment, the additional therapeutic agent is a viral replication inhibitor.

In another embodiment, the additional therapeutic agent is an HCV NS3 protease inhibitor.

In still another embodiment, the additional therapeutic agent is an HCV NS5B polymerase inhibitor.

In another embodiment, the additional therapeutic agent is a nucleoside inhibitor.

In another embodiment, the additional therapeutic agent is an interferon.

In yet another embodiment, the additional therapeutic agent is an HCV replicase inhibitor.

In another embodiment, the additional therapeutic agent is an antisense agent.

In another embodiment, the additional therapeutic agent is a therapeutic vaccine.

In a further embodiment, the additional therapeutic agent is a virion production inhibitor.

In another embodiment, the additional therapeutic agent is an antibody therapy.

In another embodiment, the additional therapeutic agent is an HCV NS2 inhibitor.

In still another embodiment, the additional therapeutic agent is an HCV NS4A inhibitor.

In another embodiment, the additional therapeutic agent is an HCV NS4B inhibitor.

In another embodiment, the additional therapeutic agent is an HCV NS5A inhibitor

In yet another embodiment, the additional therapeutic agent is an HCV NS3 helicase inhibitor.

In another embodiment, the additional therapeutic agent is an HCV IRES inhibitor.

In another embodiment, the additional therapeutic agent is an HCV p7 inhibitor.

In a further embodiment, the additional therapeutic agent is an HCV entry inhibitor.

In another embodiment, the additional therapeutic agent is an HCV assembly inhibitor.

In one embodiment, the additional therapeutic agents comprise a viral protease inhibitor and a viral polymerase inhibitor.

In still another embodiment, the additional therapeutic agents comprise a viral protease inhibitor and an immunomodulatory agent.

In yet another embodiment, the additional therapeutic agents comprise a polymerase inhibitor and an immunomodulatory agent.

In another embodiment, the additional therapeutic agents comprise a viral protease inhibitor and a nucleoside.

In another embodiment, the additional therapeutic agents comprise an immunomodulatory agent and a nucleoside.

In one embodiment, the additional therapeutic agents comprise an HCV protease inhibitor and an HCV polymerase inhibitor.

In another embodiment, the additional therapeutic agents comprise a nucleoside and an HCV NS5A inhibitor.

In another embodiment, the additional therapeutic agents comprise a viral protease inhibitor, an immunomodulatory agent and a nucleoside.

In a further embodiment, the additional therapeutic agents comprise a viral protease inhibitor, a viral polymerase inhibitor and an immunomodulatory agent.

In another embodiment, the additional therapeutic agent is ribavirin.

HCV polymerase inhibitors useful in the present compositions and treatments include, VP-19744 (Wyeth/ViroPharma), PSI-7851 (Pharmasset), RG7128 (Roche/Pharmasset), PSI-7977 (Pharmasset), PSI-938 (Pharmasset), PSI-879 (Pharmasset), PSI-661 (Pharmasset), PF-868554/filibuvir (Pfizer), VCH-759/VX-759 (ViroChem Pharma/Vertex), HCV-371 (Wyeth/VirroPharma), HCV-796 (Wyeth/ViroPharma), IDX-184 (Idenix), IDX-375 (Idenix), NM-283 (Idenix/Novartis), GL-60667 (Genelabs), JTK-109 (Japan Tobacco), PSI-6130 (Pharmasset), R1479 (Roche), R-1626 (Roche), R-7128 (Roche), MK-0608 (Isis/Merck), INX-8014 (Inhibitex), INX-8018 (Inhibitex), INX-189 (Inhibitex), GS 9190 (Gilead), A-848837 (Abbott), ABT-333 (Abbott), ABT-072 (Abbott), A-837093 (Abbott), BI-207127 (Boehringer-Ingelheim), BILB-1941 (Boehringer-Ingelheim), MK-3281 (Merck), VCH-222/VX-222 (ViroChem/Vertex), VCH-916 (ViroChem), VCH-716(ViroChem), GSK-71185 (Glaxo SmithKline), ANA598 (Anadys), GSK-625433 (Glaxo SmithKline), XTL-2125 (XTL Biopharmaceuticals), and those disclosed in Ni et al., Current Opinion in Drug Discovery and Development, 7(4):446 (2004); Tan et al., Nature Reviews, 1:867 (2002); and Beaulieu et al., Current Opinion in Investigational Drugs, 5:838 (2004).

Additional HCV polymerase inhibitors useful in the present compositions and methods include, nucleoside compounds such as those disclosed in

Other HCV polymerase inhibitors useful in the present compositions and treatments include, those disclosed in International Publication Nos. WO 08/082484, WO 08/082488, WO 08/083351, WO 08/136815, WO 09/032116, WO 09/032123, WO 09/032124 and WO 09/032125.

Interferons useful in the present compositions and methods include, interferon alfa-2a, interferon alfa-2b, interferon alfacon-1 and PEG-interferon alpha conjugates. "PEG-interferon alpha conjugates" are interferon alpha molecules covalently attached to a PEG molecule. Illustrative PEG-interferon alpha conjugates include interferon alpha-2a (Roferon™, Hoffman La-Roche, Nutley, New Jersey) in the form of pegylated interferon alpha-2a (*e.g.,* as sold under the trade name Pegasys™), interferon alpha-2b (Intron™, from Schering-Plough Corporation) in the form of pegylated interferon alpha-2b (e.g., as sold under the trade name PEG-Intron™ from Schering-Plough Corporation), interferon alpha-2b-XL (*e.g*., as sold under the trade name PEG-Intron™), interferon alpha-2c (Berofor Alpha™, Boehringer Ingelheim, Ingelheim, Germany), PEG-interferon lambda (Bristol-Myers Squibb and ZymoGenetics), interferon alfa-2b alpha fusion polypeptides, interferon fused with the human blood protein albumin (Albuferon™, Human Genome Sciences), Omega Interferon (Intarcia), Locteron controlled release interferon (Biolex/OctoPlus), Biomed-510 (omega interferon), Peg-IL-29 (ZymoGenetics), Locteron CR (Octoplus), R-7025 (Roche), IFN-α-2b-XL (Flamel Technologies), belerofon (Nautilus) and consensus interferon as defined by determination of a consensus sequence of naturally occurring interferon alphas (Infergen™, Amgen, Thousand Oaks, California).

Examples of viral protease inhbitors useful in the present compositions and methods include, an HCV protease inhibitor.

HCV protease inhibitors useful in the present compositions and methods include, those disclosed in U.S. Patent Nos. 7,494,988, 7,485,625, 7,449,447, 7,442,695, 7,425,576, 7,342,041, 7,253,160, 7,244,721, 7,205,330, 7,192,957, 7,186,747, 7,173,057, 7,169,760, 7,012,066, 6,914,122, 6,911,428, 6,894,072, 6,846,802, 6,838,475, 6,800,434, 6,767,991, 5,017,380, 4,933,443, 4,812,561 and 4,634,697; U.S. Patent Publication Nos. US20020068702, US20020160962, US20050119168, US20050176648, US20050209164, US20050249702 and US20070042968; and International Publication Nos. WO 03/006490, WO 03/087092, WO 04/092161 and WO 08/124148.

Additional HCV protease inhibitors useful in the present compositions and methods include, VX-950 (Telaprevir, Vertex), VX-500 (Vertex), VX-813 (Vertex), VBY-376 (Virobay), BI-201335 (Boehringer Ingelheim), TMC-435 (Medivir/Tibotec), ABT-450 (Abbott/Enanta), TMC-435350 (Medivir), RG7227 (Danoprevir, InterMune/Roche), EA-058 (Abbott/Enanta), EA-063 (Abbott/Enanta), GS-9256 (Gilead), IDX-320 (Idenix), ACH-1625 (Achillion), ACH-2684 (Achillion), GS-9132 (Gilead/Achillion), ACH-1095 (Gilead/Achillon), IDX-136 (Idenix), IDX-316 (Idenix), ITMN-8356 (InterMune), ITMN-8347 (InterMune), ITMN-8096 (InterMune), ITMN-7587 (InterMune), BMS-650032 (Bristol-Myers Squibb), VX-985 (Vertex) and PHX1766 (Phenomix).

Further examples of HCV protease inhbitors useful in the present compositions and methods include, those disclosed in Landro et al., Biochemistry, 36(31):9340-9348 (1997); Ingallinella et al., Biochemistry, 37(25):8906-8914 (1998); Llinàs-Brunet et al., Bioorg Med Chem Lett, 8(13):1713-1718 (1998); Martin et al., Biochemistry, 37(33):11459-11468 (1998); Dimasi et al., J Virol, 71(10):7461-7469 (1997); Martin et al., Protein Eng, 10(5):607-614 (1997); Elzouki et al., JHepat, 27(1):42-48 (1997); BioWorld Today, 9(217):4 (November 10, 1998); U.S. Patent Publication Nos. US2005/0249702 and US 2007/0274951; and International Publication Nos. WO 98/14181, WO 98/17679, WO 98/17679, WO 98/22496 and WO 99/07734 and WO 05/087731.

Further examples of HCV protease inhibitors useful in the present compositions and methods include, the following compounds: 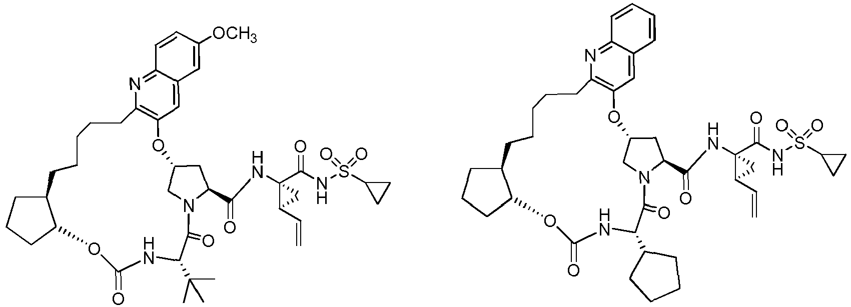 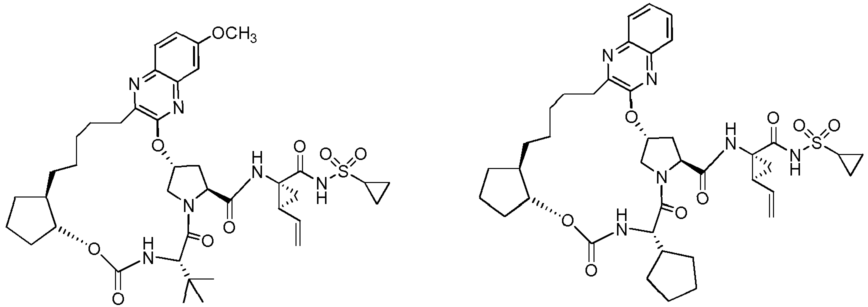 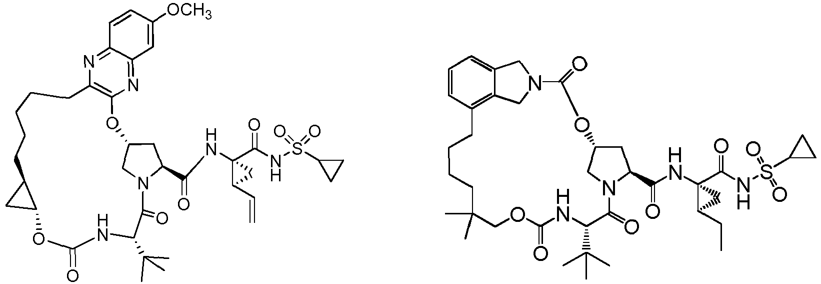 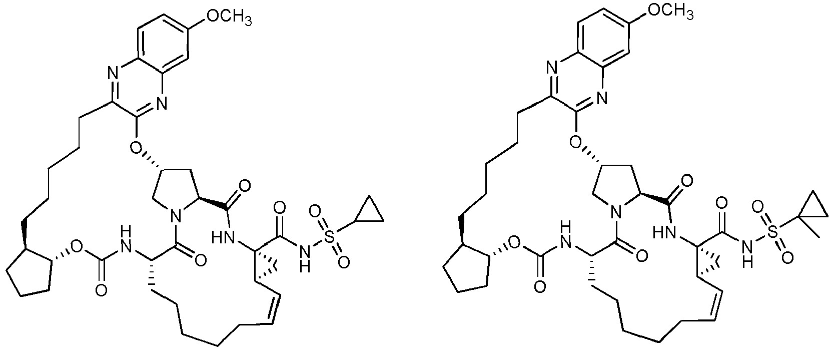 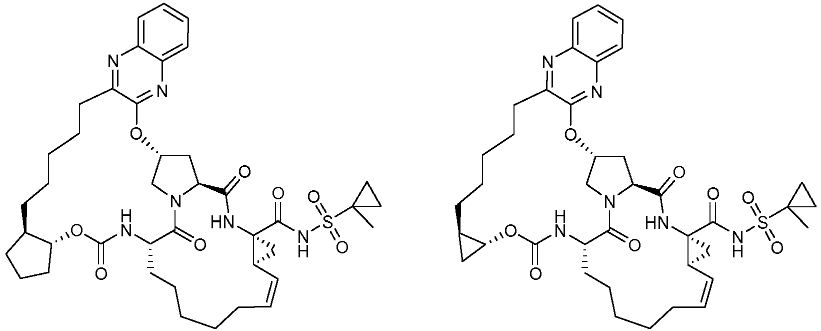 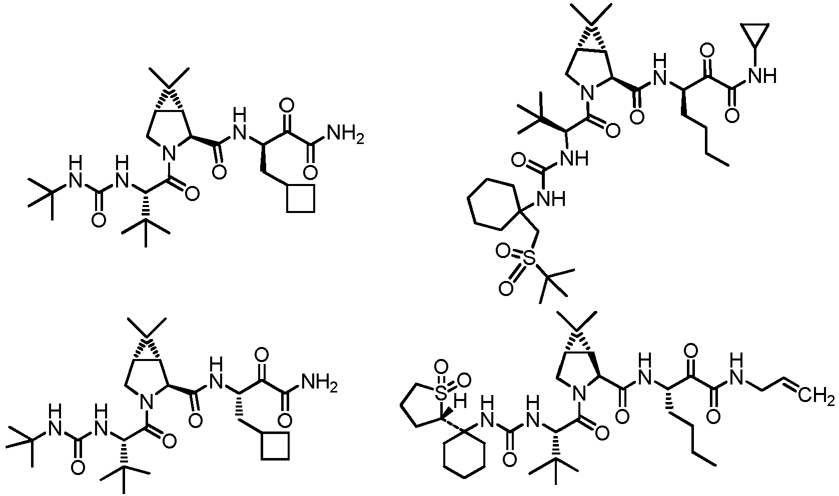 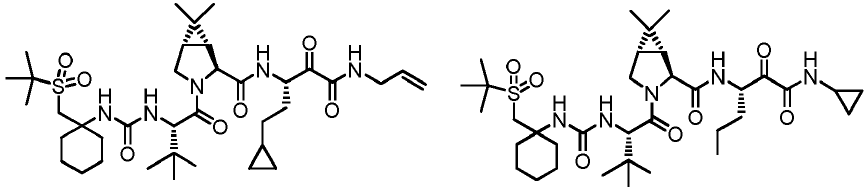 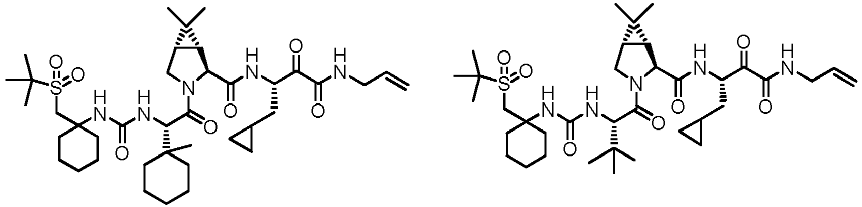 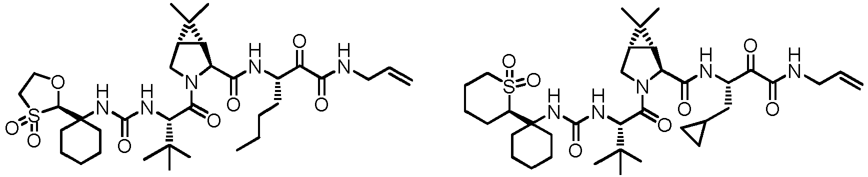 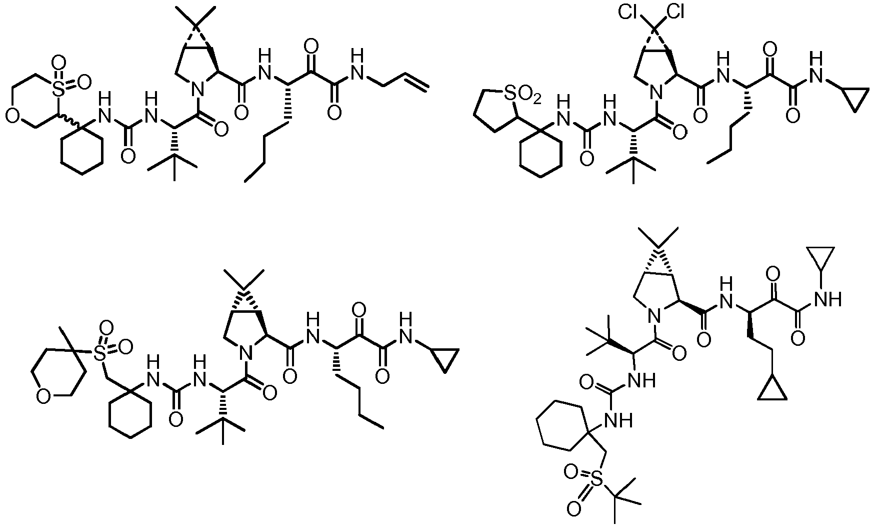 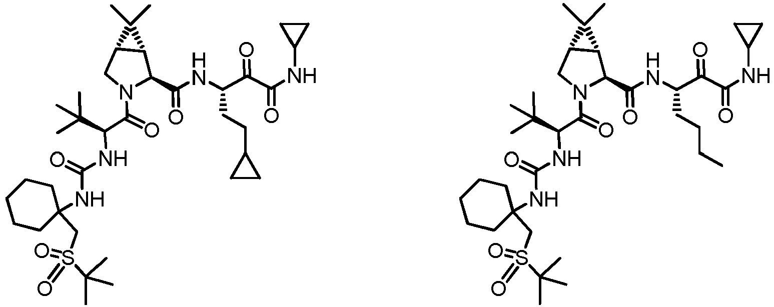 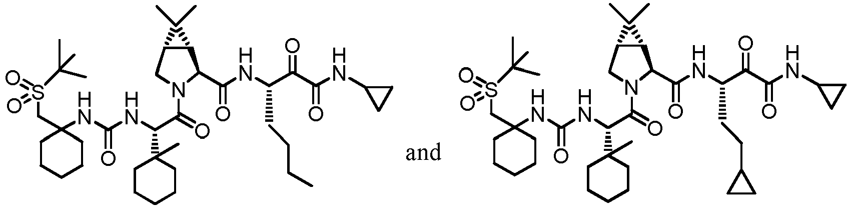 and pharmaceutically acceptable salts thereof.

Viral replication inhibitors useful in the present compositions and methods include, HCV replicase inhibitors, IRES inhibitors, NS4A inhibitors, NS3 helicase inhibitors, NS5A inhibitors, NS5B inhibitors, ribavirin, AZD-2836 (Astra Zeneca), viramidine, A-831 (Arrow Therapeutics), EDP-239 (Enanta), ACH-2928 (Achillion), GS-5885 (Gilead); an antisense agent or a therapeutic vaccine.

Viral entry inhibitors useful as second additional therapeutic agents in the present compositions and treatments include, PRO-206 (Progenics), REP-9C (REPICor), SP-30 (Samaritan Pharmaceuticals) and ITX-5061 (iTherx).

HCV NS4A inhibitors useful in the useful in the present compositions and treatments include, those disclosed in U.S. Patent Nos. 7,476,686 and 7,273,885; U.S. Patent Publication No. US20090022688; and International Publication Nos. WO 2006/019831 and WO 2006/019832. Additional HCV NS4A inhibitors useful as second additional therapeutic agents in the present compositions and methods include, but are not limited to, AZD2836 (Astra Zeneca), ACH-1095 (Achillion) and ACH-806 (Achillion).

HCV NS5A inhibitors useful in the present compositions and treatments include, ACH-2928 (Achillon), AZD-7295 (Astra Zeneca), A-832 (Arrow Therpeutics), PPI-461 (Presidio), PPI-1301 (Presidio), GS-5885 (Gilead) and BMS-790052 (Bristol-Myers Squibb).

HCV replicase inhibitors useful in the present compositions and treatments include, those disclosed in U.S. Patent Publication No. US20090081636.

Therapeutic vaccines useful in the present compositions and treatments include, IC41 (Intercell Novartis), CSL123 (Chiron/CSL), GI 5005 (Globeimmune), TG-4040 (Transgene), GNI-103 (GENimmune), Hepavaxx C (ViRex Medical), ChronVac-C (Inovio/Tripep), PeviPROTM (Pevion Biotect), HCV/MF59 (Chiron/Novartis), MBL-HCV1 (MassBiologics), GI-5005 (GlobeImmune), CT-011 (CureTech/Teva) and Civacir (NABI).

Examples of further additional therapeutic agents useful in the present compositions and treatments include, Ritonavir (Abbott), TT033 (Benitec/Tacere Bio/Pfizer), Sirna-034 (Sirna Therapeutics), GNI-104 (GENimmune), GI-5005 (GlobeImmune), IDX-102 (Idenix), Levovirin™ (ICN Pharmaceuticals, Costa Mesa, California); Humax (Genmab), ITX-2155 (Ithrex/Novartis), PRO 206 (Progenics), HepaCide-I (NanoVirocides), MX3235 (Migenix), SCY-635 (Scynexis); KPE02003002 (Kemin Pharma), Lenocta (VioQuest Pharmaceuticals), IET - Interferon Enhancing Therapy (Transition Therapeutics), Zadaxin (SciClone Pharma), VP 50406™ (Viropharma, Incorporated, Exton, Pennsylvania); Taribavirin (Valeant Pharmaceuticals); Nitazoxanide (Romark); Debio 025 (Debiopharm); GS-9450 (Gilead); PF-4878691 (Pfizer); ANA773 (Anadys); SCV-07 (SciClone Pharmaceuticals); NIM-881 (Novartis); ISIS 14803™ (ISIS Pharmaceuticals, Carlsbad, California); Heptazyme™ (Ribozyme Pharmaceuticals, Boulder, Colorado); Thymosin™ (SciClone Pharmaceuticals, San Mateo, California); Maxamine™ (Maxim Pharmaceuticals, San Diego, California); NKB-122 (JenKen Bioscience Inc., North Carolina); Alinia (Romark Laboratories), INFORM-1 (a combination of R7128 and ITMN-191); and mycophenolate mofetil (Hoffman-LaRoche, Nutley, New Jersey).

The doses and dosage regimen of the other agents used in the combination therapies of the present invention for the treatment or prevention of HCV infection can be determined by the attending clinician, taking into consideration the approved doses and dosage regimen in the package insert; the age, sex and general health of the patient; and the type and severity of the viral infection or related disease or disorder. When administered in combination, the Substituted Benzofuran Compound(s) and the other agent(s) can be administered simultaneously (*i.e.,* in the same composition or in separate compositions one right after the other) or sequentially. This particularly useful when the components of the combination are given on different dosing schedules, *e.g*., one component is administered once daily and another component is administered every six hours, or when the preferred pharmaceutical compositions are different, *e.g*., one is a tablet and one is a capsule. A kit comprising the separate dosage forms is therefore advantageous.

Generally, a total daily dosage of the at least one Substituted Benzofuran Compound(s) alone, or when administered as combination therapy, can range from about 1 to about 2500 mg per day, although variations will necessarily occur depending on the target of therapy, the patient and the route of administration. In one embodiment, the dosage is from about 10 to about 1000 mg/day, administered in a single dose or in 2-4 divided doses. In another embodiment, the dosage is from about 1 to about 500 mg/day, administered in a single dose or in 2-4 divided doses. In still another embodiment, the dosage is from about 1 to about 100 mg/day, administered in a single dose or in 2-4 divided doses. In yet another embodiment, the dosage is from about 1 to about 50 mg/day, administered in a single dose or in 2-4 divided doses. In another embodiment, the dosage is from about 500 to about 1500 mg/day, administered in a single dose or in 2-4 divided doses. In still another embodiment, the dosage is from about 500 to about 1000 mg/day, administered in a single dose or in 2-4 divided doses. In yet another embodiment, the dosage is from about 100 to about 500 mg/day, administered in a single dose or in 2-4 divided doses.

In one embodiment, when the additional therapeutic agent is INTRON-A interferon alpha 2b (commercially available from Schering-Plough Corp.), this agent is administered by subcutaneous injection at 3MIU(12 mcg)/0.5mL/TIW for 24 weeks or 48 weeks for first time treatment.

In another embodiment, when the additional therapeutic agent is PEG-INTRON interferon alpha 2b pegylated (commercially available from Schering-Plough Corp.), this agent is administered by subcutaneous injection at 1.5 mcg/kg/week, within a range of 40 to 150 mcg/week, for at least 24 weeks.

In another embodiment, when the additional therapeutic agent is ROFERON A interferon alpha 2a (commercially available from Hoffmann-La Roche), this agent is administered by subcutaneous or intramuscular injection at 3MIU(11.1 mcg/mL)/TIW for at least 48 to 52 weeks, or alternatively 6MIU/TIW for 12 weeks followed by 3MIU/TIW for 36 weeks.

In still another embodiment, when the additional therapeutic agent is PEGASUS interferon alpha 2a pegylated (commercially available from Hoffmann-La Roche), this agent is administered by subcutaneous injection at 180 mcg/1mL or 180 mcg/0.5mL, once a week for at least 24 weeks.

In yet another embodiment, when the additional therapeutic agent is INFERGEN interferon alphacon-1 (commercially available from Amgen), this agent is administered by subcutaneous injection at 9 mcg/TIW is 24 weeks for first time treatment and up to 15 mcg/TIW for 24 weeks for non-responsive or relapse treatment.

In a further embodiment, when the additional therapeutic agent is Ribavirin (commercially available as REBETOL ribavirin from Schering-Plough or COPEGUS ribavirin from Hoffmann-La Roche), this agent is administered at a daily dosage of from about 600 to about 1400 mg/day for at least 24 weeks.

In one embodiment, one or more compounds of the present invention are administered with one or more additional therapeutic agents selected from: an interferon, an immunomodulator, a viral replication inhibitor, an antisense agent, a therapeutic vaccine, a viral polymerase inhibitor, a nucleoside inhibitor, a viral protease inhibitor, a viral helicase inhibitor, a viral polymerase inhibitor a virion production inhibitor, a viral entry inhibitor, a viral assembly inhibitor, an antibody therapy (monoclonal or polyclonal), and any agent useful for treating an RNA-dependent polymerase-related disorder.

In another embodiment, one or more compounds of the present invention are administered with one or more additional therapeutic agents selected from an HCV protease inhibitor, an HCV polymerase inhibitor, an HCV replication inhibitor, a nucleoside, an interferon, a pegylated interferon and ribavirin. The combination therapies can include any combination of these additional therapeutic agents.

In another embodiment, one or more compounds of the present invention are administered with one additional therapeutic agent selected from an HCV protease inhibitor, an interferon, a pegylated interferon and ribavirin.

In still another embodiment, one or more compounds of the present invention are administered with two additional therapeutic agents selected from an HCV protease inhibitor, an HCV replication inhibitor, a nucleoside, an interferon, a pegylated interferon and ribavirin.

In another embodiment, one or more compounds of the present invention are administered with an HCV protease inhibitor and ribavirin. In another specific embodiment, one or more compounds of the present invention are administered with a pegylated interferon and ribavirin.

In another embodiment, one or more compounds of the present invention are administered with three additional therapeutic agents selected from an HCV protease inhibitor, an HCV replication inhibitor, a nucleoside, an interferon, a pegylated interferon and ribavirin.

In one embodiment, one or more compounds of the present invention are administered with one or more additional therapeutic agents selected from an HCV polymerase inhibitor, a viral protease inhibitor, an interferon, and a viral replication inhibitor. In another embodiment, one or more compounds of the present invention are administered with one or more additional therapeutic agents selected from an HCV polymerase inhibitor, a viral protease inhibitor, an interferon, and a viral replication inhibitor. In another embodiment, one or more compounds of the present invention are administered with one or more additional therapeutic agents selected from an HCV polymerase inhibitor, a viral protease inhibitor, an interferon, and ribavirin.

In one embodiment, one or more compounds of the present invention are administered with one additional therapeutic agent selected from an HCV polymerase inhibitor, a viral protease inhibitor, an interferon, and a viral replication inhibitor. In another embodiment, one or more compounds of the present invention are administered with ribavirin.

In one embodiment, one or more compounds of the present invention are administered with two additional therapeutic agents selected from an HCV polymerase inhibitor, a viral protease inhibitor, an interferon, and a viral replication inhibitor.

In another embodiment, one or more compounds of the present invention are administered with ribavirin, interferon and another therapeutic agent.

In another embodiment, one or more compounds of the present invention are administered with ribavirin, interferon and another therapeutic agent, wherein the additional therapeutic agent is selected from an HCV polymerase inhibitor, a viral protease inhibitor, and a viral replication inhibitor.

In still another embodiment, one or more compounds of the present invention are administered with ribavirin, interferon and a viral protease inhibitor.

In another embodiment, one or more compounds of the present invention are administered with ribavirin, interferon and an HCV protease inhibitor.

In another embodiment, one or more compounds of the present invention are administered with ribavirin, interferon and boceprevir or telaprevir.

In a further embodiment, one or more compounds of the present invention are administered with ribavirin, interferon and an HCV polymerase inhibitor.

In another embodiment, one or more compounds of the present invention are administered with pegylated-interferon alpha and ribavirin.

### Compositions and Administration

Due to their activity, the Substituted Benzofuran Compounds are useful in veterinary and human medicine. As described above, the Substituted Benzofuran Compounds are useful for treating or preventing HCV infection in a patient in need thereof.

When administered to a patient, the Substituted Benzofuran Compounds can be administered as a component of a composition that comprises a pharmaceutically acceptable carrier or vehicle. The present invention provides pharmaceutical compositions comprising an effective amount of at least one Substituted Benzofuran Compound and a pharmaceutically acceptable carrier. In the pharmaceutical compositions and methods of the present invention, the active ingredients will typically be administered in admixture with suitable carrier materials suitably selected with respect to the intended form of administration, *i.e.,* oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active drug component may be combined with any oral non-toxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Solid form preparations include powders, tablets, dispersible granules, capsules, cachets and suppositories. Powders and tablets may be comprised of from about 0.5 to about 95 percent inventive composition. Tablets, powders, cachets and capsules can be used as solid dosage forms suitable for oral administration.

Moreover, when desired or needed, suitable binders, lubricants, disintegrating agents and coloring agents may also be incorporated in the mixture. Suitable binders include starch, gelatin, natural sugars, corn sweeteners, natural and synthetic gums such as acacia, sodium alginate, carboxymethylcellulose, polyethylene glycol and waxes. Among the lubricants there may be mentioned for use in these dosage forms, boric acid, sodium benzoate, sodium acetate, sodium chloride, and the like. Disintegrants include starch, methylcellulose, guar gum, and the like. Sweetening and flavoring agents and preservatives may also be included where appropriate.

Liquid form preparations include solutions, suspensions and emulsions and may include water or water-propylene glycol solutions for parenteral injection.

Liquid form preparations may also include solutions for intranasal administration.

Also included are solid form preparations which are intended to be converted, shortly before use, to liquid form preparations for either oral or parenteral administration. Such liquid forms include solutions, suspensions and emulsions.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Additionally, the compositions of the present invention may be formulated in sustained release form to provide the rate controlled release of any one or more of the components or active ingredients to optimize therapeutic effects, *i.e.,* antiviral activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

In one embodiment, the one or more Substituted Benzofuran Compounds are administered orally.

In another embodiment, the one or more Substituted Benzofuran Compounds are administered intravenously.

In one embodiment, a pharmaceutical preparation comprising at least one Substituted Benzofuran Compound is in unit dosage form. In such form, the preparation is subdivided into unit doses containing effective amounts of the active components.

Compositions can be prepared according to conventional mixing, granulating or coating methods, respectively, and the present compositions can contain, in one embodiment, from about 0.1% to about 99% of the Substituted Benzofuran Compound(s) by weight or volume. In various embodiments, the present compositions can contain, in one embodiment, from about 1% to about 70% or from about 5% to about 60% of the Substituted Benzofuran Compound(s) by weight or volume.

The quantity of Substituted Benzofuran Compound in a unit dose of preparation may be varied or adjusted from about 1 mg to about 2500 mg. In various embodiment, the quantity is from about 10 mg to about 1000 mg, 1 mg to about 500 mg, 1 mg to about 100 mg, and 1 mg to about 100 mg.

For convenience, the total daily dosage may be divided and administered in portions during the day if desired. In one embodiment, the daily dosage is administered in one portion. In another embodiment, the total daily dosage is administered in two divided doses over a 24 hour period. In another embodiment, the total daily dosage is administered in three divided doses over a 24 hour period. In still another embodiment, the total daily dosage is administered in four divided doses over a 24 hour period.

The amount and frequency of administration of the Substituted Benzofuran Compounds will be regulated according to the judgment of the attending clinician considering such factors as age, condition and size of the patient as well as severity of the symptoms being treated. Generally, a total daily dosage of the Substituted Benzofuran Compounds range from about 0.1 to about 2000 mg per day, although variations will necessarily occur depending on the target of therapy, the patient and the route of administration. In one embodiment, the dosage is from about 1 to about 200 mg/day, administered in a single dose or in 2-4 divided doses. In another embodiment, the dosage is from about 10 to about 2000 mg/day, administered in a single dose or in 2-4 divided doses. In another embodiment, the dosage is from about 100 to about 2000 mg/day, administered in a single dose or in 2-4 divided doses. In still another embodiment, the dosage is from about 500 to about 2000 mg/day, administered in a single dose or in 2-4 divided doses.

The compositions of the invention can further comprise one or more additional therapeutic agents, selected from those listed above herein. Accordingly, in one embodiment, the present invention provides compositions comprising: (i) at least one Substituted Benzofuran Compound or a pharmaceutically acceptable salt thereof; (ii) one or more additional therapeutic agents that are not a Substituted Benzofuran Compound; and (iii) a pharmaceutically acceptable carrier, wherein the amounts in the composition are together effective to treat HCV infection.

In one embodiment, the present invention provides compositions comprising a Compound of Formula (I) and a pharmaceutically acceptable carrier.

In another embodiment, the present invention provides compositions comprising a Compound of Formula (I), a pharmaceutically acceptable carrier, and a second therapeutic agent selected from the group consisting of HCV antiviral agents, immunomodulators, and anti-infective agents.

In another embodiment, the present invention provides compositions comprising a Compound of Formula (I), a pharmaceutically acceptable carrier, and wto additional therapeutic agents, each of which are independently selected from the group consisting of HCV antiviral agents, immunomodulators, and anti-infective agents.

### Kits

In one aspect, the present invention provides a kit comprising a therapeutically effective amount of at least one Substituted Benzofuran Compound, or a pharmaceutically acceptable salt of said compound and a pharmaceutically acceptable carrier, vehicle or diluent.

In another aspect the present invention provides a kit comprising an amount of at least one Substituted Benzofuran Compound, or a pharmaceutically acceptable salt of said compound and an amount of at least one additional therapeutic agent listed above, wherein the amounts of the two or more active ingredients result in a desired therapeutic effect. In one embodiment, the one or more Substituted Benzofuran Compounds and the one or more additional therapeutic agents are provided in the same container. In one embodiment, the one or more Substituted Benzofuran Compounds and the one or more additional therapeutic agents are provided in separate containers.

## Claims

1. A compound having the formula; 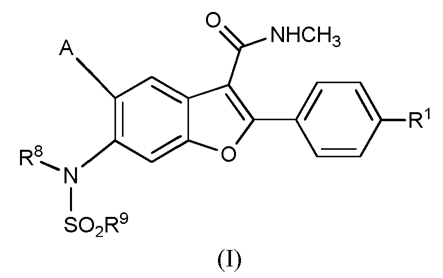 or a pharmaceutically acceptable salt thereof,
wherein:
A has the structure: 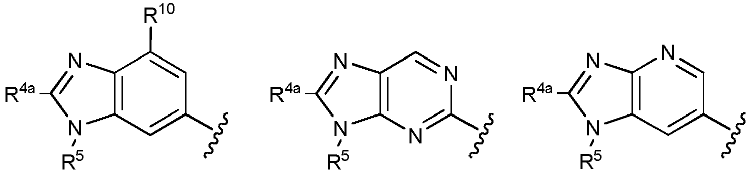 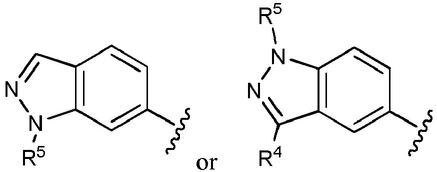
R¹ is halo;
R⁴ is selected from H, pyridyl and phenyl, wherein said phenyl group can be optionally substituted with C₁-C₆ alkyl, C₁-C₆ haloalkyl or halo;
R^{4a} is selected from H, methyl and phenyl, wherein said phenyl group can be optionally substituted with up to 2 groups, which can be the same or different, and are selected from C₁-C₆ alkyl, C₁-C₆ haloalkyl, halo, -CN, -O-(C₁-C₆ alkyl) and -O-(C₁-C₆ haloalkyl);
R⁵ is H; C₁-C₆ hydroxyalkyl; -C(O)O-t-butyl; pyridyl; tetrahydropyranyl; phenyl, which can be optionally substituted with -O-(C₁-C₆ alkyl), halo, -CN, -CF₃, -OCF₃ or -SO₂CH₃; benzyl, which can be optionally substituted with -CF₃; and pyrrolidinyl, which can be optionally substituted on its ring nitrogen atom with -C(O)O-t-butyl;
R⁸ and R⁹ are each C₁-C₆ alkyl and
each occurrence of R¹⁰ is independently H or halo.

2. The compound of claims 1, wherein R¹ is F; R⁸ and R⁹ are each methyl; and each occurrence of R¹⁰ is independently H or F.

3. The compound of claim 1 which is selected from: 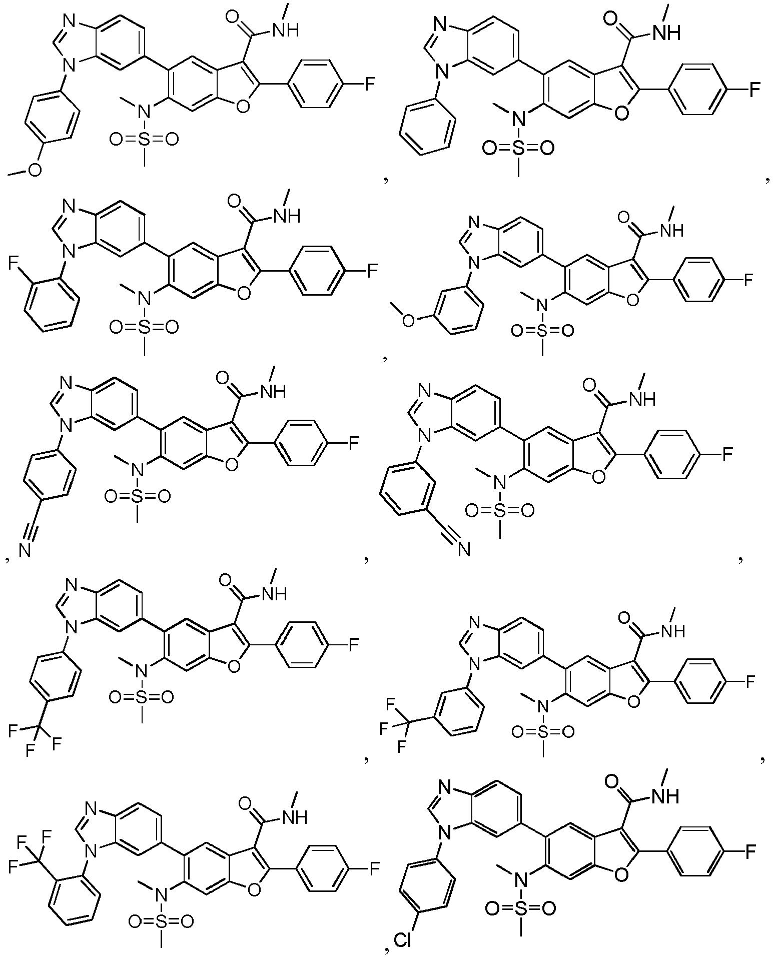 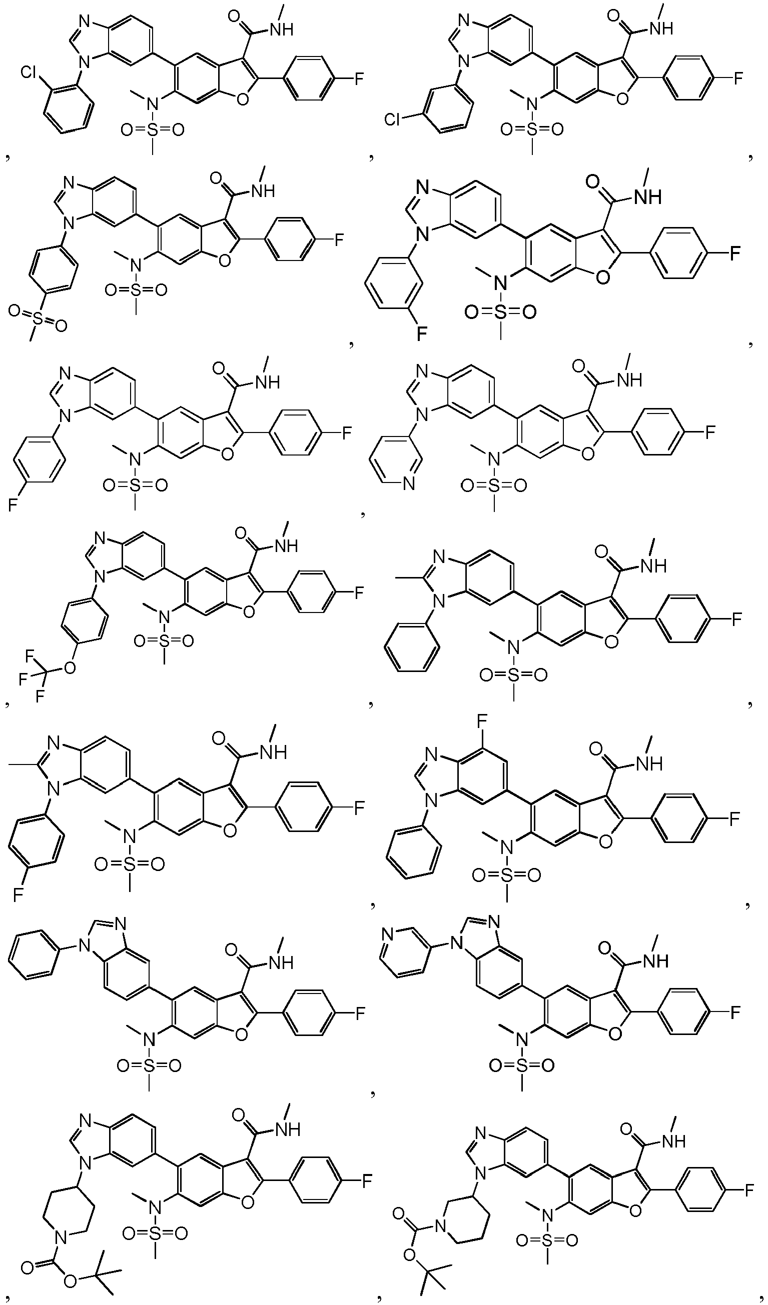 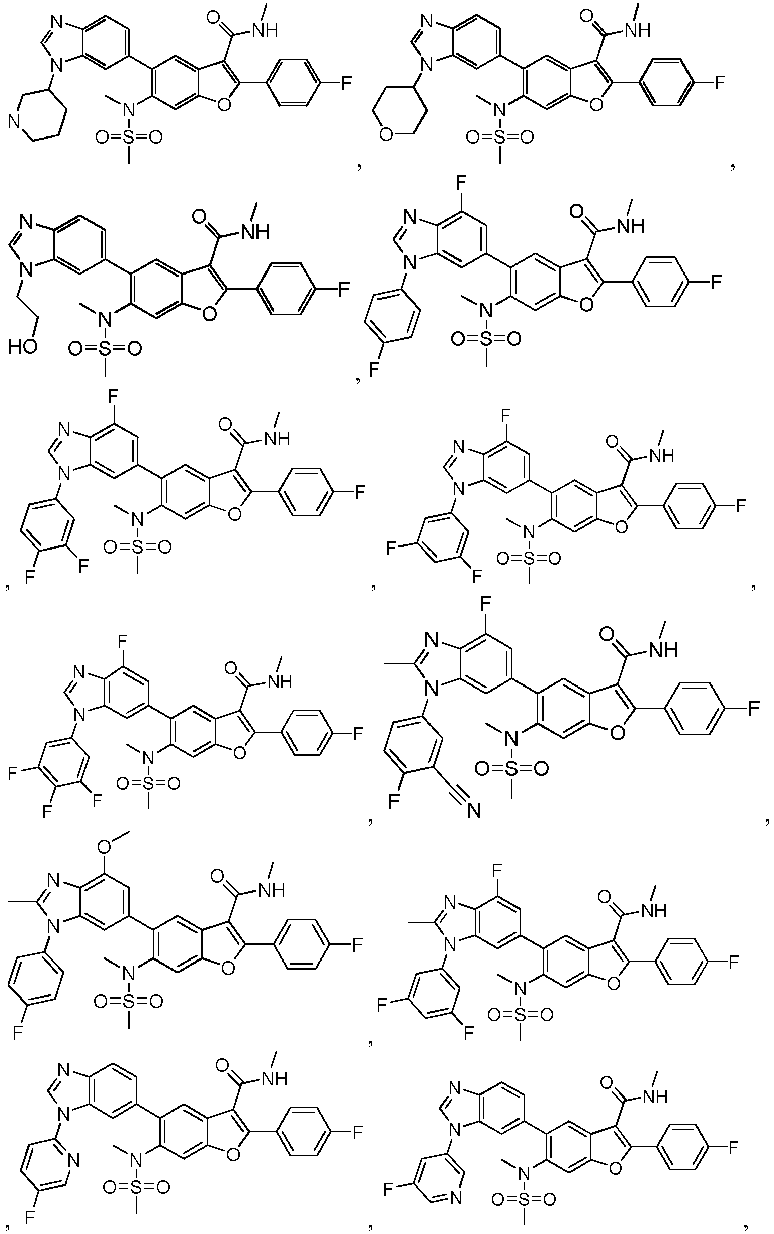 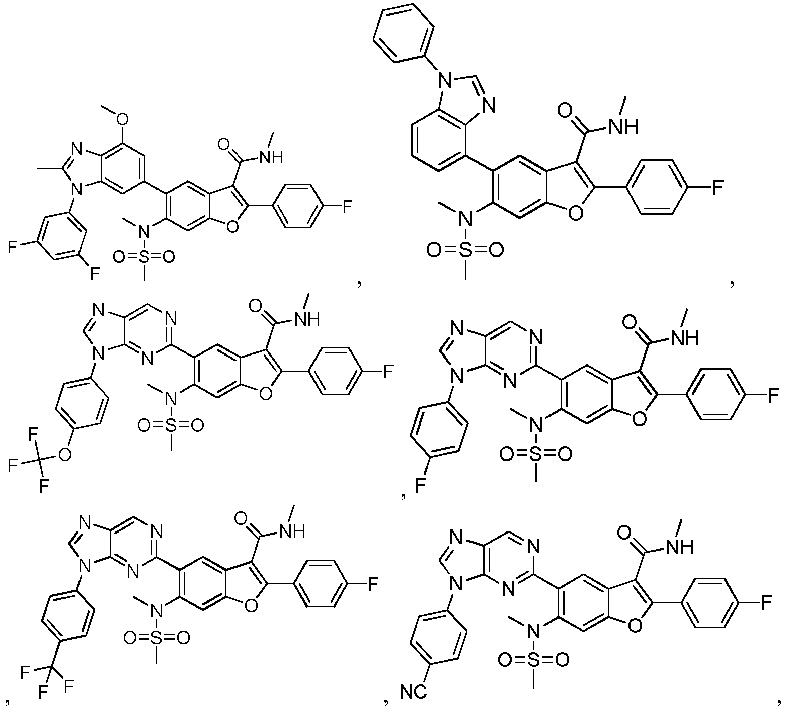 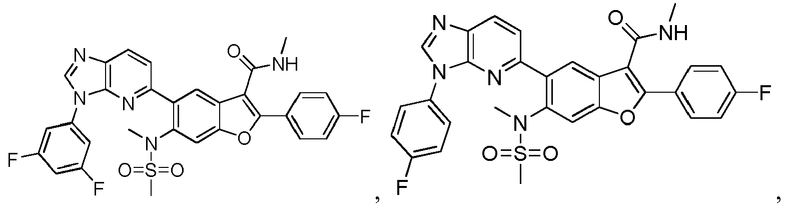 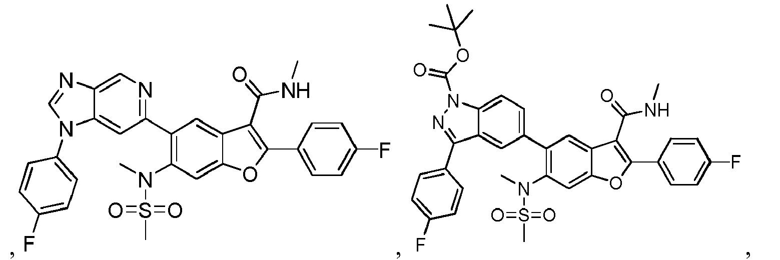 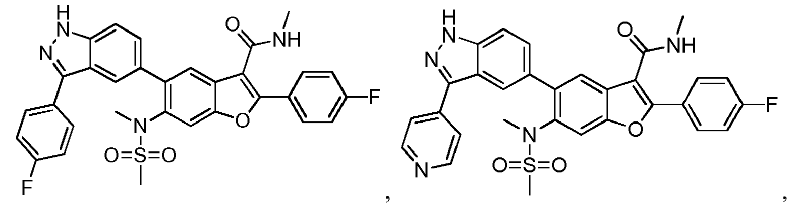 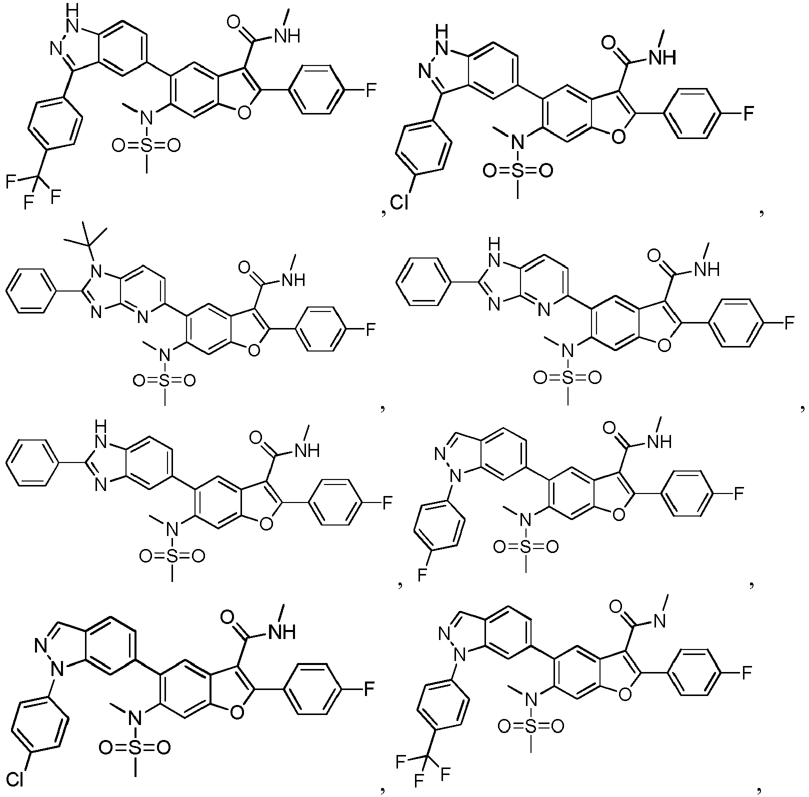 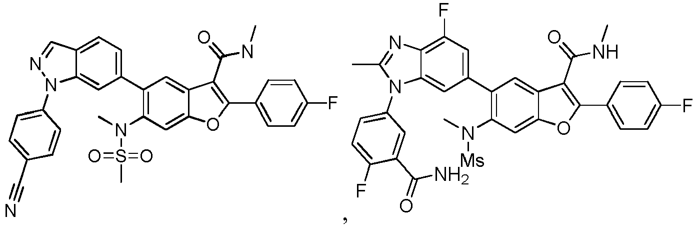 and 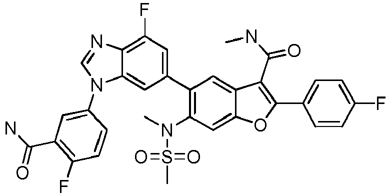 or a pharmaceutically acceptable salt thereof.

4. A pharmaceutical composition comprising a pharmaceutically acceptable carrier and an effective amount of the compound of any one of claims 1-3 or a pharmaceutically acceptable salt thereof.

5. The pharmaceutical composition of claim 4, further comprising a second therapeutic agent selected from the group consisting of HCV antiviral agents, immunomodulators, and anti-infective agents.

6. The pharmaceutical composition of claim 5, wherein the second therapeutic agent is selected from the group consisting of HCV protease inhibitors, HCV NS5A inhibitors and HCV NS5B polymerase inhibitors.

7. The compoundof any one of claims 1-3 for use in inhibiting HCV NS5B activity or for preventing and/or treating infection by HCV in a patient in need thereof.

## Patentansprüche

1. Eine Verbindung der Formel: 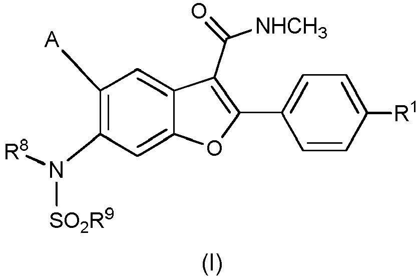 oder ein pharmazeutisch annehmbares Salz davon,
wobei:
A die Struktur: 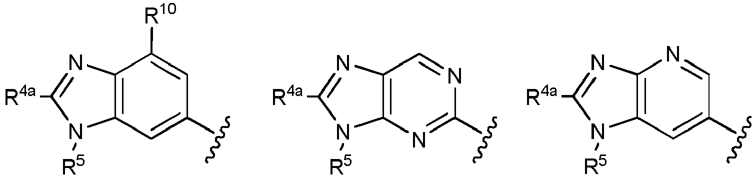 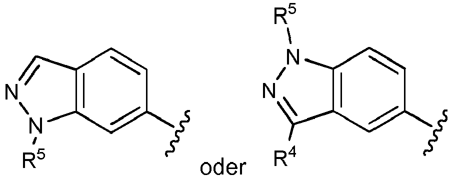 besitzt,
R¹ Halogen ist,
R⁴ ausgewählt ist aus H, Pyridyl und Phenyl, wobei die Phenylgruppe gegebenenfalls substituiert sein kann mit C₁-C₆-Alkyl, C₁-C₆-Halogenalkyl oder Halogen,
R^{4a} ausgewählt ist aus H, Methyl und Phenyl, wobei die Phenylgruppe gegebenenfalls substituiert sein kann mit bis zu 2 Gruppen, die gleich oder verschieden sein können und ausgewählt sind aus C₁-C₆-Alkyl, C₁-C₆-Halogenalkyl, Halogen, -CN, -O-(C₁-C₆-Alkyl) und -O-(C₁-C₆-Halogenalkyl),
R⁵ H; C₁-C₆-Hydroxyalkyl; -C(O)O-t-Butyl; Pyridyl; Tetrahydropyranyl; Phenyl, das gegebenenfalls substituiert sein kann mit -O-(C₁-C₆-Alkyl), Halogen, -CN, -CF₃, -OCF₃ oder -SO₂CH₃; Benzyl, das gegebenenfalls substituiert sein kann mit -CF₃; und Pyrrolidinyl, das an seinem Ring-Stickstoffatom gegebenenfalls substituiert sein kann mit -C(O)O-t-Butyl; ist,
R⁸ und R⁹ jeweils C₁-C₆-Alkyl sind und
R¹⁰ bei jedem Vorkommen unabhängig H oder Halogen ist.

2. Die Verbindung nach Anspruch 1, wobei R¹ F ist, R⁸ und R⁹ jeweils Methyl sind, und R¹⁰ bei jedem Vorkommen unabhängig H oder F ist.

3. Die Verbindung nach Anspruch 1, die ausgewählt ist aus: 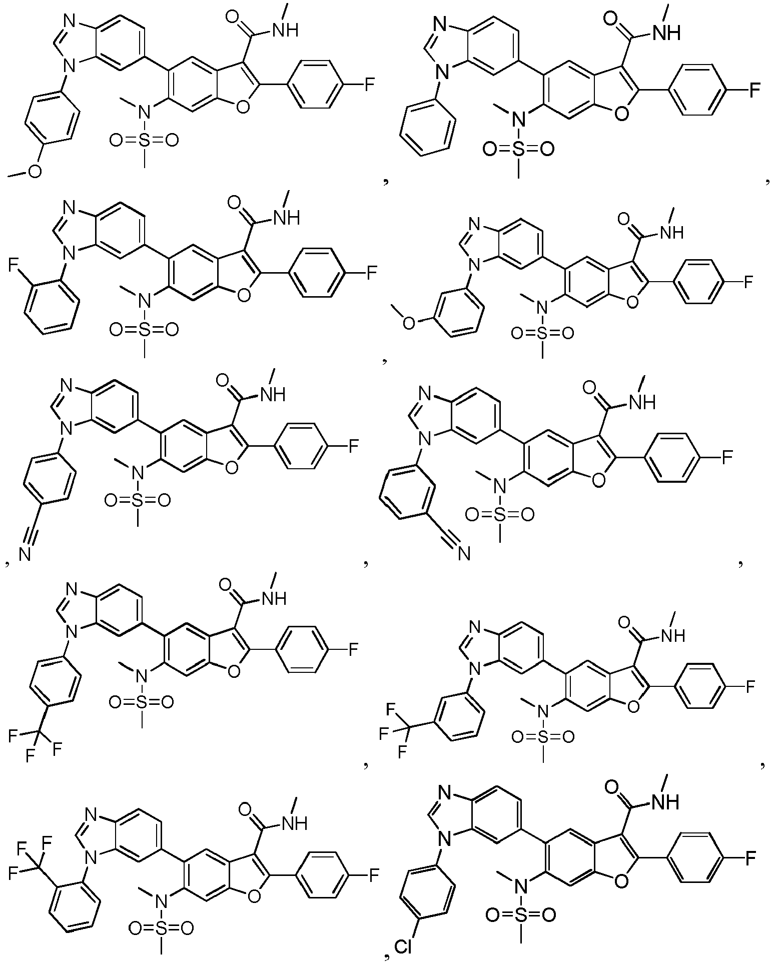 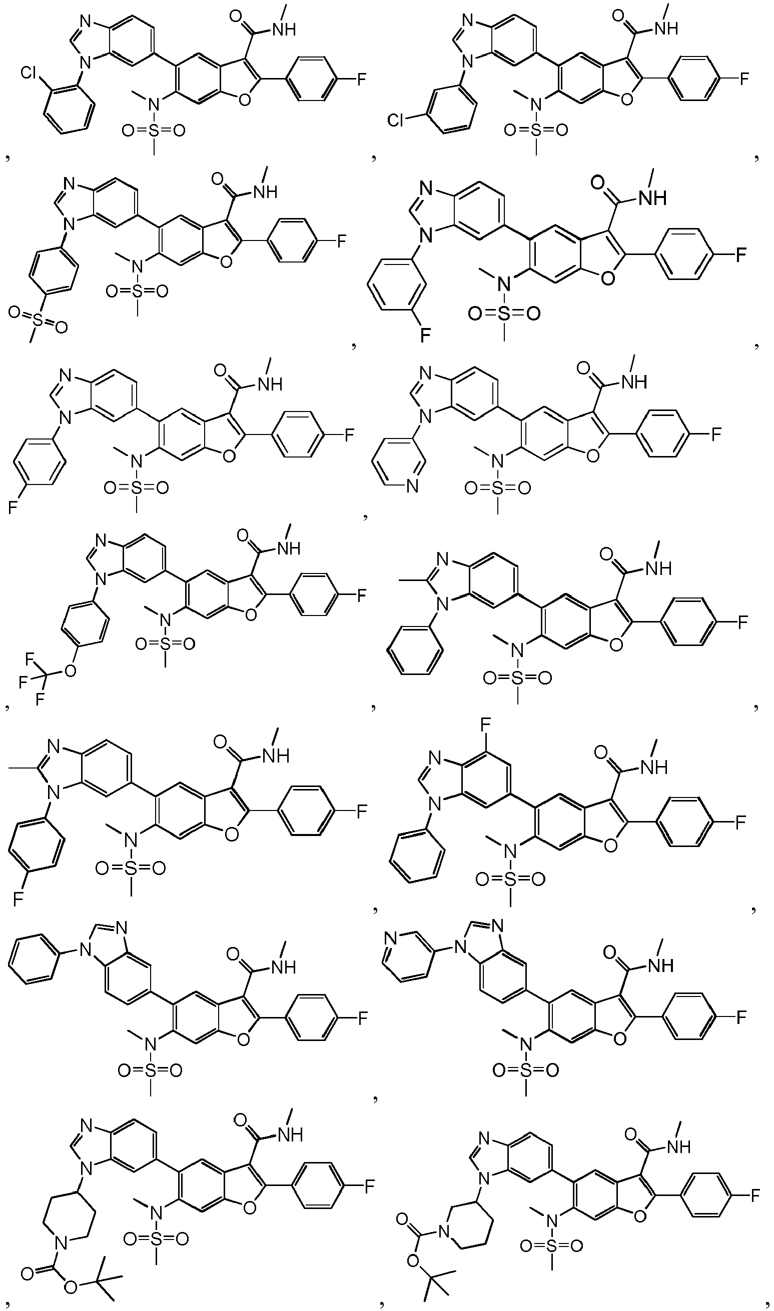 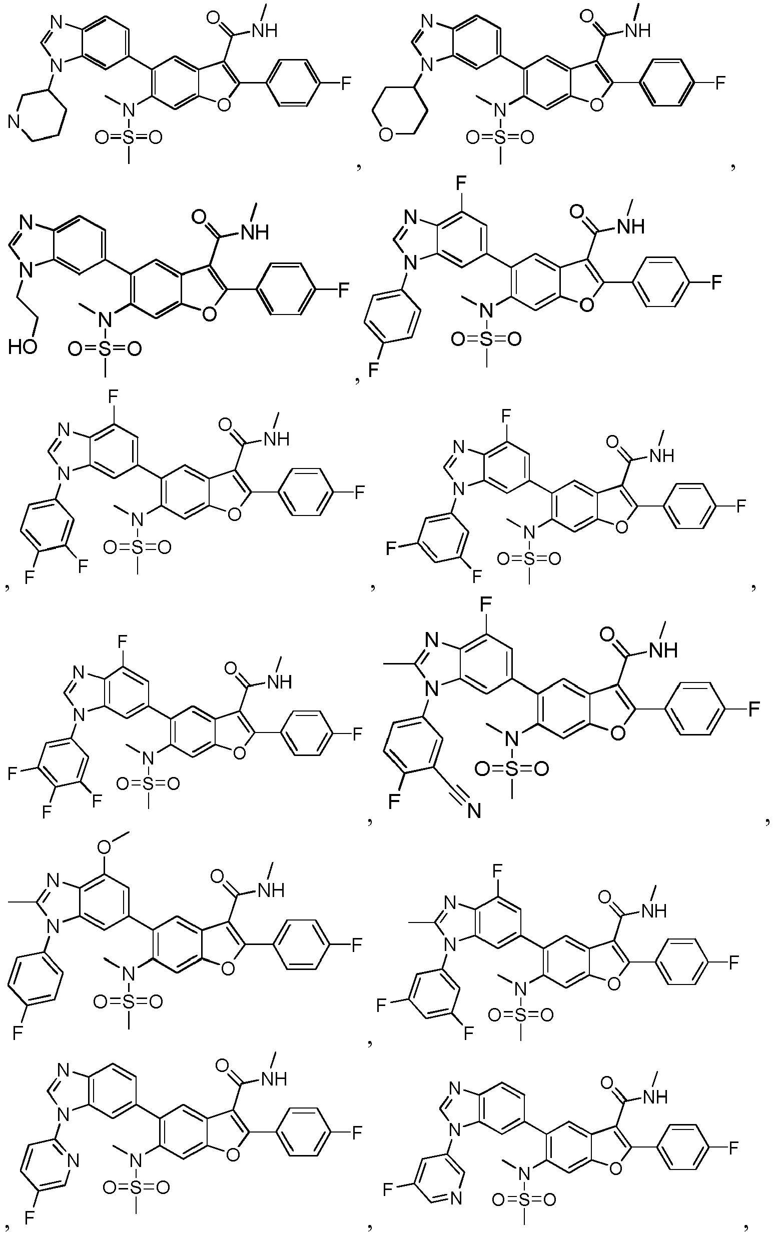 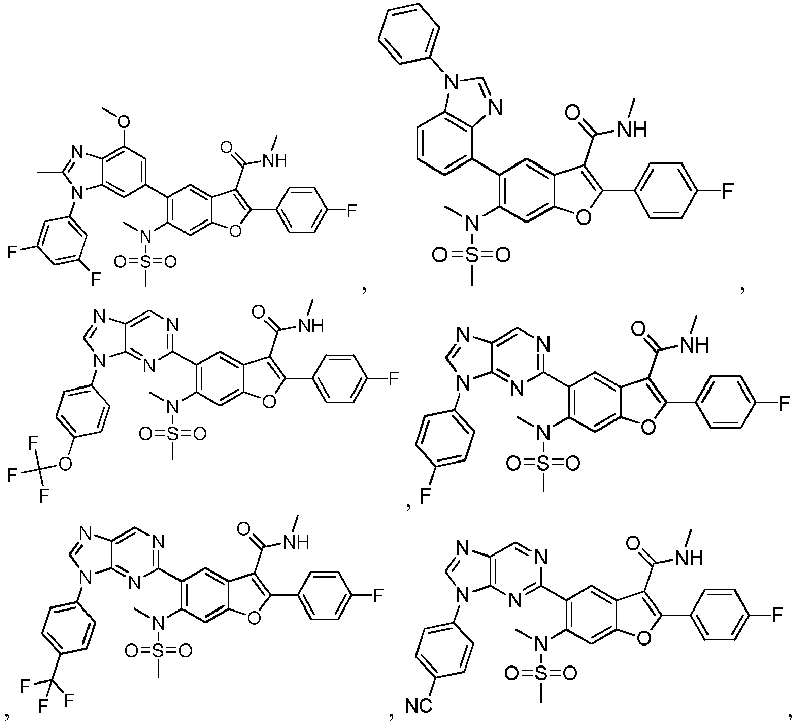 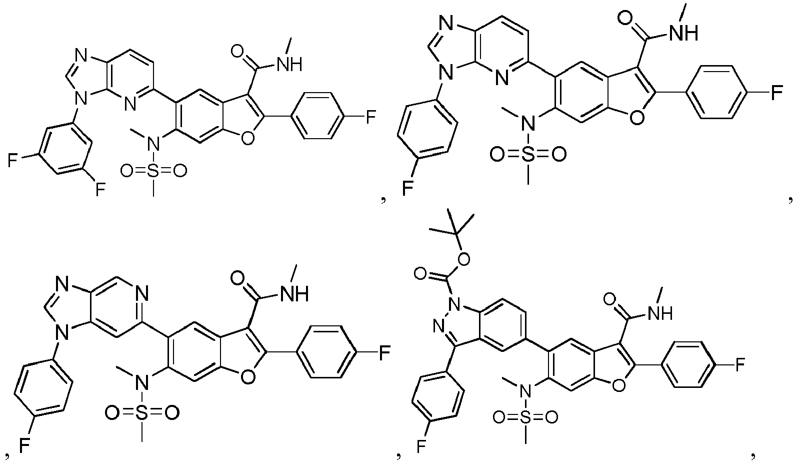 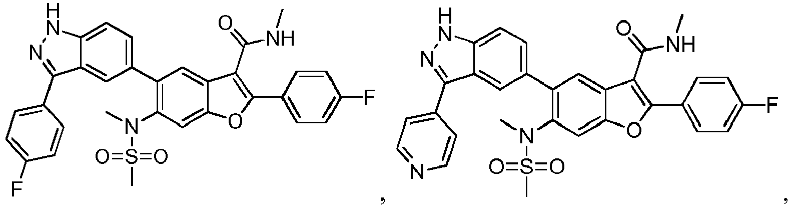 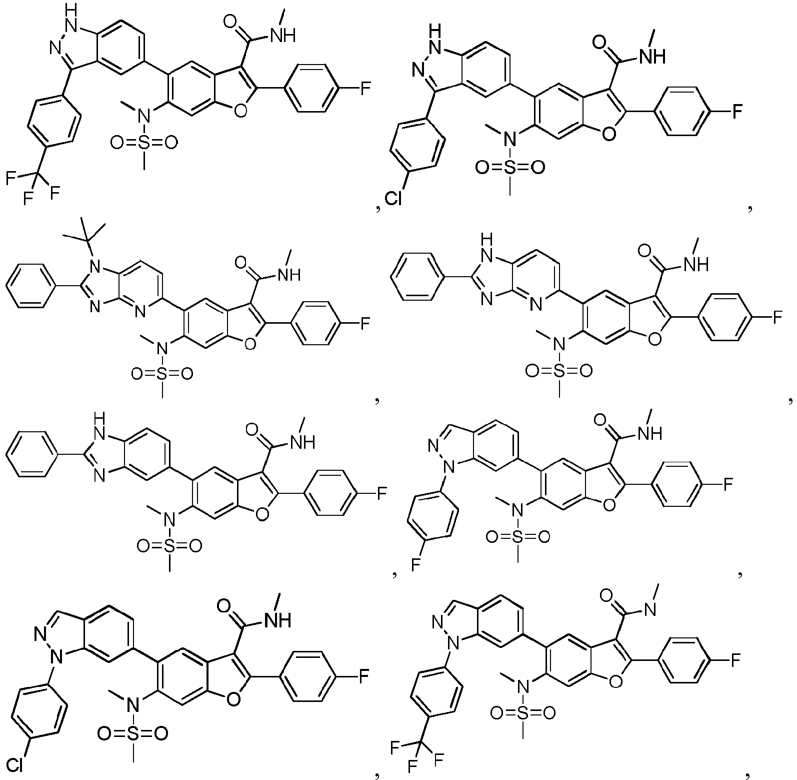 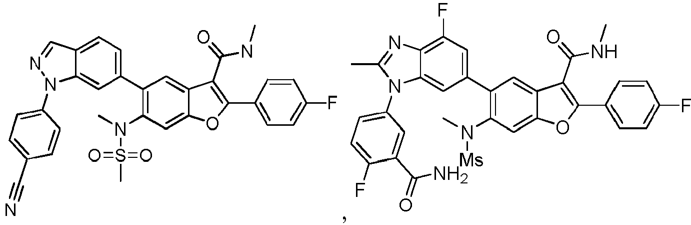 und 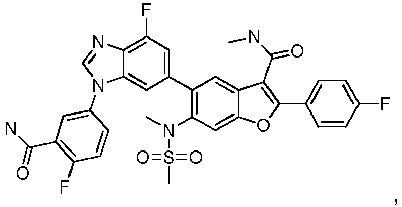 oder ein pharmazeutisch annehmbares Salz davon.

4. Eine pharmazeutische Zusammensetzung, die einen pharmazeutisch annehmbaren Träger und eine wirksame Menge der Verbindung nach einem der Ansprüche 1-3 oder eines pharmazeutisch annehmbaren Salzes davon umfasst.

5. Die pharmazeutische Zusammensetzung nach Anspruch 4, die ferner ein zweites therapeutisches Mittel umfasst, ausgewählt aus der Gruppe bestehend aus antiviral gegen HCV wirkenden Mitteln, Immunmodulatoren und Antiinfektiva.

6. Die pharmazeutische Zusammensetzung nach Anspruch 5, wobei das zweite therapeutische Mittel ausgewählt ist aus der Gruppe bestehend aus HCV-Protease-Inhibitoren, HCV-NS5A-Inhibitoren und HCV-NS5B-Polymerase-Inhibitoren.

7. Die Verbindung nach einem der Ansprüche 1-3 zur Verwendung zur Inhibierung der HCV-NS5B-Aktivität oder zur Prävention und/oder Behandlung einer HCV-Infektion bei einem Patienten, der diese benötigt.

## Revendications

1. Composé présentant la formule: 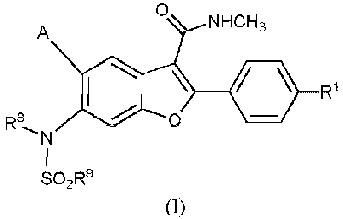 ou un sel pharmaceutiquement acceptable de celui-ci,
dans lequel:
A présente la structure: 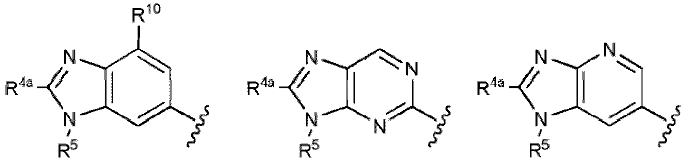 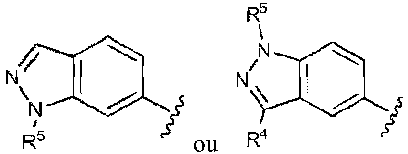
R¹ est un halo;
R⁴ est choisi parmi H, un pyridyle et un phényle, dans lequel ledit groupe phényle peut être optionnellement substitué avec un C₁-C₆ alkyle, un C₁-C₆ haloalkyle ou un halo;
R^{4a} est choisi parmi H, un méthyle et un phényle, dans lequel ledit groupe phényle peut être optionnellement substitué avec jusqu'à 2 groupes, qui peuvent être identiques ou différents, et sont choisis parmi un C₁-C₆ alkyle, un C₁-C₆ haloalkyle, un halo, -CN, un - O-(C₁-C₆ alkyle) et un -O-(C₁-C₆ haloalkyle);
R⁵ est H; un C₁-C₆ hydroxyalkyle; un -C(O)O-t-butyle; un pyridyle; un tétrahydropyranyle; un phényle, qui peuvent être optionnellement substitués avec un -O-(C₁-C₆ alkyle), un halo, -CN, -CF₃, -OCF₃ ou -SO₂CH₃; un benzyle, qui peut être optionnellement substitué avec -CF₃; et un pyrrolidinyle, qui peut être optionnellement substitué sur son atome d'azote de cycle avec un -C(O)O-t-butyle;
R⁸ et R⁹ sont chacun un C₁-C₆ alkyle et
chaque occurrence de R¹⁰ est indépendamment H ou un halo.

2. Composé selon la revendication 1, dans lequel R¹ est F; R⁸ et R⁹ sont chacun un méthyle; et chaque occurrence de R¹⁰ est indépendamment H ou F.

3. Composé selon la revendication 1 qui est choisi parmi: 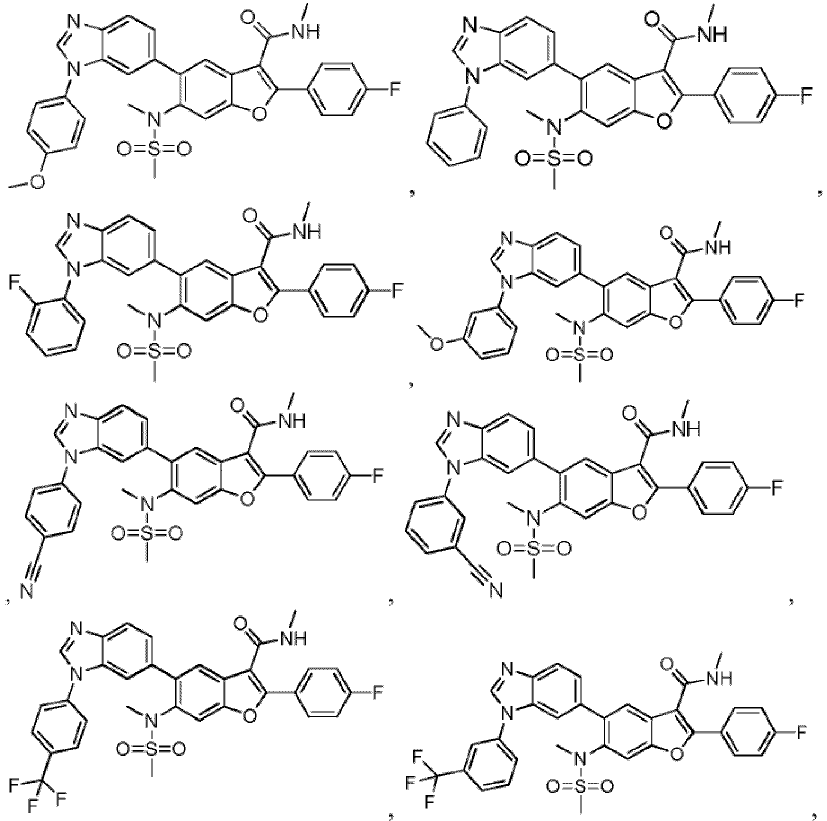 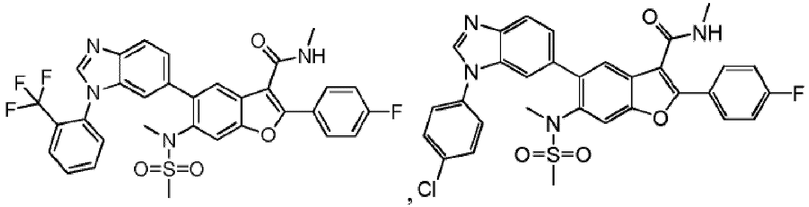 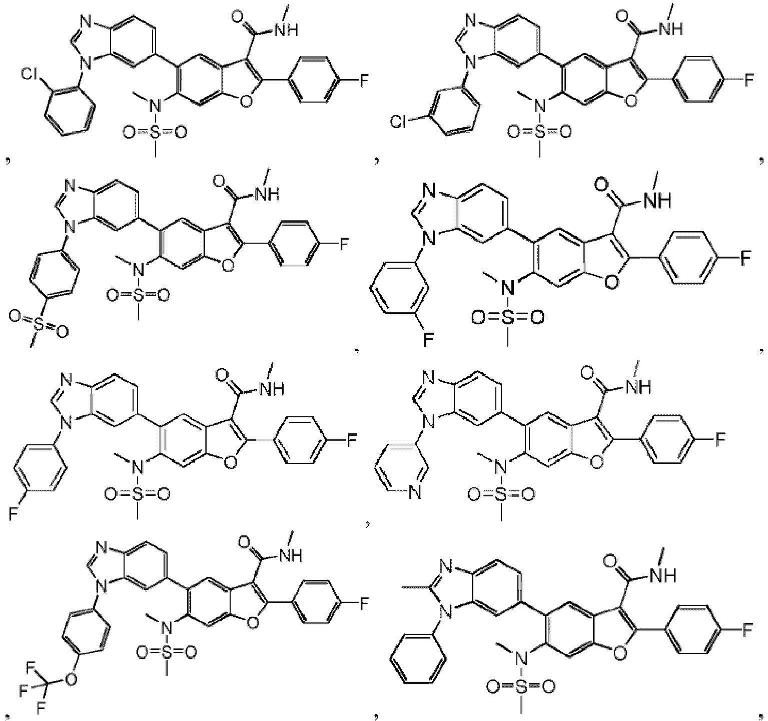 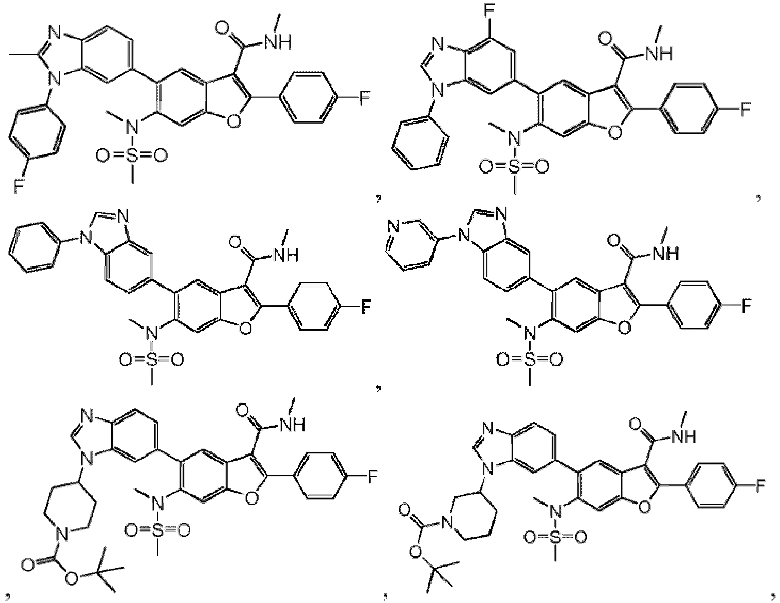 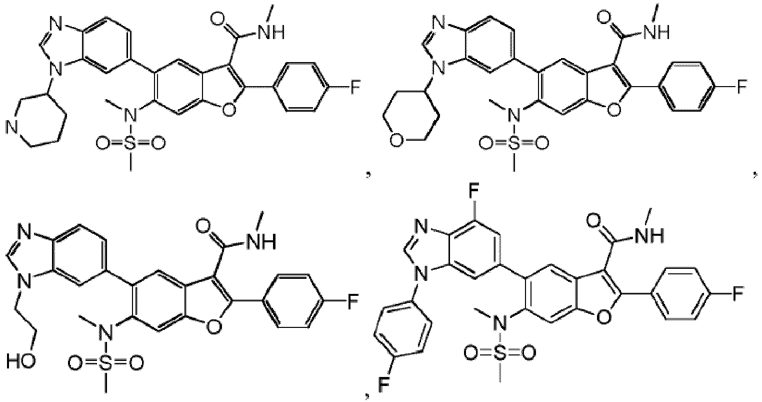 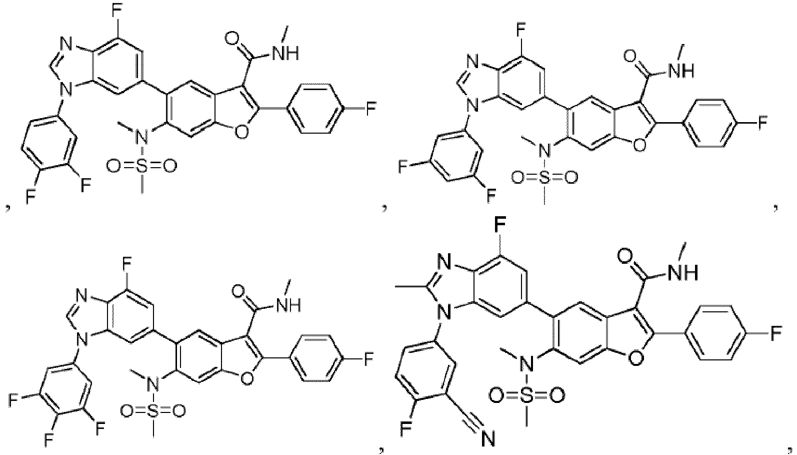 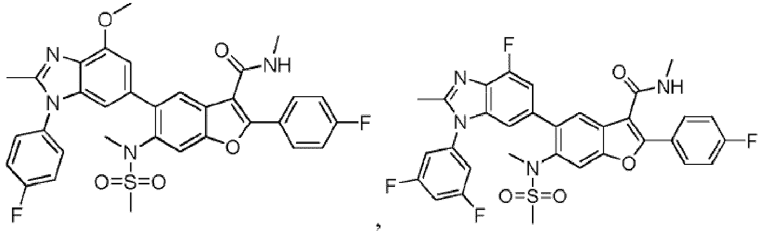 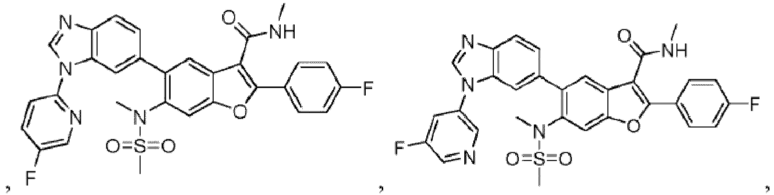 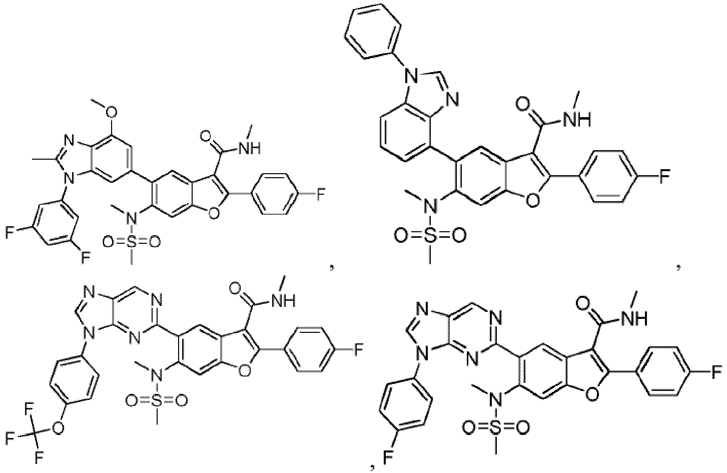 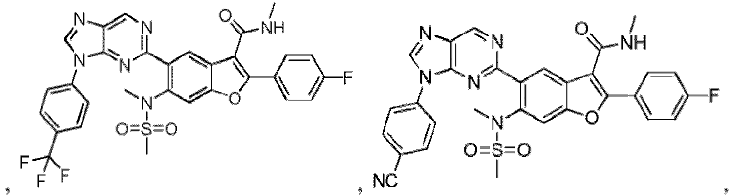 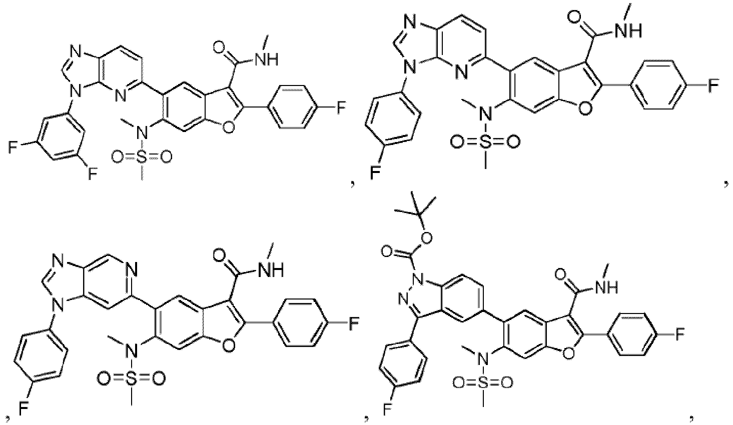 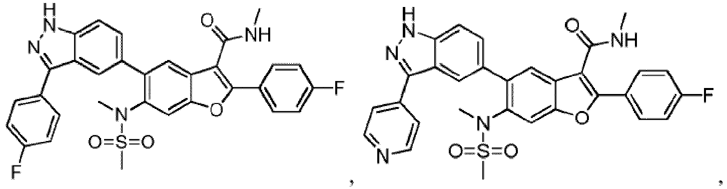 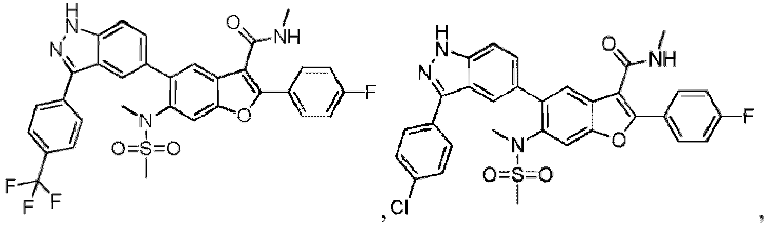 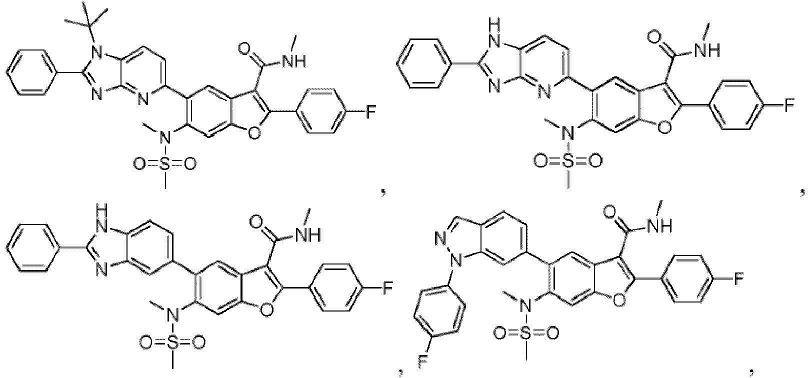 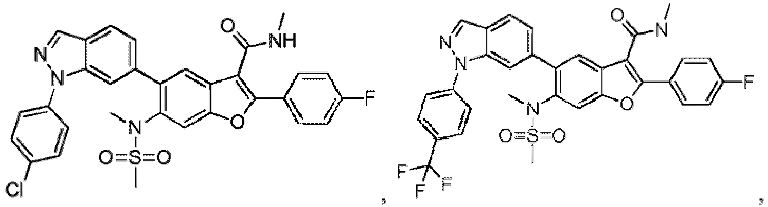 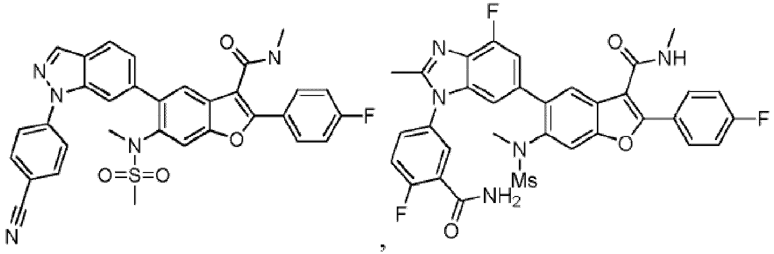 et 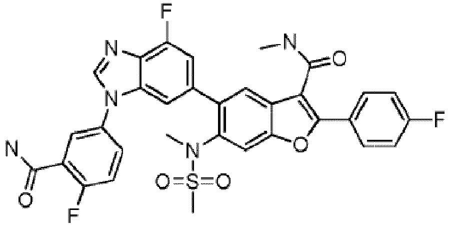 ou un sel pharmaceutiquement acceptable de ceux-ci.

4. Composition pharmaceutique comprenant un support pharmaceutiquement acceptable et une quantité efficace du composé selon l'une quelconque des revendications 1-3 ou d'un sel pharmaceutiquement acceptable de celui-ci.

5. Composition pharmaceutique selon la revendication 4, comprenant en outre un deuxième agent thérapeutique choisi dans le groupe constitué d'agents antiviraux anti-VHC, d'immuno-modulateurs et d'agents anti-infectieux.

6. Composition pharmaceutique selon la revendication 5, dans laquelle le deuxième agent thérapeutique est choisi dans le groupe constitué d'inhibiteurs de protéase du VHC, d'inhibiteurs de NS5A du VHC et d'inhibiteurs de NS5B polymérase du VHC.

7. Composé selon l'une quelconque des revendications 1-3 pour une utilisation dans l'inhibition de l'activité de NS5B du VHC ou pour la prévention et/ou le traitement d'une infection par le VHC chez un patient en ayant besoin.
